(19) Europäisches Patentamt
European Patent Office
Office européen des brevets

(11) EP 4 663 640 A1

(12) **EUROPEAN PATENT APPLICATION**
published in accordance with Art. 153(4) EPC

(43) Date of publication:
**17.12.2025 Bulletin 2025/51**

(21) Application number: **24752675.9**

(22) Date of filing: **17.01.2024**

(51) International Patent Classification (IPC):
***C07D 473/04*** (2006.01) ***C07D 487/04*** (2006.01)
***A61K 31/519*** (2006.01) ***A61P 25/00*** (2006.01)

(52) Cooperative Patent Classification (CPC):
**A61K 31/519; A61K 31/522; A61P 1/00; A61P 1/16; A61P 3/10; A61P 9/00; A61P 9/06; A61P 9/12; A61P 13/12; A61P 17/00; A61P 17/06; A61P 25/00; A61P 25/08; A61P 25/16; A61P 25/18;**
(Cont.)

(86) International application number:
**PCT/CN2024/072849**

(87) International publication number:
**WO 2024/164818 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.02.2023 CN 202310083623**

(71) Applicants:
- **Shanghai Institute of Materia Medica, Chinese Academy of Sciences Shanghai 201203 (CN)**
- **China Pharmaceutical University Nanjing, Jiangsu 210009 (CN)**

(72) Inventors:
- **WANG, Kai Shanghai 201203 (CN)**
- **CAO, Zhengyu Nanjing, Jiangsu 210009 (CN)**
- **SHEN, Jianhua Shanghai 201203 (CN)**
- **LIU, Mengru Nanjing, Jiangsu 210009 (CN)**
- **SONG, Zhaoxiang Shanghai 201203 (CN)**
- **HU, Yixin Nanjing, Jiangsu 210009 (CN)**
- **XU, Tifei Shanghai 201203 (CN)**
- **ZHAO, Fang Nanjing, Jiangsu 210009 (CN)**
- **LIANG, Huaduan Nanjing, Jiangsu 210009 (CN)**

(74) Representative: **dompatent Partnerschaft von Patentanwälten und Rechtsanwälten mbB Deichmannhaus am Dom Bahnhofsvorplatz 1 50667 Köln (DE)**

(54) **XANTHINE COMPOUND AND USE THEREOF**

(57) A xanthine compound and the use thereof. The structure of the compound is as shown in formula I-a, wherein the definition of each substituent is as described in the description and claims. The compound of formula I-a is an inhibitor or an agonist of TRPC4 and TRPC5 ion channels, and has a potential application value in preventing, delaying or treating diseases related to abnormal function or expression of TRPC4/5.

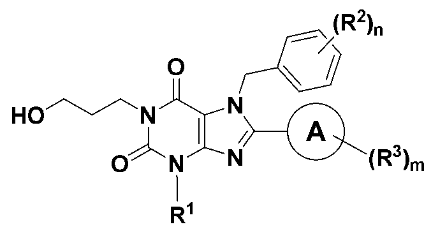

**( I-a )**

(52) Cooperative Patent Classification (CPC): (Cont.)
**A61P 25/20; A61P 25/22; A61P 25/24;**
**A61P 25/28; A61P 29/00; A61P 31/12;**
**A61P 35/00; C07D 473/04; C07D 487/04**

## Description

### Technical Field

The present invention relates to the field of pharmaceutical chemistry, and in particular, to xanthine compounds, preparation method and use thereof. The compounds are inhibitors or agonists of TRPC4 and TRPC5 ion channels, and have potential application value in preventing, delaying or treating a disease related to abnormal TRPC4/5 function or expression.

### Background Art

Transient receptor potential (TRP) ion channels are a class of six transmembrane channel proteins widely distributed in the peripheral and central nervous systems. They are molecular receptors that sense changes in the cell environment, transmit signals, and maintain cell homeostasis. According to sequence similarity, mammalian TRP ion channels can be divided into six subfamilies, including TRPA, TRPC, TRPM, TRPML, TRPP, and TRPV. Classical transient receptor potential (TRPC) channels are non-selective cation channels that allow $Ca^{2+}$ and $Na^{+}$ to pass through. Mammalian TRPC consists of six members, TRPC1, TRPC3, TRPC4, TRPC5, TRPC6, and TRPC7. Among them, the amino acid sequences of TRPC3, TRPC6, and TRPC7 have high consistency, and the sequences of TRPC1, TRPC4, and TRPC5 have high consistency. TRPC family proteins exist in the form of homozygous or heterozygous tetramers and play the role of ion channels. TRPC5 easily forms heterozygous tetramers with TRPC1 and TRPC4.

TRPC4/5 is expressed in multiple parts of the brain, including the hippocampus, amygdala, frontal cortex, etc., and is associated with the occurrence of neurological diseases such as anxiety, depression, epilepsy, and addiction. TRPC4 or TRPC5 gene knockout, or the use of TPRC4/5 inhibitors can relieve anxiety and depression symptoms in mice. A TRPC4/5 inhibitor BI-1358894 developed by Boehringer Ingelheim Pharmaceuticals is currently undergoing clinical trials for the treatment of mental illnesses such as depression, borderline personality disorder, and post-traumatic stress disorder.

Podocytes are special cells that form the glomerular filtration barrier. TRPC5 is also expressed in glomerular podocytes, and its dysfunction is associated with the formation of chronic kidney disease. TRPC5 is involved in the regulation of angiotensin-stimulated cell migration and actin remodeling. In a variety of animal models of kidney disease, TRPC5 gene knockout or TPRC5 inhibitors can significantly reduce proteinuria levels, protect podocytes from damage, and inhibit the progression of kidney disease. GFB-887, a TRPC4/5 inhibitor developed by Goldfinch Biotechnology, is currently undergoing clinical trials for the treatment of kidney diseases such as diabetic nephropathy and focal segmental glomerulosclerosis.

TRPC4/5 is expressed in skin keratinocytes and plays an important role in regulating keratinocyte differentiation. Therefore, targeted regulation of TRPC4/5 may be used to treat skin lesions with abnormal keratinocyte function as the pathological basis.

TRPC5 is expressed in the liver and portal vein. A series of endogenous phospholipids that play an important role in the formation of liver disease, such as lysolecithin (LPC), can significantly activate TRPC5. In a mouse model of cholestatic liver disease induced by bile acid feeding, TRPC5 gene knockout can alleviate liver damage and abnormal liver lipid metabolism. It can be seen that TRPC5 is also a potential target for the treatment of liver diseases.

TRPC4/5 are also expressed in peripheral sensory neurons and are involved in pain perception. Compared with wild-type animals, $TRPC4^{-/-}$ mice are resistant to mustard oil-induced pain and have an increased pain threshold. In a variety of mouse models of neuropathic pain, inflammatory pain, and spontaneous pain, TRPC5 gene knockout or TRPC5 inhibitors can effectively alleviate mechanical allodynia, and this effect is intrinsically linked to the biological function regulated by LPC.

TRPC4/5 is also associated with cardiovascular disease. Activation of TRPC5 channels can significantly promote the proliferation and migration of vascular smooth muscle cells. Inhibition of TRPC5 can significantly reduce hypoxia-ischemia-induced apoptosis and oxidative stress of endothelial cells, reduce endothelium-dependent vasoconstriction, and inhibit the formation of atherosclerotic plaques. It can also reduce the expression of myocardial hypertrophy-related genes and maintain the stability of cardiac electrical activity, thereby playing an anti-atherosclerotic, anti-myocardial hypertrophy and anti-arrhythmic role. In addition, TRPC5-mediated calcium influx is a key signaling mechanism for the release of a variety of endothelial-derived contractile factors, which contributes to the development of hypertension. TRPC5 promotes the proliferation of vascular smooth muscle cells and increases oxidative stress, thereby promoting the occurrence of hypertension.

TRPC4/5 is associated with tumor angiogenesis, tumor resistance and metastasis. TRPC5 is highly expressed in colorectal cancer, promoting extracellular calcium influx to activate the Wnt5a/β-catenin pathway, reduce tumor differentiation, and increase tumor stem cells. TRPC5 expression levels are negatively correlated with the prognosis of colorectal cancer patients. TRPC5 can also reduce E-cadherin, increase the expression of mesenchymal markers, and

promote tumor cell migration, invasion and proliferation. In addition, calcium ion dysregulation caused by activation of TRPC5 can promote anaerobic inducible factor1 (HIF-1)-mediated tumor angiogenesis, allowing cancer cells to escape from sites exposed to high doses of anticancer drugs and obtain additional nutrients to help tumors survive, thereby reducing the effect of chemotherapy. In breast cancer models, inhibition of TRPC5 can reduce chemotherapy resistance. On the other hand, activation of TRPC4/5 can also induce tumor cell death through calcium ion overload. For example, the natural product (-)-Englerin A is a potent TRPC4/5 agonist that exhibits antiproliferative effects on a variety of tumor cells such as renal cancer, lung cancer, breast cancer, and skin cancer.

TRPC4/5 is expressed in the smooth muscle and myenteric plexus of the small intestine, and is involved in regulating intestinal smooth muscle cell contraction, gastrointestinal motility, and enteric nerve signaling. Therefore, compounds that target and inhibit the activity of TRPC4/5 channels also have potential application value in the treatment of intestinal diseases.

WO2014143799 discloses xanthine compounds as shown in the following general formula, wherein $R^2$, a substituent at position 8, is defined as C1-C6 alkyl, C1-C6 heteroalkyl, C1-C6 alkoxy, C3-C7 cycloalkyloxy, C6-C10 aryl, C6-C10 aryloxy, C7-C16 aryl alkoxy, heterocycloalkyl, heteroaryl, heteroaryloxy, etc. Compound 260 in this application, as a key research compound, has good TRPC5 inhibitory activity and also shows good efficacy in depression and anxiety mouse models. In another comparative document (PLoS ONE. 2018, 13, e0191225.), compound 260 is also referred to as HC-070. The document shows in detail the activity of HC-070 in inhibiting TRPC4/5 channels and the anti-anxiety and anti-depressant effects in various animal models. However, HC-070 lacks good selectivity for some other ion channels and can significantly inhibit hERG potassium channel and TRPC3.

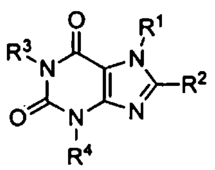

Formula I

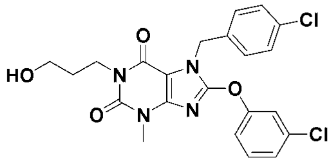

Compound 260/ HC-070

WO2019011802 discloses compounds in which the 8-position of xanthine is substituted by "3-pyridyl" or "2-piperazinyl" as shown below. Compared with HC-070, they maintain higher TRPC5 inhibitory activity while also reducing the inhibitory effect on hERG channels.

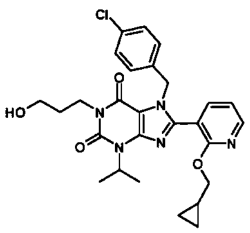

Example 5

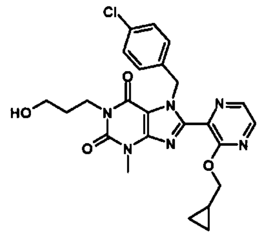

Example 12

## Summary of the invention

The main object of the present invention is to provide a class of xanthine compounds used as TRPC4 and TRPC5 regulators, preparation method and therapeutic use thereof.

In a first aspect, the present invention provides a fused pyrimidinedione compound represented by general formula (I), a stereoisomer thereof and a pharmaceutically acceptable salt thereof;

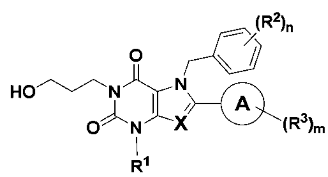

(I)

wherein, ring A is a saturated or partially unsaturated C3-C10 cyclic hydrocarbyl, including mono, fused, spiro and

bridged ring systems; X is N or CH; $R^1$ is C1-C6 alkyl or C1-C6 haloalkyl; n is an integer from 1 to 4, and each $R^2$ is independently H, deuterium, a halogen atom, C1-C6 alkyl, C1-C6 haloalkyl, C1-C6 alkoxy, C1-C6 haloalkoxy, or cyano; m is an integer from 1 to 6, and each $R^3$ is independently selected from H, deuterium, C1-C6 alkyl, C1-C6 haloalkyl, C1-C6 alkoxy, C1-C6 haloalkoxy, C3-C8 cycloalkyl, C6-C10 aryl, 5-8 membered heteroaryl, 4-8 membered heterocyclyl, halogen atom, cyano, hydroxyl, or $-NR^4R^5$; or $R^3$ and the connected carbon on the A ring together form 4-8 membered heterocycle or C6-C10 aryl; the cycloalkyl, aryl, heteroaryl, heterocyclyl is unsubstituted or optionally substituted by 1-4 groups selected from a group consisting of C1-C6 alkyl, C1-C6 alkoxy, halogen atom, and cyano; $R^4$ and $R^5$ are each independently H, C1-C6 alkyl, or C3-C8 cycloalkyl.

In a second aspect, the present invention also provides a xanthine compound represented by general formula (I-a), stereoisomer thereof and pharmaceutically acceptable salt thereof;

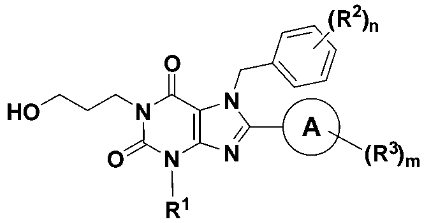

**( I-a )**

wherein, ring A is a saturated or partially unsaturated C3-C10 cyclic hydrocarbyl, preferably including a mono, fused, spiro and bridged ring systems;

$R^1$ is C1-C6 alkyl or C1-C6 haloalkyl;

n is an integer from 1 to 4, and each $R^2$ is independently H, deuterium, halogen, C1-C6 alkyl, C1-C6 haloalkyl, C1-C6 alkoxy, C1-C6 haloalkoxy, or cyano;

m is an integer from 1 to 6, and each $R^3$ is independently selected from H, deuterium, C1-C6 alkyl, C1-C6 haloalkyl, C1-C6 alkoxy, C1-C6 haloalkoxy, C3-C8 cycloalkyl, C6-C10 aryl, 5-8 membered heteroaryl, 4-8 membered heterocyclyl, halogen, cyano, hydroxyl, $-NR^4R^5$; or $R^3$ and the connected carbon on the A ring together form a 4-8 membered heterocycle or a C6-C10 aryl group; the cycloalkyl, aryl, heteroaryl, heterocyclyl is unsubstituted or optionally substituted by 1-4 groups selected from a group consisting of C1-C6 alkyl, C1-C6 haloalkyl, C1-C6 alkoxy, C1-C6 haloalkoxy, halogen, and cyano;

$R^4$ and $R^5$ are each independently H, C1-C6 alkyl, or C3-C8 cycloalkyl.

In another preferred embodiment, ring A is a saturated or partially unsaturated C3-C8 monocyclic or bridged hydrocarbyl.

In another preferred embodiment, the A ring is selected from the following group:

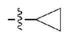,

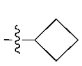, 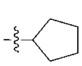, 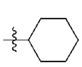, 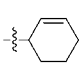, 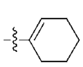, 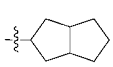, 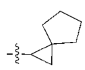, 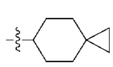, 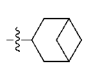, 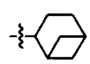, 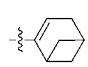, 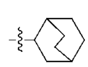,

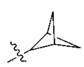.

In another preferred embodiment, ring A is cyclohexyl (

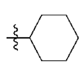

) or cyclohexenyl (such as

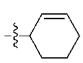, 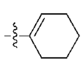).

In another preferred embodiment, the compound has a structure as shown in general formula (I-b);

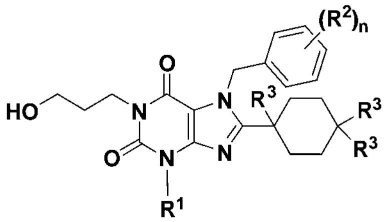

**( I-b )**

wherein, $R^1$ is a C1-C6 alkyl;

n is an integer from 1 to 4, and each $R^2$ is independently H, CN, C1-C6 haloalkyl or halogen atom;

each $R^3$ is independently selected from H, deuterium, C1-C6 alkyl, C1-C6 haloalkyl, C1-C6 alkoxy, C1-C6 haloalkoxy, C6-C10 aryl, C3-C8 cycloalkyl, 5-8 membered heteroaryl, 4-8 membered heterocyclyl, halogen atom, cyano, hydroxyl, or $-NR^4R^5$; or two $R^3$ and the connected carbon on the A ring together form a 4-8 membered heterocyclic ring;

$R^4$ and $R^5$ are each independently H or C1-C6 alkyl.

In another preferred embodiment, $R^1$ is a C1-C4 alkyl. In another preferred embodiment, $R^1$ is methyl, ethyl or isopropyl.

In another preferred embodiment, n is 1, 2 or 3, and each $R^2$ is independently H, deuterium, halogen, C1-C4 alkyl, C1-C4 haloalkyl, C1-C4 alkoxy, C1-C4 haloalkoxy, or cyano. In another preferred embodiment, n is 1, 2 or 3, and each $R^2$ is independently H, deuterium, F, Cl, Br, methyl, ethyl or isopropyl. In another preferred embodiment, n is 1, $R^2$ is F, Cl or Br, located in the para position. In another preferred embodiment, n is 2, and each $R^2$ is independently F, Cl or Br, located in the para and meta positions.

In another preferred embodiment, $R^2$ is -F or -Cl.

In another preferred embodiment, m is 1, 2, 3 or 4, and each $R^3$ is independently selected from H, deuterium, C1-C4 alkyl, C1-C4 haloalkyl, C1-C4 alkoxy, C1-C4 haloalkoxy, C3-C6 cycloalkyl, phenyl, 5-6 membered heteroaryl, 5-6 membered heterocyclyl, F, Cl, Br, cyano, hydroxyl, or $-NR^4R^5$; or $R^3$ and the connected carbon on the A ring together form a 5-6 membered heterocycle or phenyl; the cycloalkyl, phenyl, heteroaryl, heterocyclyl is unsubstituted or optionally substituted with 1-4 groups selected from the following groups: C1-C6 alkyl, C1-C6 haloalkyl, C1-C6 alkoxy, C1-C6 haloalkoxy, halogen, and cyano.

In another preferred embodiment, each $R^3$ is independently selected from H, methyl, ethyl, n-propyl, isopropyl, trifluoromethyl, difluoromethyl, monofluoromethyl, phenyl, cyclobutyl, cyclopropyl, cyclopentyl, cyclohexyl, methoxy, ethoxy, n-propoxy, isopropoxy, hydroxyl, F, Cl, Br, cyano, amino, methylamino, dimethylamino, oxetanyl, dioxolanyl, morpholinyl, piperazinyl, pyrrolidinyl, or piperidinyl.

In another preferred embodiment, each $R^3$ is independently selected from H, C1-C6 alkyl, C1-C6 haloalkyl, C1-C6 alkoxy, C3-C8 cycloalkyl, phenyl, 4-8 membered heterocyclyl, halogen atom, cyano, hydroxyl, or $-NR^4R^5$.

In another preferred embodiment, $R^4$ and $R^5$ are each independently H or a C1-C4 alkyl.

In another preferred embodiment, each $R^3$ is independently selected from H, C1-C3 haloalkyl or halogen atom. In another preferred embodiment, $R^3$ is -F.

In another preferred embodiment, $R^3$ and the connected carbon on the A ring together form

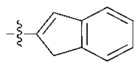 or 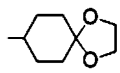.

In another preferred embodiment, the compound represented by general formula (I), (I-a) or (I-b) is selected from Example compounds E1 to E73-2.

In a third aspect, the present invention provides a pharmaceutical composition comprising at least one effective therapeutic dose of a compound represented by general formula (I) or (I-a) or (I-b), a stereoisomer thereof or a pharmaceutically acceptable salt thereof, and a pharmaceutically acceptable excipient. The pharmaceutical excipient is a pharmaceutically acceptable carrier, excipient, sustained-release agent, odorant, flavoring agent, and the like.

In the pharmaceutical composition, the compound of the present invention is used as the active ingredient, and its weight accounts for 0.1 to 99.9% of the total weight of the pharmaceutical composition, and the rest are pharmaceutically acceptable excipient; the preferred ratio of the compound of the present invention to the excipient is: the compound of the present invention as the active ingredient accounts for more than 60% of the total weight, and the rest accounts for 0-40%

of the total weight, and the amount of the rest is preferably 1-20%, and most preferably 1-10%.

The compound or pharmaceutical composition of the present invention can be prepared into various dosage forms based on the conventional processes in the field of pharmaceutical preparations, such as tablets, capsules, powders, syrups, solutions, suspensions, sprays, creams, ointments, gels, transdermal patches, etc., and can be present in suitable solid or liquid carriers or diluents. The pharmaceutical composition of the present invention can also be stored in suitable sterile equipment for injection or infusion.

The compounds or pharmaceutical compositions of the present invention can be used in mammals, including humans and animals. The administration routes include oral administration, nasal inhalation, topical administration to the skin, intravenous injection, intramuscular injection, subcutaneous injection, etc. In another preferred embodiment, the preferred administration route of the compounds or pharmaceutical compositions of the present invention is oral administration.

The solid dosage form of the compound or pharmaceutical composition of the present invention for oral administration includes capsules, tablets, pills, powders and granules. Solid carriers include: starch, lactose, dicalcium phosphate, microcrystalline cellulose, sucrose and white clay, etc., and liquid carriers include: sterile water, polyethylene glycol, nonionic surfactants and edible oils (such as corn oil, peanut oil and sesame oil), etc., as long as they are suitable for the characteristics of the active ingredient and the required specific administration mode. Commonly used adjuvants in the preparation of pharmaceutical compositions can also be advantageously included, such as flavoring agents, pigments, preservatives and antioxidants such as vitamin E, vitamin C, BHT and BHA.

Liquid dosage forms for oral administration include pharmaceutically acceptable emulsions, solutions, suspensions, syrups or tinctures. In addition to the active ingredient, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butylene glycol, dimethylformamide and oils, particularly cottonseed oil, peanut oil, corn germ oil, olive oil, castor oil and sesame oil or mixtures of these substances, etc. In addition to these inert diluents, the composition may also contain adjuvants, such as wetting agents, emulsifiers and suspending agents, sweeteners, flavoring agents and spices.

In addition to the active ingredients, suspensions may contain suspending agents such as, for example, ethoxylated isostearyl alcohols, polyoxyethylene sorbitol and sorbitan esters, microcrystalline cellulose, aluminum methanol and agar, or mixtures of these substances.

When used as a pharmaceutical preparation, the compounds shown in the present invention are preferably in unit doses, each dose containing 0.01 mg - 200 mg of the active ingredient, preferably 0.5 mg - 50 mg, for single or divided use. Regardless of the method of administration, the optimal dose for an individual should be determined according to the specific treatment. Usually, it is started with a small dose and gradually increased until the most suitable dose is found.

In a fourth aspect, the present invention provides use of a compound represented by general formula (I) or (I-a) or (I-b), a stereoisomer thereof, a pharmaceutically acceptable salt thereof or a pharmaceutical composition thereof as a regulator of TRPC4 and TRPC5 ion channels, and thus for the manufacture of a drug for preventing, delaying or treating a disease related to abnormal function or expression of TRPC4 and/or TRPC5.

In the present invention, the regulators of the TRPC4 and TRPC5 ion channels include inhibitors and agonists.

In a preferred embodiment, the regulators of TRPC4 and TRPC5 ion channels are TRPC4 inhibitors and TRPC5 inhibitors.

In the present invention, the diseases related to the abnormal function and expression of TRPC4 and/or TRPC5 include but are not limited to mental illness, neurodegenerative disease, kidney disease, pain, epilepsy, liver disease, cardiovascular disease, cancer, skin disease, intestinal disease, and the like.

In a preferred embodiment, the disease related to the abnormal function and expression of TRPC4 or/and TRPC5 is a mental illness. The mental illness includes diseases related to depression, such as borderline personality disorder, depression, dysthymia, postpartum depression, bipolar disorder, etc.; the mental illness also includes symptoms related to anxiety and fear, such as post-traumatic stress disorder, panic disorder, agoraphobia, social phobia, generalized anxiety disorder, social anxiety disorder, separation anxiety, etc.; the mental illness also includes affective disorders and mental disorders caused by various other reasons, such as schizophrenia, mania, obsessive-compulsive disorder, apathy, neurasthenia, paranoia, etc.

In another preferred embodiment, the mental illness is borderline personality disorder, depression, anxiety, or post-traumatic stress disorder.

The compound or its pharmaceutical composition of the present invention can be used alone or in combination with drugs of other mechanisms when used to treat mental illness. The drugs of other mechanisms include but are not limited to tricyclic antidepressants (TCA), monoamine oxidase inhibitors (MAOI), serotonin (5-HT) reuptake inhibitors (SSRI), serotonin and norepinephrine reuptake inhibitors (SNRI), norepinephrine specific 5-HT antidepressants (NaSSA), 5-HT receptor antagonists and reuptake inhibitors (SARI), and the like. SSRI and SNRI are preferred. Representative drugs of SSRI include fluoxetine, paroxetine, citalopram, sertraline, fluvoxamine, etc. Representative drugs of SNRI include duloxetine, venlafaxine, milnacipran, etc.

In another preferred embodiment, the disease associated with the abnormal function and expression of TRPC4 and/or TRPC5 is a neurodegenerative disease. The neurodegenerative diseases include Alzheimer's disease (AD), Parkinson's disease, Huntington's disease, amyotrophic lateral sclerosis (ALS), memory impairment, amnesia, aphasia, chronic fatigue syndrome, Creutzfeldt-Jakob disease, dissociative amnesia, fugue amnesia, learning disabilities, sleep disorders, and other brain diseases caused by trauma or aging.

In another preferred embodiment, the disease related to the abnormal function and expression of TRPC4 and/or TRPC5 is pain. The pain includes nociceptive pain, mechanical pain, inflammatory pain, cancer pain and neuropathic pain (e.g. osteoarthritis pain, rheumatoid arthritis pain, post-herpetic neuralgia, pain caused by burns). The pain can be chronic pain or acute pain.

In another preferred embodiment, the disease related to the abnormal function and expression of TRPC4 or/and TRPC5 is epilepsy. Epilepsy can be caused by excitotoxicity of various origins. Excessive neuronal discharges can usually drive epileptic activity. Compounds that reduce the hyperexcitability of related neuronal populations have significant potential in reducing epileptic activity. Inhibiting TRPC5-mediated calcium influx may reduce hyperexcitability and thus reduce epileptic activity.

In another preferred embodiment, the disease related to the abnormal function and expression of TRPC4 or/and TRPC5 is a kidney disease. The kidney disease includes polar kidney damage and chronic kidney damage caused by various reasons, such as kidney damage caused by toxic substances, kidney damage caused by viral or bacterial infection, hypertensive nephropathy, diabetic nephropathy, glomerulonephritis, lupus nephritis, IgA nephropathy, nephrotic syndrome, membranous nephropathy, minimal change nephropathy, and also includes hereditary kidney diseases, such as polycystic kidney disease, focal segmental glomerulosclerosis, etc.

In another preferred embodiment, the disease related to the abnormal function and expression of TRPC4 and/or TRPC5 is a skin disease. Further, the skin disease is related to the abnormal function of keratinocytes, such as but not limited to psoriasis, ichthyosis, palmoplantar keratoderma, Olmsted's disease, toad skin disease or menopausal keratoderma.

In another preferred embodiment, the disease related to the abnormal function and expression of TRPC4 and/or TRPC5 is a disease related to the cardiovascular system, including but not limited to hypertension, atherosclerosis, coronary heart disease, angina pectoris, myocardial infarction, arrhythmia, stroke, pulmonary hypertension, etc.

In another preferred embodiment, the disease related to the abnormal function and expression of TRPC4 and/or TRPC5 is liver disease, including but not limited to cholestatic liver disease, alcoholic steatohepatitis, non-alcoholic steatohepatitis, viral hepatitis, autoimmune liver disease, chemical liver injury, liver fibrosis, cirrhosis, and hepatocellular carcinoma.

In another preferred embodiment, the disease associated with the abnormal function and expression of TRPC4 and/or TRPC5 is cancer, including but not limited to renal cell carcinoma, breast cancer, colorectal cancer, etc.

In another preferred embodiment, the disease related to the abnormal function and expression of TRPC4 and/or TRPC5 is an intestinal disease, including but not limited to ulcerative colitis, short bowel syndrome, irritable bowel syndrome, intestinal cramps, diarrhea, abdominal pain, etc.

Lysolecithin (LPC) can directly activate TRPC5, independent of other indirect activation mechanisms, such as the GPCR-PLC pathway. A series of biological effects of LPC are related to its activation of TRPC5, so TRPC5 inhibitors may be used to combat diseases associated with LPC level disorders. The diseases include but are not limited to atherosclerosis, cardiovascular disease, central and peripheral nervous system diseases, osteoarthritis, hepatitis, hepatocellular carcinoma, pneumonia, obesity, gastrointestinal diseases, bacterial infections, parasitic diseases, diabetes, tumors, pain, etc.

In a fifth aspect, the present invention provides a method for preparing the compound of the present invention, stereoisomer, and pharmaceutically acceptable salt thereof, wherein the method is selected from one of the synthetic routes shown in the following Route A and Route B.

When the compound of the present invention has a structure shown in the general formula (II-a) to (II-d), it can be prepared according to the synthetic route shown in route A; wherein halo is a halogen atom, $R^{3a}$ is a C1-C6 alkyl, THP represents a tetrahydropyran protective group, and the other substituents are defined the same as above.

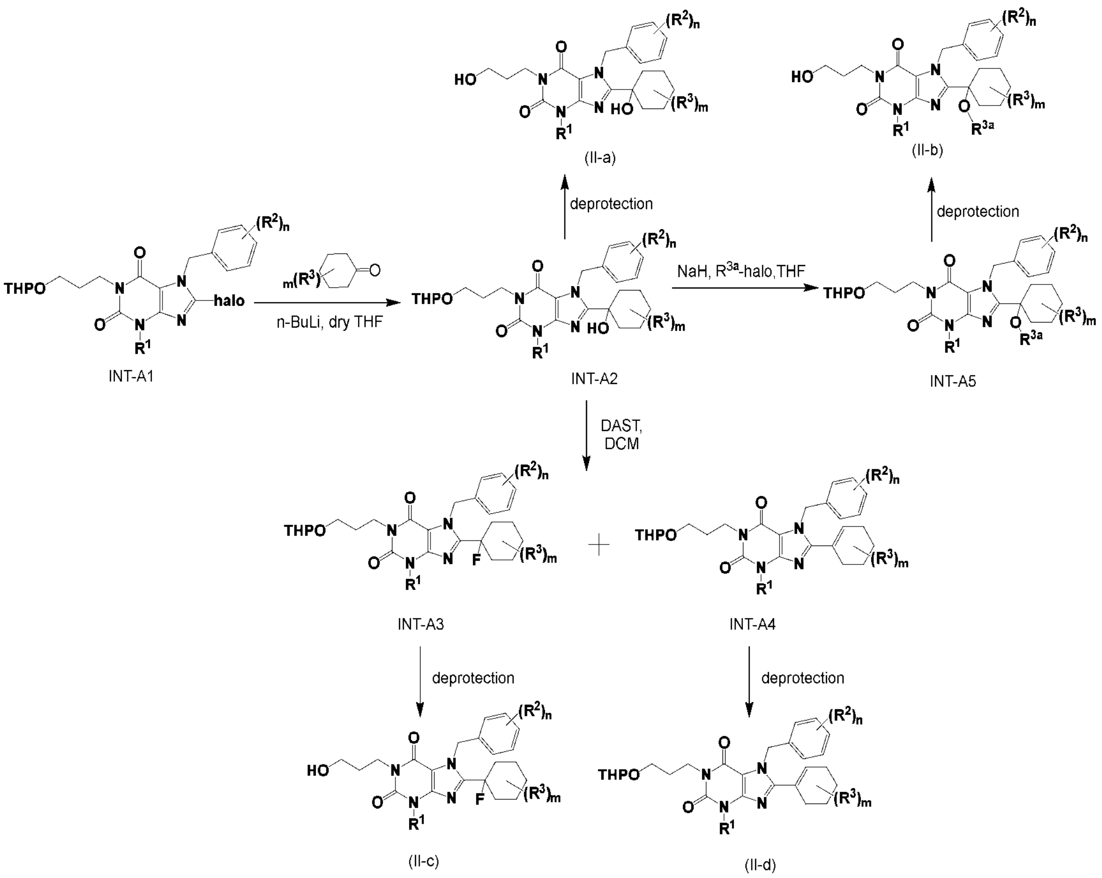


**Route A**

The reaction steps of route A include:

(1.1) The intermediate INT-A1 (synthesized according to WO2014143799) undergoes lithium halogen exchange and nucleophilic addition reaction with a ketone raw material (commercially purchased) to generate the intermediate INT-A2; the reaction is carried out in an ultra-dry aprotic solvent; the solvent is, for example, but not limited to, dichloromethane (DCM) and tetrahydrofuran (THF), with tetrahydrofuran being the preferred solvent. The reaction temperature is generally -40 to -100 °C, preferably -78 °C;

(1.2) Intermediate INT-A2 is subjected to fluorination reaction with diethylaminosulfur trifluoride (DAST), and the reaction temperature is generally between 0 °C and room temperature to generate intermediates INT-A3 and INT-A4; the reaction is carried out in an aprotic solvent such as DCM; the reaction temperature is generally between 0 °C and room temperature;

(1.3) Intermediate INT-A2 is alkylated with $R^{3a}$-halo and NaH in tetrahydrofuran, and the reaction temperature is generally room temperature to generate intermediate INT-A5;

(1.4) The intermediates INT-A2, INT-A3, INT-A4, and INT-A5 are deprotected from THP protecting groups to generate compounds of the general formula (II-a), (II-b), (II-c), and (II-d); the deprotection reaction of the hydroxyl is usually carried out under the action of an acidic reagent; the acidic reagent is for example but not limited to trifluoroacetic acid, hydrogen chloride, and p-toluenesulfonic acid.

When the compound of the present invention has a structure represented by the sub-general formula (II-e), it can be prepared according to the following route B; wherein halo is a halogen atom, THP represents a tetrahydropyran protective group, and the definitions of other substituents are the same as above.

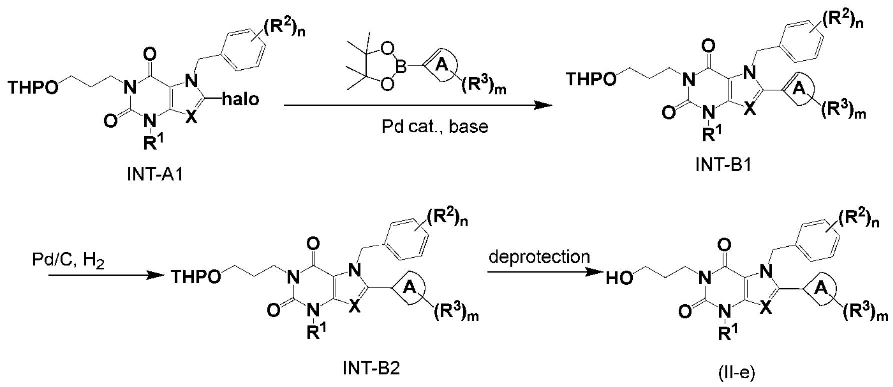

INT-A1
INT-B1
INT-B2
(II-e)

## Route B

The reaction steps of route B include:

(2.1) Intermediate INT-A1 undergoes Suzuki coupling reaction with boron ester raw materials (commercially purchased or synthesized according to literature methods) to generate intermediate INT-B1; the Suzuki coupling reaction is usually carried out under the action of a palladium catalyst and a base; the palladium catalyst is, for example, but not limited to, $Pd(Ph_3P)_2Cl_2$, $Pd(dppf)Cl_2$, $Pd(Ph_3P)_4$; the base is, for example, but not limited to, triethylamine, DIPEA, $K_2CO_3$, $Cs_2CO_3$; the reaction is carried out in a suitable organic solvent or a mixed solvent of an organic solvent and water, such as DMF or 1,4-dioxane; the reaction temperature is generally room temperature to 100 °C;

(2.2) The intermediate INT-B1 undergoes a hydrogenation reduction reaction to generate the intermediate INT-B2; the reduction reaction is carried out under the catalysis of palladium/carbon; the reaction temperature is generally room temperature; the reaction solvent includes a protic solvent and aprotic solvent, such as but not limited to methanol, ethanol, ethyl acetate, THF, etc.

(2.3) The intermediate INT-B2 is deprotected from the THP protecting group to generate a compound of the general formula (II-e). The deprotection reaction of the hydroxyl is usually carried out under the action of an acidic reagent; the acidic reagent is for example but not limited to trifluoroacetic acid, hydrogen chloride, and p-toluenesulfonic acid.

Those skilled in the art can adjust the reaction conditions appropriately according to literature data or actual conditions encountered during the synthesis process.

It should be understood that within the scope of the present invention, the above-mentioned technical features of the present invention and the technical features specifically described below (such as examples) can be combined with each other to form a new or preferred technical solution. Each feature disclosed in the specification can be replaced by any alternative feature that provides the same, equal or similar purpose. Due to space limitations, they will not be described one by one here.

### Brief description of the drawings

Figure 1 shows the effect of compound E21 on TGF-β-induced hepatic stellate cell activation (α-SMA labeling); (A) images under a microscope; (B) statistics of the proportion of α-SMA-positive cells; **, $p < 0.01$; Mean ± SEM.

Figure 2 shows the drug concentrations distributed in various tissues of rats 2 hours after oral administration of 10 mg/kg compound E68; Mean ± SD, N = 3.

Figure 3 shows the difference in the number of marbles buried by mice before and after oral administration of Fluoxetine (10 mg/kg), HC-070 (1 mg/kg) and E68 (0.1, 0.3, 1 mg/kg) in the marble burying test; ns, $p > 0.05$; *, $p < 0.05$; **, $p < 0.01$; Mean ± SEM, N = 12-13.

Figure 4 shows the difference in immobility time in the tail suspension box before and after oral administration of Fluoxetine (10 mg/kg), HC-070 (1 mg/kg) and E68 (0.1, 0.3, 1 mg/kg) to mice in the tail suspension test; ns, $p > 0.05$; *, $p < 0.05$; **, $p < 0.01$; Mean ± SEM, N = 12-13.

Figure 5 shows the difference in the number of marbles buried by mice before and after oral administration of HC-070 (0.1, 0.3 mg/kg) and E68 (0.03, 0.1 mg/kg) in the marble burying test; ns, $p > 0.05$; **, $p < 0.01$; Mean ± SEM, N = 12-13.

Figure 6 shows the difference in immobility time in the tail suspension box before and after oral administration of HC-070 (0.1, 0.3 mg/kg) and E68 (0.03, 0.1 mg/kg) to mice in the tail suspension test; ns, $p > 0.05$; *, $p < 0.05$; **, p <

0.01; Mean ± SEM, N = 12-13.

**Detailed description of the invention**

The inventors of the present application have developed a xanthine compound through extensive and in-depth research, in which the 8-position is substituted by a "cycloalkyl". Compared with HC-070, the compound of the present invention has stronger inhibitory activity on TRPC4 and TRPC5, and weaker inhibition on hERG and TRPC3, and therefore has better selectivity. In the liver microsome stability test, the compound of the present invention also shows better metabolic stability, and therefore has better drug properties. In addition, some compounds in the present invention unexpectedly show significant TRPC5 agonist activity, rather than inhibitory activity. On this basis, the present invention has been completed.

**Term**

In the present invention, unless otherwise specified, the terms used have the general meanings well known to those skilled in the art.

In the present invention, the term "C1-C6" refers to having 1, 2, 3, 4, 5 or 6 carbon atoms, and so on. "4-8 members" refers to having 4, 5, 6, 7 or 8 ring atoms, and so on.

In the present invention, the "alkyl" is a branched or straight-chain hydrocarbyl having a specific number of carbon atoms, and representative examples include but are not limited to methyl, ethyl, n-propyl, and isopropyl.

In the present invention, the "alkoxy" refers to -O-alkyl. For example, the term "C1-C6 alkoxy" refers to a straight or branched alkoxy having 1 to 6 carbon atoms, and representative examples include but are not limited to methoxy, ethoxy, n-propoxy, isopropoxy and butoxy.

In the present invention, the "haloalkyl" and "haloalkoxy" refer to groups in which the hydrogen atoms in an "alkyl" or "alkoxy" group having a specific number of carbon atoms are partially or completely replaced by "halogen atoms".

In the present invention, the "cycloalkyl" represents a non-aromatic cyclic aliphatic hydrocarbyl with a specific number of ring carbon atoms, and "C3-C10 cycloalkyl" represents a cyclic aliphatic hydrocarbyl consisting of 3 to 10 ring carbon atoms; the "cycloalkyl" described in the present invention includes not only monocyclic aliphatic hydrocarbyls, but also fused, spiro and bridged ring systems consisting of multiple cyclic aliphatic hydrocarbons; the "cyclic hydrocarbyl" described in the present invention includes not only aliphatic hydrocarbyls with fully saturated carbon atoms (i.e. cycloalkyl), but also aliphatic hydrocarbyls with unsaturated bonds in some carbon atoms (such as cycloalkenyl), but does not include aromatic groups consisting entirely of unsaturated carbons; examples of the "cyclic hydrocarbyl" described in the present invention include but are not limited to:

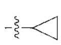, 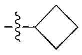, 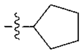, 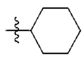, 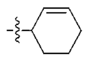, 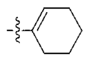, 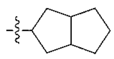,

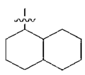, 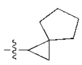, 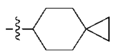, 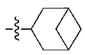, 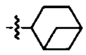, 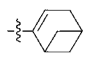, 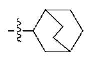, 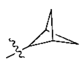, 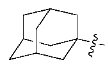.

In the present invention, "aryl" is defined as a monocyclic or bicyclic ring system consisting of a specific number of carbon atoms and obeying Hückel's rule, including phenyl and naphthyl.

In the present invention, "heteroaryl" is defined as a monocyclic and bicyclic ring system having a specific number of ring atoms and containing 1, 2, 3 or 4 heteroatoms (selected from N, O, S) and obeying the Hückel rule; examples of "heteroaryl" include but are not limited to pyridine, pyrrole, imidazole, thiophene, benzimidazole, benzothiophene, benzofuran, etc.

In the present invention, "heterocyclyl" is defined as a non-aromatic saturated or partially unsaturated monocyclic and bicyclic ring system having a specific number of ring atoms and containing 1, 2, 3 or 4 heteroatoms (selected from N, O, S); examples of "heterocyclyl" include but are not limited to oxetanyl, dioxolanyl, morpholinyl, piperazinyl, pyrrolidinyl, piperidinyl, decahydroquinolinyl, benzotetrahydrofuranyl, and the like.

In the present invention, the "halogen atom" includes fluorine, chlorine, bromine and iodine.

The term "substituted" as used herein means replacement by one or more (e.g., 2, 3, 4, or 5) groups. If it is not specified on a specific atom, it means that it can occur on any atom where the number of substituents has not yet reached saturation. When multiple substituents are selected from the same series, they can be the same or different.

The term "optionally" used in the present invention means that the defined group may be selected from a series of candidate groups or may not be selected.

"Pharmaceutically acceptable salt" of the present invention can be a salt formed by an anion and a positively

charged group on the compound of formula (I), (I-a) or (I-b). Suitable anions are chloride ion, bromide ion, iodide ion, sulfate, nitrate, phosphate, citrate, methanesulfonate, trifluoroacetate, acetate, malate, toluenesulfonate, tartrate, fumarate, glutamate, glucuronide, lactate, glutarate or maleate. Similarly, salts can be formed by a cation and a negatively charged group on the compound of formula (I). Suitable cations include sodium, potassium, magnesium, calcium and ammonium ions, such as tetramethylammonium ions.

In another preferred embodiment, the "pharmaceutically acceptable salt" refers to a salt formed by a compound of formula (I), (I-a) or (I-b) with an acid selected from the group consisting of hydrofluoric acid, hydrochloric acid, hydrobromic acid, phosphoric acid, acetic acid, oxalic acid, sulfuric acid, nitric acid, methanesulfonic acid, aminosulfonic acid, salicylic acid, trifluoromethanesulfonic acid, naphthalenesulfonic acid, maleic acid, citric acid, acetic acid, lactic acid, tartaric acid, succinic acid, oxalic acid, pyruvic acid, malic acid, glutamic acid, p-toluenesulfonic acid, naphthalenesulfonic acid, ethanesulfonic acid, naphthalene disulfonic acid, malonic acid, fumaric acid, propionic acid, oxalic acid, trifluoroacetic acid, stearic acid, pamoic acid, hydroxymaleic acid, phenylacetic acid, benzoic acid, glutamic acid, ascorbic acid, p-aminobenzenesulfonic acid, 2-acetoxybenzoic acid and isethionic acid; or a sodium salt, potassium salt, calcium salt, aluminum salt or ammonium salt formed by a compound of formula (I), (I-a) or (I-b) and an inorganic base; or a methylamine salt, ethylamine salt or ethanolamine salt formed by a compound of general formula (I), (I-a) or (I-b) and an organic base.

The general formula (I), (I-a) compound or (I-b) compound of the present invention or pharmaceutically acceptable salt thereof is distilled, crystallized or recrystallized from water or an organic solvent, and the compound may contain the solvent molecules used. In addition, different crystallization conditions may lead to different crystal forms of the compound. Therefore, compounds of the general formula (I), compounds shown in (I-a) or compounds (I-b) or pharmaceutically acceptable salts thereof, containing crystallizing solvents in different chemical dosages as well as all crystalline forms, are within the scope of the present invention.

In the present invention, the "effective therapeutic dose" means that the subjects receiving the treatment with the dose are cured, improved, effectively prevented, or the incidence of the disease or side effects is significantly reduced compared with the subjects who have not received the treatment with the dose; in addition, it also includes an effective dose that enhances normal physiological functions.

In the present invention, the term "regulator" includes inhibitors and agonists. The term "inhibitor" can also be expressed as "antagonist" or "blocker", which have the same meaning in the present invention and refer to compounds or mixtures that can be used to reduce or inhibit biological activity. The term "agonist" can also be expressed as "activator", which has the same meaning in the present invention and refers to drug molecules or mixtures that can be used to increase biological activity.

TRPC family proteins exist not only in the form of homozygous tetramers, but also often in the form of heterozygous tetramers. Taking TRPC5 as an example, it not only constitutes TRPC5:C5 homozygous channels, but also constitutes TRPC4:C5, TRPC1:C5, TRPC1:C4:C5 and other heterozygous channels. Therefore, the TRPC5 inhibitor or TRPC5 agonist of the present invention not only means to inhibit or excite TRPC5 homozygous channels, but also means to inhibit or excite TRPC5 heterozygous channels. Similarly, it has that meaning for TPRC4.

Some compounds shown in the general formula (I), (I-a), and (I-b) of the present invention have chiral centers, potential chiral centers, or unsaturated bonds, and can form various forms of stereoisomers, such as racemates, enantiomers, diastereomers, E/Z isomers, cis-trans isomers, tautomers, etc. Unless otherwise specified, in the specification and the appended claims, a given chemical formula or name is intended to cover all forms of stereoisomers, and mixtures consisting of individual isomers in different proportions, and pharmaceutically acceptable salts thereof. A person of ordinary skill in the art can use commonly used laboratory separation methods to separate the compounds containing asymmetric centers in the present invention to obtain single isomers, but this does not destroy the novelty of the compounds of the present invention.

Replacing hydrogen atom with deuterium atom to change the physical and chemical properties of compounds has become a well-known structural modification method for those skilled in the art. Unless otherwise specified, the present invention intends to include the deuterated forms of the compounds shown in general formula (I), (I-a), and (I-b) in the present invention.

The present invention will be further described below with reference to examples. It should be particularly noted that these examples are only used to illustrate the present invention and are not intended to limit the present invention in any way. All parameters and other descriptions in the examples are based on mass unless otherwise specified. The fillers used for column chromatography separation are all silica gel unless otherwise specified. The experimental methods in the following examples that do not specify specific conditions are usually carried out under conventional conditions or under conditions recommended by the manufacturer.

Unless otherwise defined, all professional and scientific terms used herein have the same meanings as those familiar to those skilled in the art. In addition, any methods and materials similar or equivalent to those described herein can be applied to the present invention.

Abbreviations: DCM: dichloromethane; DAST: diethylaminosulfur trifluoride; DMAP: 4-dimethylaminopyridine; DMF: N,N-dimethylformamide; DMSO: dimethyl sulfoxide; TFA: trifluoroacetic acid; THF: tetrahydrofuran.

## I. Preparation Example

**Example E1: 5-(4-chlorobenzyl)-6-(cyclopent-1-en-1-yl)-3-(3-hydroxypropyl)-1-methyl-1,5-dihydro-2H-pyrrolo [3,2-d]pyrimidine-2,4(3H)-dione**

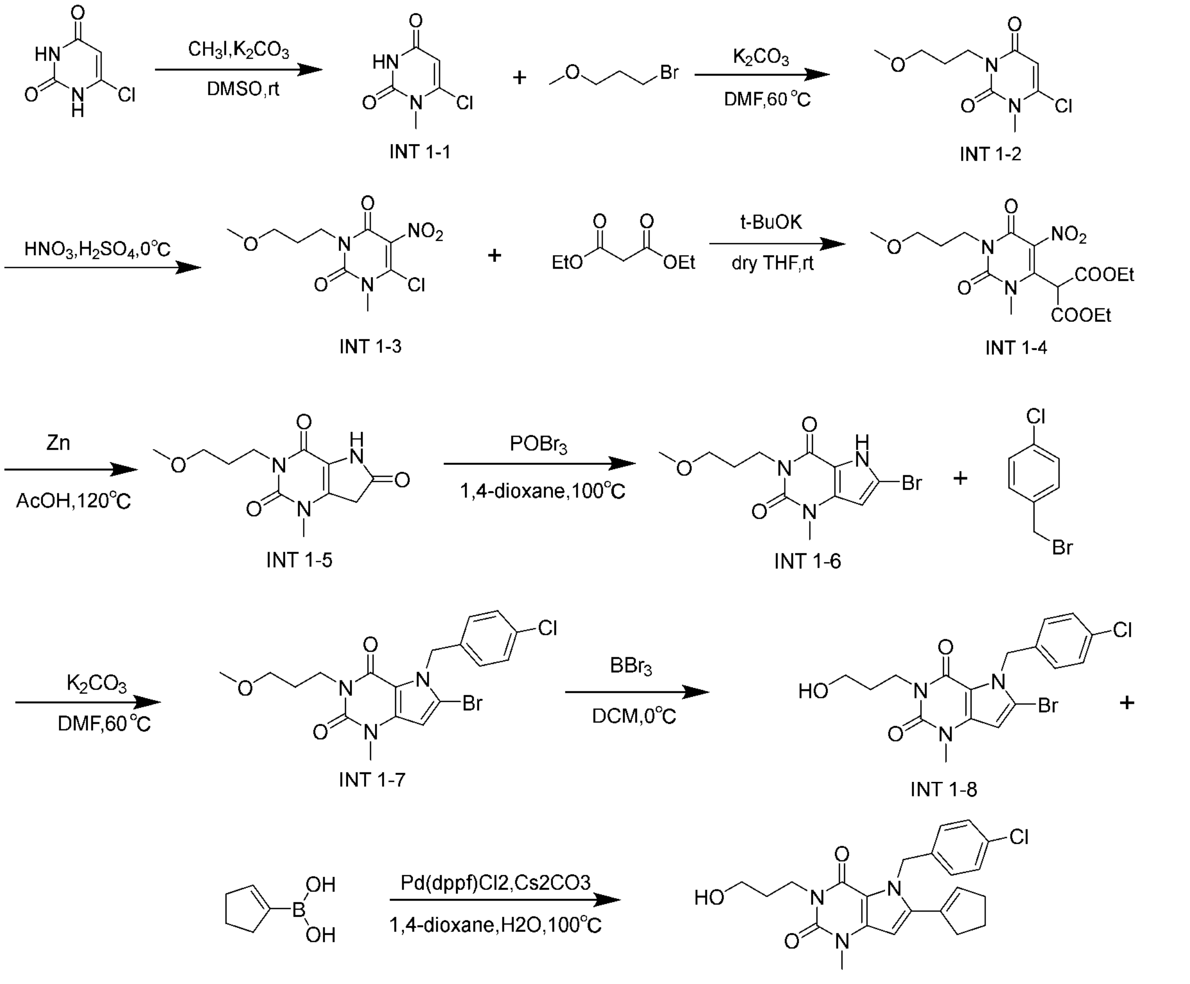

INT 1-1, INT 1-2, INT 1-3, INT 1-4, INT 1-5, INT 1-6, INT 1-7, INT 1-8

**E1**

6-Chlorouracil (10 g, 68.493 mmol) was dissolved in 40 mL DMSO, and $K_2CO_3$ (4.8 g, 34.783 mmol) and $CH_3I$ (12.7 mL, 205.48 mmol) were added to sequence, and stirred at room temperature for 3 h. TLC showed that the reaction was complete, and 50 mL $H_2O$ was added, and the mixture was filtered and washed with water to obtain 7.9 g of a white solid, namely the intermediate INT 1-1.

The intermediate INT 1-1 (5 g, 31.25 mmol) was dissolved in 10 mL DMF, $K_2CO_3$ (8.65 g, 62.5 mmol) and 3-bromopropyl methyl ether (5.35 mL, 46.875 mmol) were added, and heated at 60 °C for 3 h. TLC showed that the reaction was complete, and the organic phase was separated by EA/saturated NaCl aqueous solution, dried over anhydrous sodium sulfate, and separated by a flash silica gel column, eluted with DCM/MeOH as the mobile phase, and the target product was collected and evaporated to dryness to obtain 4 g of a jelly, namely the intermediate INT1-2.

The intermediate INT 1-2 (1 g, 4.31 mmol) was dissolved in 3 mL of concentrated sulfuric acid, stirred at 0 °C for 5 min, and 3 mL of concentrated nitric acid was slowly added dropwise, and stirred at 0 °C for 1 h. TLC showed that the reaction was complete, EA/ice water was used for partitioning, the organic layer was washed four times with saturated brine, dried over anhydrous sodium sulfate, and separated by a flash silica gel column, eluted with DCM/MeOH as the mobile phase, and the target product was collected and evaporated to dryness to obtain 800 mg of a jelly, namely the intermediate INT 1-3.

Diethyl malonate (3.24 mL, 21.66 mmol) was dissolved in 10 mL of dry 1,4-dioxane, potassium tert-butoxide (2.16 g, 19.494 mmol) was added, and the mixture was stirred at room temperature for 10 min, and then intermediate INT 1-3 (3 g, 10.83 mmol) was added, and the mixture was stirred at room temperature for 1 h. TLC showed that the reaction

was complete, 2M HCl was adjusted to acidic, EA/saturated NaCl aqueous solution was used for partitioning, and the organic phase was separated, dried over anhydrous sodium sulfate, and separated by a flash silica gel column, eluted with DCM/MeOH as the mobile phase, and the target product was collected and evaporated to dryness to obtain 3.2 g of a jelly, namely intermediate INT 1-4.

The intermediate INT 1-4 (3 g, 7.463 mmol) was dissolved in 15 mL of acetic acid, heated to 60 °C, and zinc powder (7.3 g, 111.94 mmol) was added, and the reaction was carried out in a pressure tube at 120 °C for 12 hours. TLC showed that the reaction was complete, and the crude product was evaporated under reduced pressure. Then, it was separated by a flash silica gel column, eluted with MeOH/DCM as the mobile phase, and the target product was collected and evaporated to dryness to obtain 1.8 g of a jelly, namely the intermediate INT 1-5.

The intermediate INT 1-5 (100 mg, 7.463 mmol) was dissolved in 3 mL of dry 1,4-dioxane, and phosphorus oxybromide (340 mg, 22.389 mmol) was added under nitrogen protection, and stirred at 100 °C for 1 h. TLC showed that the reaction was complete, and the mixture was partitioned with EA/saturated $NaHCO_3$ aqueous solution, and the organic phase was separated, dried over anhydrous sodium sulfate, and separated by a flash silica gel column, eluted with DCM/MeOH as the mobile phase, and the target product was collected and evaporated to dryness to obtain 90 mg of a jelly, namely the intermediate INT 1-6.

The intermediate INT 1-6 (90 mg, 0.286 mmol) was dissolved in 5 mL DMF, $K_2CO_3$ (1.73 g, 12.5 mmol) and 4-chlorobenzyl bromide (116 mg, 0.572 mmol) were added, and heated at 60 °C for 2 h. TLC showed that the reaction was complete, and the organic phase was separated by EA/saturated NaCl aqueous solution, dried over anhydrous sodium sulfate, and separated by flash silica gel chromatography, eluted with DCM/MeOH as the mobile phase, and the target product was collected and evaporated to dryness to obtain 80 mg of a white solid, namely the intermediate INT 1-7.

The intermediate INT 1-7 (40 mg, 0.091 mmol) was dissolved in 3 mL of dry DCM, and 182 μL of 1 M $BBr_3$ solution was added at 0 °C and stirred at this temperature for 1 h. TLC showed that the reaction was complete, and the crude product was evaporated under reduced pressure. Then, the product was separated by a flash silica gel column, eluted with MeOH/DCM as the mobile phase, and the target product was collected and evaporated to obtain 30 mg of a white solid, namely the intermediate INT 1-8.

INT 1-8 (25 mg, 0.049 mmol), 1-cyclopenteneboronic acid (13.7 mg, 0.122 mmol), $Pd(dppf)Cl_2$ (5.38 mg, 0.007 mmol), $Cs_2CO_3$ (48 mg, 0.147 mmol), 1,4-dioxane (10 mL) and $H_2O$ (3 mL) were added to a pressure tube, degassed with nitrogen for 3 min, the pressure tube was sealed, and heated at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 25 mg of a jelly. The crude product was purified by preparative liquid chromatography using an acetonitrile/water system as the mobile phase for elution, and the target product was collected and evaporated to dryness to obtain 15 mg of a white solid, namely Example E1. $^1H$ NMR (500 MHz, Chloroform-*d*) δ 7.31 - 7.28 (m, 2H), 6.96 (d, J = 8.5 Hz, 2H), 5.98 (s, 1H), 5.90 - 5.88 (m, 1H), 5.74 (s, 2H), 4.18 (t, *J* = 5 Hz, 2H), 3.52 (s, 3H), 3.51 - 3.49 (m, 2H), 2.76 - 2.64 (m, 2H), 2.56 - 2.51 (m, 2H), 1.99 (p, *J* = 7.5 Hz, 2H), 1.93 - 1.86(m, 2H). MS (ESI): m/z 414.1 $[M+H]^+$.

**Example E2: 5-(4-chlorobenzyl)-6-cyclopentyl-3-(3-hydroxypropyl)-1-methyl-1,5-dihydro-2H-pyrrolo[3,2-d]pyrimidine-2,4(3H)-dione**

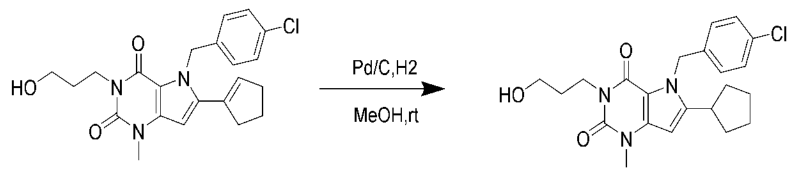


INT 1-8 E2

E1 (30 mg, 0.072 mmol) was dissolved in 3 ml of methanol, and 10 mg of Pd/C was added. After hydrogen substitution, the mixture was reacted at room temperature for 2 h. After suction filtration, the filtrate was concentrated to dryness and purified by preparative liquid chromatography column with acetonitrile/water system as mobile phase. The target product was collected and evaporated to dryness to obtain 20 mg of white solid, which is Example E2. $^1H$ NMR (500 MHz, Chloroform-*d*) δ 7.30 - 7.28 (m, 2H), 6.94 (d, *J* = 8.5 Hz, 2H), 5.89 (s, 1H), 5.66 - 5.64 (m, 2H), 4.19 - 4.16 (m, 2H), 3.58 - 3.53 (m, 2H), 3.52 - 3.49 (m, 3H), 2.96 (h, *J* = 8.1 Hz, 1H), 2.03 - 1.94 (m, 2H), 1.94 - 1.87 (m, 2H), 1.85 - 1.77 (m, 2H), 1.72 - 1.51 (m, 4H). MS (ESI): m/z 416.2 $[M+H]^+$.

**Example E3: 5-(4-chlorobenzyl)-6-(2-cyclopentylcyclopropyl)-3-(3-hydroxypropyl)-1-methyl-1,5-dihydro-2H-pyrrolo[3,2-d]pyrimidine-2,4(3H)-dione**

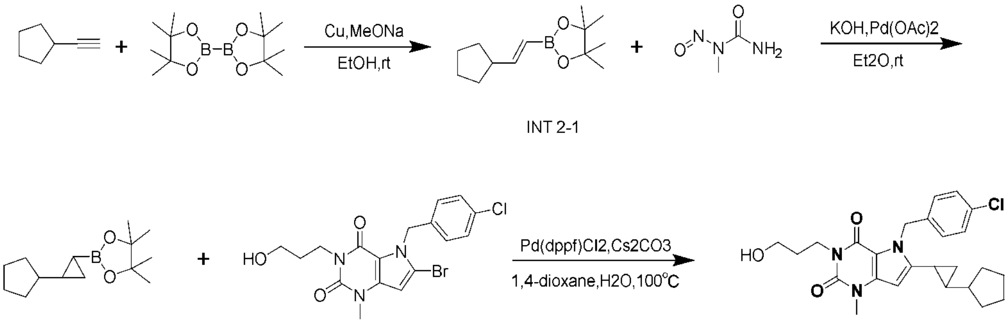


INT 2-1

INT 2-2 INT 1-8 **E3**

Bis(pinacolato)diboron (2.026g, 7.976mmol), Cu (36mg, 0.571 mmol), and MeONa (62mg, 1.142mmol) were dissolved in EtOH in sequence, and cyclopentyl acetylene (500mg, 5.319mmol) was added under nitrogen protection, and stirred at room temperature for 12h. TLC showed that the reaction was complete, and EA/saturated NH4 Cl aqueous solution was used for partitioning to separate the organic phase, which was then dried over anhydrous sodium sulfate, and separated by a flash silica gel column, eluted with PE/EA as the mobile phase, and the target product was collected and evaporated to dryness to obtain 350mg of a jelly, namely the intermediate INT 2-1.

KOH (1.5 g, 26.78 mmol) was dissolved in 3 ml $H_2O$, and 3 ml of diethyl ether was added. Methylnitrosourea (250 mg, 2.427 mmol) was added at 0 °C. After stirring for 5 min, the organic layer was transferred to a diethyl ether solution containing INT 2-1 (50 mg, 0.225 mmol) and $Pd(OAc)_2$ (20 mg, 0.089 mmol) and stirred at 0 °C for 5 min. TLC showed that the reaction was complete. The insoluble matter was removed by filtration, and the filtrate was evaporated to dryness under reduced pressure to obtain a crude product. Then, the product was separated by a rapid silica gel column, eluted with PE/EA as the mobile phase, and the target product was collected and evaporated to dryness to obtain 30 mg of a jelly, namely the intermediate INT 2-2.

INT 1-8 (25 mg, 0.049 mmol), INT 2-2 (25 mg, 0.106 mmol), $Pd(dppf)Cl_2$ (5.38 mg, 0.007 mmol), $Cs_2CO_3$ (48 mg, 0.147 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 20 mg of a jelly. The crude product was purified by preparative liquid chromatography column, eluted with acetonitrile/water system as mobile phase, and the target product was collected and evaporated to dryness to obtain 15 mg of white solid, namely Example E3. $^1H$ NMR (500 MHz, Chloroform-*d*) δ 7.31 - 7.29 (m, 2H), 7.02 (d, $J$ = 8.5 Hz, 2H), 5.83 - 5.72 (m, 1H), 5.71 - 5.52 (m, 2H), 4.21 - 4.15 (m, 2H), 3.58 - 3.51 (m, 2H), 3.49 - 3.43 (m, 3H), 1.94 - 1.87 (m, 2H), 1.78 - 1.70 (m, 2H), 1.68 - 1.61 (m, 2H), 1.59 - 1.51 (m, 2H), 1.49 (dt, $J$ = 9.1, 4.8 Hz, 1H), 1.41 (p, $J$ = 8.0 Hz, 1H), 1.34 - 1.19 (m, 2H), 1.15 - 1.00 (m, 1H), 0.93 - 0.84 (m, 2H). MS (ESI): m/z 456.2 $[M+H]^+$.

**Example E4: 7-(4-chlorobenzyl)-8-(2-cyclopentylcyclopropyl)-1-(3-hydroxypropyl)-3-methyl-3,7-dihydro-1H-purine-2,6-dione**

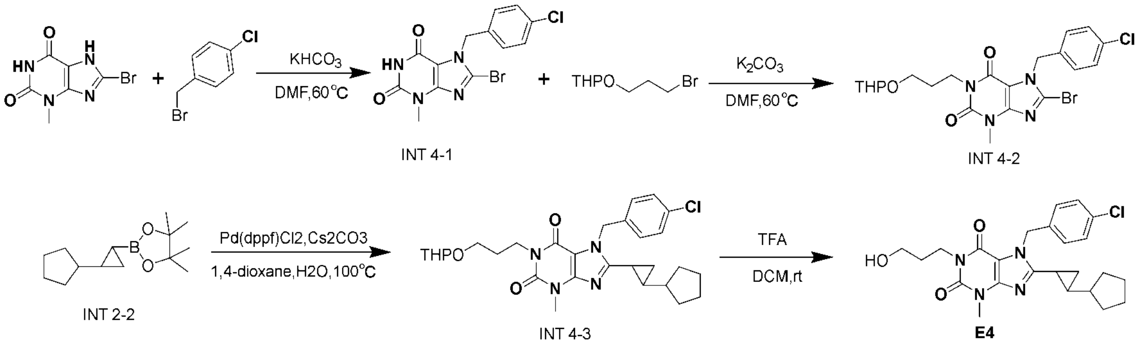


8-Bromo-3-methyl-3,7-dihydro-purine-2,6-dione (10 g, 40.816 mmol) was dissolved in 30 ml DMF, $KHCO_3$ (6.18 g, 61.8 mmol) and 4-chlorobenzyl bromide (8.37 g, 40.829 mmol) were added, and stirred at 60 °C for 2 h. TLC showed that the reaction was complete, 100 ml $H_2O$ was added, and the mixture was filtered and dried to obtain 14.1 g of a white solid, namely the intermediate INT 4-1.

INT 4-1 (14.1 g, 38.3 mmol) was dissolved in 30 ml DMF, $K_2CO_3$ (10.6 g, 76.8 mmol) and 2-(3-bromopropoxy) tetrahydro-2H-pyran (7.8 ml, 45.978 mmol) were added, and stirred at 60 °C for 2 h. When TLC showed that the reaction was complete, 100 ml $H_2O$ was added, and the mixture was filtered and dried to obtain 20.3 g of a white solid, which was the intermediate INT 4-2.

INT 4-2 (100 mg, 0.196 mmol), INT 2-2 (92.5 mg, 0.392 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 80 mg of a jelly, namely the intermediate INT4-3.

INT 4-3 (80 mg, 0.148 mmol) was dissolved in 2 mL DCM, and then 0.4 mL TFA was added and stirred at room temperature for 1 h. TLC showed that the reaction was complete. The solvent was evaporated under reduced pressure, and the residue was purified by preparative liquid chromatography column, eluted with acetonitrile/water system as mobile phase, and the target product was collected and evaporated to dryness to obtain 50 mg of white solid, namely Example E4. $^1$H NMR (500 MHz, Chloroform-*d*) δ 7.35 (d, J = 8.5 Hz, 2H), 7.19 (d, J = 8.5 Hz, 2H), 5.61 (d, *J* = 4.5 Hz, 2H), 4.19 (t, *J* = 5 Hz, 2H), 3.55 (s, 3H), 3.52 (t, *J* = 5.5 Hz, 2H), 2.08 - 1.99 (m, 1H), 1.93 - 1.86 (m, 2H), 1.81 - 1.72 (m, 1H), 1.71 - 1.60 (m, 4H), 1.58 - 1.51 (m, 2H), 1.50 - 1.39 (m, 2H), 1.25 - 1.16 (m, 1H), 1.03 - 0.98 (m, 1H), 0.95 - 0.88 (m, 1H). MS (ESI): m/z 457.2 [M+H]$^+$.

**Example E5: 5-(4-chlorobenzyl)-3-(3-hydroxypropyl)-1-methyl-6-(4-phenylcyclohexyl)-1,5-dihydro-2H-pyrrolo [3,2-d]pyrimidine-2,4(3H)-dione**

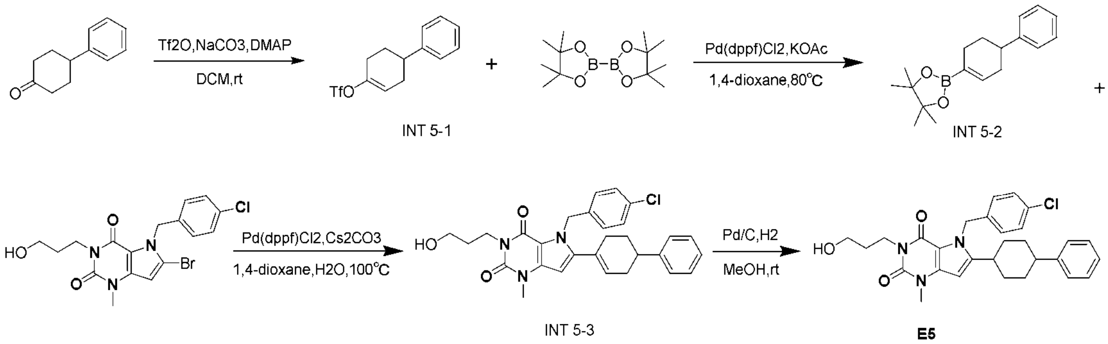


4-Phenylcyclohexanone (870 mg, 5 mmol) was dissolved in ultra-dry DCM, $NaCO_3$ was added and stirred for 10 min, $Tf_2O$ (1.55 g, 5.5 mmol) was added dropwise at 0°C, and stirred at room temperature for 3 h. TLC showed that the reaction was complete. 5 ml of MeOH was added, and the reaction solution was evaporated to dryness under reduced pressure to obtain a crude product. Then, the product was separated by a flash silica gel column, eluted with PE/EA as the mobile phase, and the target product was collected and evaporated to dryness to obtain 1 g of a colorless oily liquid, namely the intermediate INT 5-1.

INT 5-1 (300 mg, 0.98 mmol), bis(pinacolato)diboron (498 mg, 1.96 mmol), Pd(dppf)$Cl_2$ (71 mg, 0.097 mmol), KOAc (288 mg, 2.939 mmol), and 1,4-dioxane (10 mL) were added to a pressure tube, degassed with nitrogen for 3 min, the pressure tube was sealed, and the mixture was heated and stirred at 80 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with PE/EA as the mobile phase, and the target product was collected and evaporated to dryness to obtain 260 mg of a jelly, which is the intermediate INT 5-2.

INT 1-8 (25 mg, 0.049 mmol), INT 5-2 (25 mg, 0.088 mmol), Pd(dppf)$Cl_2$ (5.38 mg, 0.007 mmol), $Cs_2CO_3$ (48 mg, 0.147 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 20 mg of a jelly, which is the intermediate INT 5-3.

INT 5-3 (20 mg, 0.04 mmol) was dissolved in 3 ml methanol, 5 mg Pd/C was added, and the mixture was reacted at room temperature for 2 h after hydrogen substitution. After filtration, the filtrate was concentrated to dryness, purified by preparative liquid chromatography column and eluted with acetonitrile/water system as mobile phase. The target product was collected and evaporated to dryness to obtain 15 mg white solid, i.e. Example E5. $^1$H NMR (500 MHz, Chloroform-d) δ 7.38 - 7.34 (m, 1H), 7.34 - 7.29 (m, 4H), 7.27 - 7.20 (m, 2H), 7.01 - 6.90 (m, 2H), 5.93 - 5.89 (m, 1H), 5.71 - 5.64 (m, 2H), 4.23 - 4.13 (m, 2H), 3.54 - 3.51 (m, 2H), 3.49 (s, 3H), 2.94 - 2.86 (m, 1H), 2.68 - 2.56 (m, 1H), 2.14 - 1.98 (m, 2H), 1.96 - 1.81 (m, 4H), 1.81 - 1.74 (m, 2H), 1.70 - 1.62 (m, 2H). MS (ESI): m/z 506.2 $[M+H]^+$.

**Example E6: 7-(4-chlorobenzyl)-1-(3-hydroxypropyl)-3-methyl-8-(1,2,3,6-tetrahydro-[1,1'-biphenyl]-4-yl)-3,7-dihydro-1H-purine-2,6-dione**

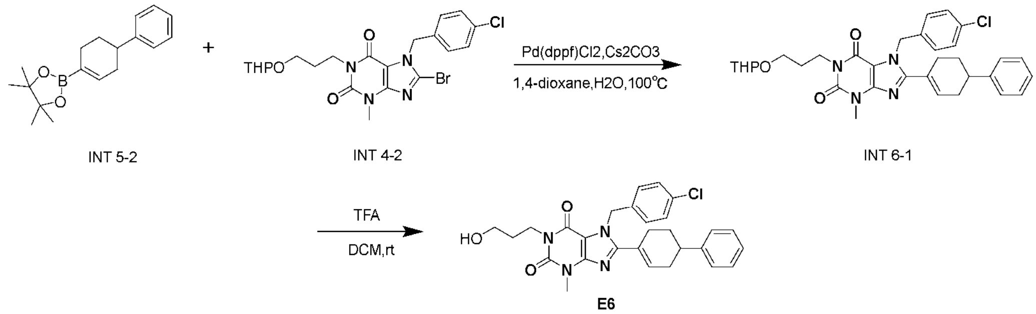


INT 5-2 INT 4-2 INT 6-1

E6

INT 4-2 (100 mg, 0.196 mmol), INT 5-2 (111.3 mg, 0.392 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 100 mg of a jelly, which is the intermediate INT 6-1.

INT 6-1 (100 mg, 0.17 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which wad then evaporated to dryness to obtain 80 mg of a white solid, i.e., Example E6. $^1$H NMR (500 MHz, Chloroform-*d*) δ 7.40 - 7.32 (m, 3H), 7.30 - 7.29 (m, 2H), 7.27 - 7.25 (m, 2H), 7.09 (d, *J*= 8.0 Hz, 2H), 6.24-6.20 (m, 1H), 5.62 (s, 2H), 4.23 - 4.16 (m, 2H), 3.66 - 3.63 (m, 3H), 3.56 - 3.52 (m, 2H), 2.92 - 2.85 (m, 1H), 2.66 - 2.46 (m, 3H), 2.42 - 2.33 (m, 1H), 2.19 - 2.08 (m, 1H), 2.02-1.92 (m, 3H). MS (ESI): m/z 505.2 $[M+H]^+$.

**Example E7: 7-(4-chlorobenzyl)-1-(3-hydroxypropyl)-8-(4-isopropoxycyclohexyl)-3-methyl-3,7-dihydro-1H-purine-2,6-dione**

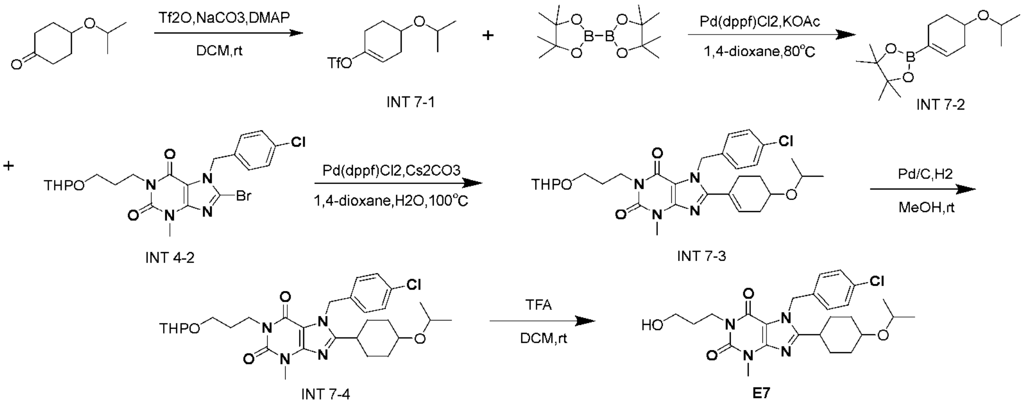


4-isopropoxycyclohexanone (250 mg, 1.6 mmol) was dissolved in ultra-dry DCM, $NaCO_3$ was added and stirred for 10 min, $Tf_2O$ (497 mg, 1.76 mmol) was added dropwise at 0°C, and stirred at room temperature for 3 h. TLC showed that the reaction was complete. 5 ml of MeOH was added, and the reaction solution was evaporated to dryness under reduced pressure to obtain a crude product. Then, the product was separated by a flash silica gel column, eluted with PE/EA as the mobile phase, and the target product was collected and evaporated to dryness to obtain 200 mg of a colorless oily liquid, namely the intermediate INT 7-1.

INT 7-1 (190 mg, 0.66 mmol), bis(pinacolato)diboron (335 mg, 1.319 mmol), Pd(dppf)$Cl_2$ (50 mg, 0.068 mmol), KOAc (241 mg, 1.975 mmol), and 1,4-dioxane (10 mL) were added to a pressure tube, degassed with nitrogen for 3 min, the pressure tube was sealed, and the mixture was heated and stirred at 80 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with PE/EA as the mobile phase, and the target product was collected and evaporated to dryness to obtain 150 mg of a jelly, which is the intermediate INT 7-2.

INT 4-2 (100 mg, 0.196 mmol), INT 7-2 (104.3 mg, 0.392 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 120 mg of a jelly, which is the intermediate INT 7-3.

INT 7-3 (100 mg, 0.175 mmol) was dissolved in 5 ml methanol, 20 mg Pd/C was added, and the mixture was reacted at room temperature for 2 h after hydrogen substitution. After suction filtration, the filtrate was concentrated to dryness to obtain 80 mg of a jelly, which was the intermediate INT 7-4.

INT 7-4 (80 mg, 0.14 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which wad then evaporated to dryness to obtain 55 mg of a white solid, i.e., Example E7. $^1$H NMR (500 MHz, DMSO-$d_6$) δ 7.42 (d, J = 8.5 Hz, 2H), 7.21 (d, J = 8.5 Hz, 2H), 5.60 (s, 2H), 4.49 - 4.45 (m, 1H), 3.95 - 3.85 (m, 2H), 3.64 - 3.57 (m, 2H), 3.50-3.43 (m, 4H), 2.97 - 2.80 (m, 1H), 1.87 - 1.73 (m, 4H), 1.72 - 1.63 (m, 2H), 1.50 - 1.40 (m, 2H), 1.35 - 1.28 (m, 2H), 1.08 (d, *J* = 6.1 Hz, 6H). MS (ESI): m/z 489.2 $[M+H]^+$.

**Example E8: 7-(4-chlorobenzyl)-1-(3-hydroxypropyl)-8-(4-isopropoxycyclohex-1-en-1-yl)-3-methyl-3,7-dihydro-1H-purine-2,6-dione**

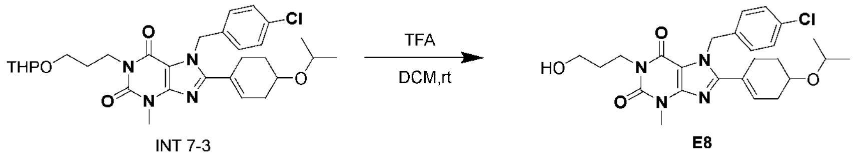


INT 7-3 (40 mg, 0.07 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect

the target product, which was then evaporated to dryness to obtain 25 mg of a white solid, i.e., Example E8. $^1H$ NMR (500 MHz, Chloroform-*d*) δ 7.32 (d, J = 8.5 Hz, 2H), 7.15 (d, J = 8.5 Hz, 2H), 6.03 - 5.99 (m, 1H), 5.58 (s, 2H), 4.20 (t, *J* = 6.0 Hz, 2H), 3.84 - 3.69 (m, 2H), 3.62 (s, 3H), 3.56 - 3.50 (m, 2H), 3.50 - 3.45 (m, 1H), 2.62 - 2.37 (m, 3H), 2.34 - 2.16 (m, 1H), 2.01 - 1.88 (m, 3H), 1.86 - 1.78 (m, 1H), 1.23 - 1.18 (m, 6H). MS (ESI): m/z 487.2 $[M+H]^+$.

**Example E9: 8-([1,1'-bis(cyclopropan)]-2-yl)-7-(4-chlorobenzyl)-1-(3-hydroxypropyl)-3-methyl-3,7-dihydro-1H-purine-2,6-dione**

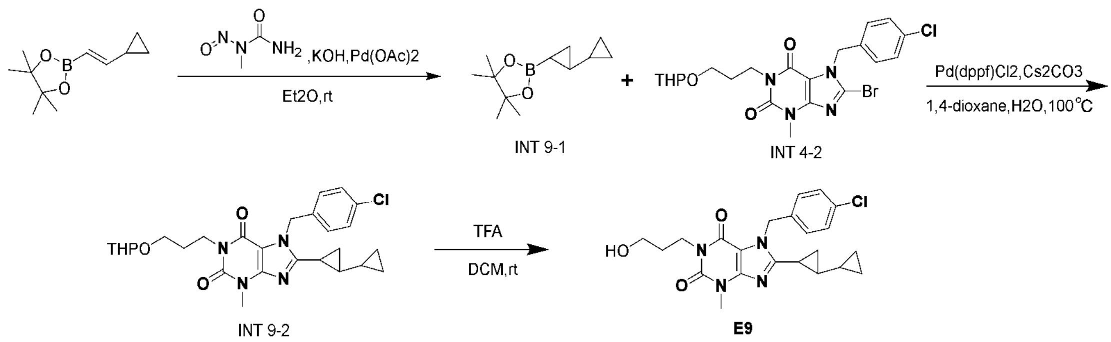


INT 9-1 INT 4-2 INT 9-2 **E9**

KOH (1.5 g, 26.78 mmol) was dissolved in 3 ml $H_2O$, and 3 ml diethyl ether was added. Methylnitrosourea (250 mg, 2.427 mmol) was added at 0 °C. After stirring for 5 min, the organic layer was transferred to a solution of (E)-2-cyclopropylvinylboronic acid pinacol ester (44 mg, 0.225 mmol) and $Pd(OAc)_2$ (20 mg, 0.089 mmol) in diethyl ether and then stirred at 0 °C for 5 min. TLC showed that the reaction was complete. The insoluble matter was removed by filtration, and the filtrate was evaporated to dryness under reduced pressure to obtain a crude product. Then, the product was separated by a rapid silica gel column, eluted with PE/EA as the mobile phase, and the target product was collected and evaporated to dryness to obtain 35 mg of a jelly, namely the intermediate INT 9-1.

INT 4-2 (100 mg, 0.196 mmol), INT 9-1 (81.5 mg, 0.392 mmol), $Pd(dppf)Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 100 mg of a jelly, which is the intermediate INT 9-2.

INT 9-2 (100 mg, 0.195 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography column, eluted with acetonitrile/water system as mobile phase, and the target product was collected and evaporated to dryness to obtain 70 mg of white solid, namely Example E9. $^1H$ NMR (500 MHz, Chloroform-d) δ 7.35 (d, J = 8.5 Hz, 2H), 7.24 (d, J = 8.5 Hz, 2H), 5.71 - 5.50 (m, 2H), 4.24 - 4.13 (m, 2H), 3.62 - 3.55 (m, 1H), 3.54 (s, 3H), 3.53 - 3.49 (m, 2H), 1.93 - 1.86(m, 2H), 1.66 (dt, J = 8.8, 4.7 Hz, 1H), 1.52-1.47 (m, 1H), 1.37 (dt, J = 9.1, 4.7 Hz, 1H), 1.02 - 0.88 (m, 1H), 0.86 - 0.79 (m, 1H), 0.57 - 0.47 (m, 1H), 0.46 - 0.35 (m, 1H), 0.22 - 0.06 (m, 2H).MS (ESI): m/z 429.2 $[M+H]^+$.

**Example E10: 7-(4-chlorobenzyl)-8-(4,4-dimethylcyclohexyl)-1-(3-hydroxypropyl)-3-methyl-3,7-dihydro-1H-purine-2,6-dione**

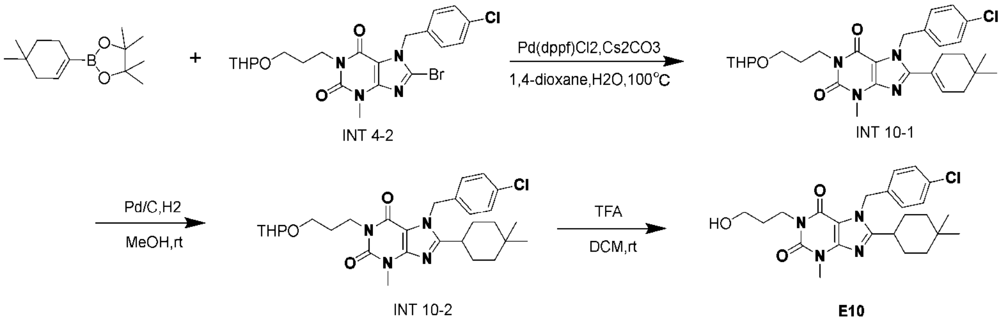


INT 4-2 INT 10-1 INT 10-2 E10

INT 4-2 (100 mg, 0.196 mmol), 4,4-(dimethylcyclohexene-1-yl)boronic acid pinacol ester (92.5 mg, 0.392 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 100 mg of a jelly, that is, the intermediate INT 10-1.

INT 10-1 (100 mg, 0.185 mmol) was dissolved in 5 ml methanol, 20 mg Pd/C was added, and the mixture was reacted at room temperature for 2 h after hydrogen substitution. After filtration, the filtrate was concentrated to dryness to obtain 80 mg of a jelly, which was the intermediate INT 10-2.

INT 7-4 (80 mg, 0.15 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 60 mg of a white solid, i.e., Example E10. $^1$H NMR (500 MHz, Chloroform-*d*) δ 7.34 (d, J = 8.5 Hz, 2H), 7.12 (d, J = 8.5 Hz, 2H), 5.54 (s, 2H), 4.20 (t, *J* = 6.0 Hz, 2H), 3.63 (s, 3H), 3.58 - 3.51 (m, 2H), 2.59 (tt, *J* = 11.9, 3.5 Hz, 1H), 1.95 - 1.83 (m, 4H), 1.51 (td, *J* = 7.5, 3.6 Hz, 4H), 1.30 - 1.19 (m, 2H), 1.02 (s, 3H), 0.97 (s, 3H). MS (ESI): m/z 459.2 [M+H]$^+$.

**Example E11: 7-(4-chlorobenzyl)-8-(4,4-dimethylcyclohex-1-en-1-yl)-1-(3-hydroxypropyl)-3-methyl-3,7-dihydro-1H-purine-2,6-dione**

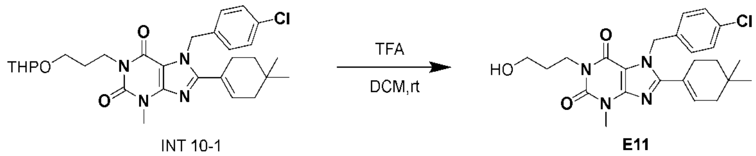


INT 10-1 E11

INT 7-3 (50 mg, 0.093 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 25 mg of a white solid, i.e., Example E11. $^1$H NMR (500 MHz, Chloroform-*d*) δ 7.32 (d, J = 8.5 Hz, 2H), 7.05 (d, J = 8.5 Hz, 2H), 6.05 - 6.02 (m, 1H), 5.59 (s, 2H), 4.23 - 4.14 (m, 2H), 3.64 (s, 3H), 3.52 (t, J = 5.5 Hz, 2H), 2.42 (dt, J = 6.3, 3.7 Hz, 2H), 2.02 - 1.99 (m, 2H), 1.93 - 1.86(m, 2H), 1.53 (t, J = 6.4 Hz, 2H), 0.98 (s, 6H). MS (ESI): m/z 457.2 [M+H]$^+$.

**Example E12: 7-(4-chlorobenzyl)-1-(3-hydroxypropyl)-3-methyl-8-(4-(trifluoromethyl)cyclohexyl)-3,7-dihydro-1H-purine-2,6-dione**

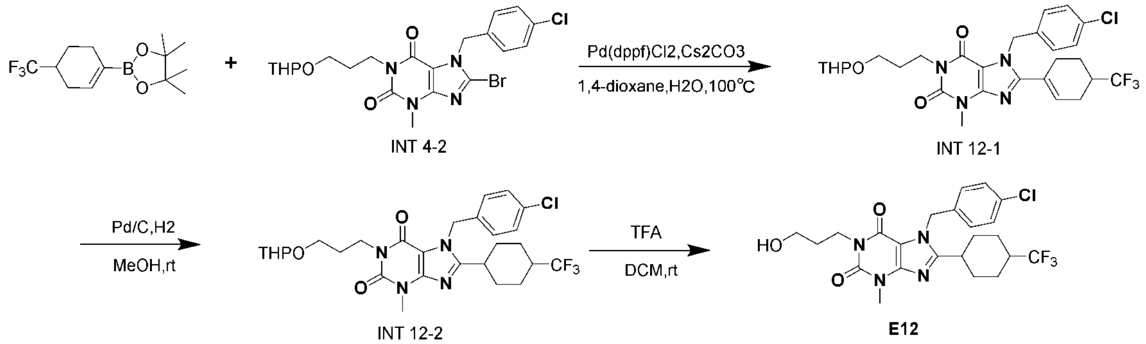


INT 4-2 INT 12-1 INT 12-2 **E12**

The synthesis method of Example E12 was the same as that of Example E10, except that 4-(trifluoromethyl)-1-cyclohexene-1-boronic acid pinacol ester was used as the raw material instead of 4,4-(dimethylcyclohexene-1-yl)boronic acid pinacol ester. $^{1}$H NMR (500 MHz, Chloroform-*d*) δ 7.34 (d, J = 8.5 Hz, 2H), 7.08 (d, J = 8.5 Hz, 2H), 5.56 (s, 2H), 4.21 (t, J = 6.0 Hz, 2H), 3.63 (s, 3H), 3.56 - 3.52 (m, 2H), 3.52 - 3.47 (m, 1H), 3.07 - 3.02 (m, 1H), 2.29 - 2.12 (m, 3H), 1.95 - 1.87 (m, 4H), 1.76 - 1.65 (m, 4H). MS (ESI): m/z 499.2 [M+H]$^+$.

**Example E13: 7-(4-chlorobenzyl)-1-(3-hydroxypropyl)-3-methyl-8-(1,4-dioxacyclo[4.5]dec-8-yl)-3,7-dihydro-1H-purine-2,6-dione**

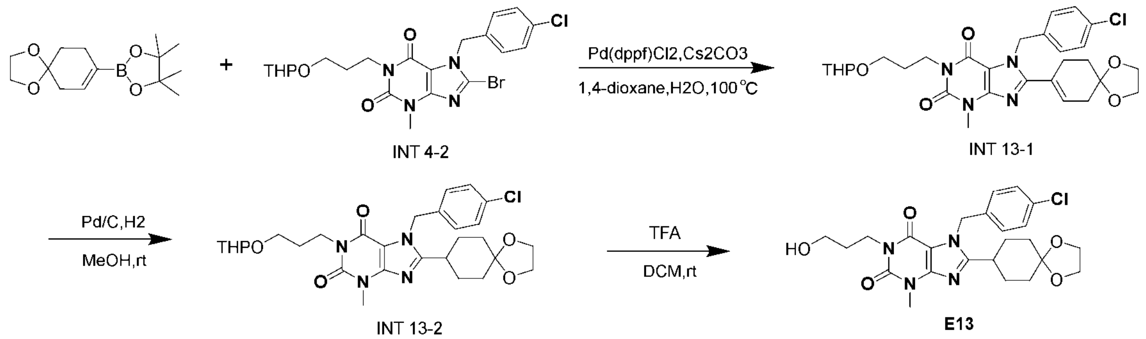


INT 4-2 INT 13-1 INT 13-2 **E13**

The synthesis method of Example E13 was the same as that of Example E10, except that 1,4-dioxa-spiro[4,5] dec-7-ene-8-boronic acid pinacol ester was used as the starting material instead of 4,4-(dimethylcyclohexen-1-yl)boronic acid pinacol ester. $^{1}$H NMR (500 MHz, Chloroform-*d*) δ 7.34 (d, J = 8.5 Hz, 2H), 7.11 (d, J = 8.5 Hz, 2H), 5.56 (s, 2H), 4.20 (t, J = 6.0 Hz, 2H), 4.00 (d, J = 1.7 Hz, 4H), 3.61 (s, 3H), 3.53 (t, J = 5.5 Hz, 2H), 2.72 (tt, J = 11.6, 3.6 Hz, 1H), 2.04 (qd, J = 13.4, 3.6 Hz, 2H), 1.94 - 1.85 (m, 4H), 1.73 - 1.66 (m, 2H), 1.58 (td, J = 13.4, 4.1 Hz, 2H). MS (ESI): m/z 489.2 [M+H]$^+$.

**Example E14: 8-([1,1'-bis(cyclopropan)]-2-yl)-7-(4-chlorobenzyl)-1-(3-hydroxypropyl)-3-isopropyl-3,7-dihydro-1H-purine-2,6-dione**

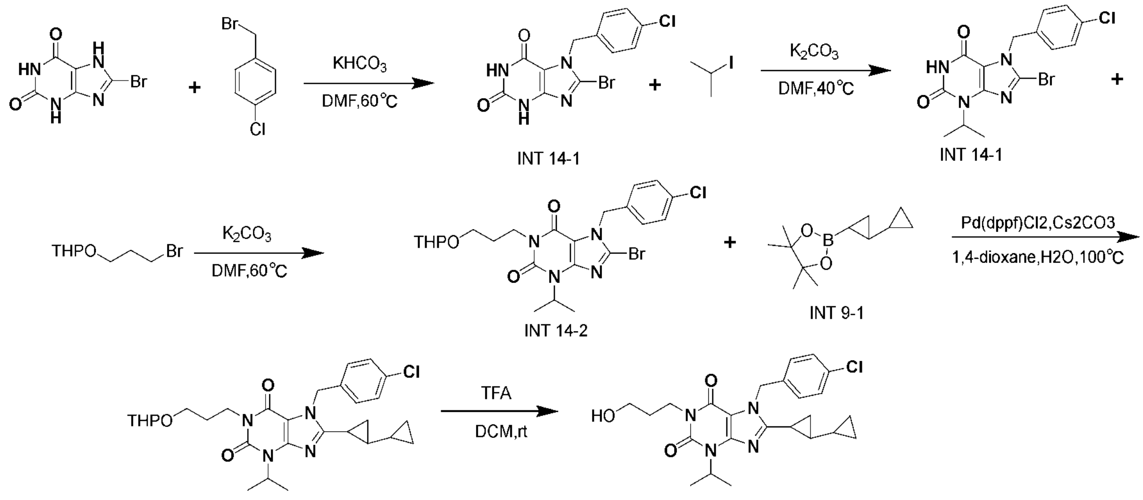


INT 14-1 INT 14-1 INT 14-2 INT 9-1 INT 14-3 E14

8-Bromoxanthine (2 g, 8.658 mmol) was dissolved in 10 ml DMF, $KHCO_3$ (1.298 g, 12.987 mmol) and 4-chlorobenzyl bromide (1.77 g, 8.658 mmol) were added, and stirred at 60 °C for 2 h. When TLC showed that the reaction was complete, 10 ml $H_2O$ was added, and the mixture was filtered and dried to obtain 2.5 g of a white solid, namely the intermediate INT 14-1.

INT 14-1 (1 g, 2.825 mmol) was dissolved in 10 ml DMF, $K_2CO_3$ (428 mg, 3.1 mmol) and 2-iodopropane (480 mg, 2.825 mmol) were added, and stirred at 40 °C for 2 h. TLC showed that the reaction was complete, 10 ml $H_2O$ was added, and the mixture was filtered and dried to obtain 400 mg of a white solid, namely the intermediate INT 14-2.

INT 14-2 (350 mg, 0.883 mmol) was dissolved in 5 ml DMF, $K_2CO_3$ (244 mg, 1.768 mmol), 2-(3-bromopropoxy) tetrahydro-2H-pyran (236.5 mg, 1.06 mmol) were added, and stirred at 60 °C for 2 h. TLC showed that the reaction was complete. The reaction solution was poured into a saturated NaCl aqueous solution, extracted with ethyl acetate 3 times, the organic phases were combined, and then washed with a saturated NaCl aqueous solution 3 times. After dried with anhydrous $MgSO_4$, it was separated by a flash silica gel column, and eluted with MeOH/DCM as the mobile phase. The target product was collected to obtain 250 mg of a colorless oily liquid to obtain the intermediate INT 14-3.

INT 14-3 (100 mg, 0.185 mmol), INT 9-1 (81.5 mg, 0.392 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 60 mg of a jelly, which is the intermediate INT 14-4.

INT 14-4 (60 mg, 0.111 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography column, eluted with acetonitrile/water system as mobile phase, and the target product was collected and evaporated to dryness to obtain 30 mg of white solid, namely Example E14. $^1H$ NMR (500 MHz, Chloroform-d) δ 7.36 (d, J = 8.5 Hz, 2H), 7.27 (d, J = 8.3 Hz, 2H), 5.73 - 5.42 (m, 2H), 5.20 - 5.10 (m, 1H), 4.18 (t, J = 5.9 Hz, 2H), 3.70 (t, J = 7.2 Hz, 1H), 3.57 - 3.48 (m, 2H), 1.91 - 1.83 (m, 2H), 1.66 (dt, J = 8.8, 4.7 Hz, 1H), 1.55 (d, J = 6.9 Hz, 6H), 1.52 - 1.47 (m, 1H), 1.36 (dt, J = 9.1, 4.7 Hz, 1H), 0.97 - 0.79 (m, 2H), 0.57 - 0.38 (m, 2H), 0.19 - 0.08 (m, 2H). MS (ESI): m/z 457.2 $[M+H]^+$.

**Example E15: 7-(4-chlorobenzyl)-8-(2-cyclopentylcyclopropyl)-1-(3-hydroxypropyl)-3-isopropyl-3,7-dihydro-1H-purine-2,6-dione**

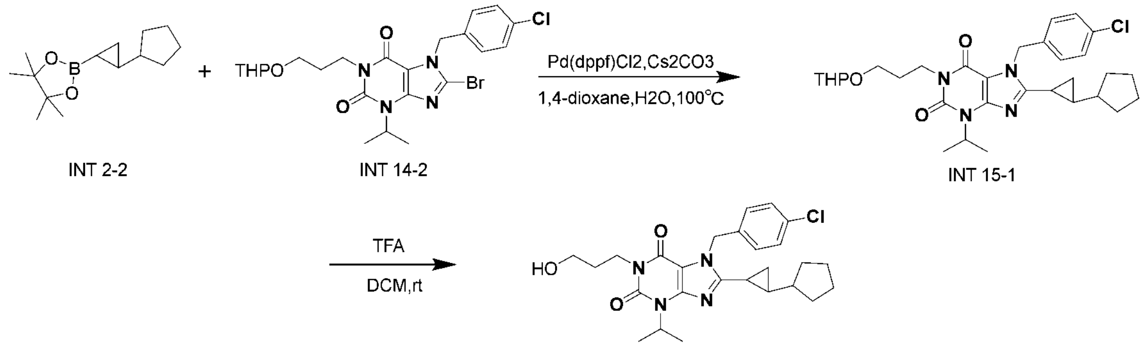


INT 2-2 INT 14-2 INT 15-1

E15

INT 14-2 (100 mg, 0.185 mmol), INT 9-1 (81.5 mg, 0.392 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 80 mg of a jelly, which is the intermediate INT 15-1.

INT 15-1 (80 mg, 0.141 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography column with acetonitrile/water system as mobile phase for elution. The target product was collected and evaporated to dryness to obtain 50 mg of white solid, namely Example E15. $^1$H NMR (500 MHz, Chloroform-*d*) δ 7.35 (d, J = 8.5 Hz, 2H), 7.23 (d, J = 8.3 Hz, 2H), 5.20 - 5.11 (m, 1H), 4.17 (t, J = 5.9 Hz, 2H), 3.70 (t, J = 7.2 Hz, 1H), 3.55 - 3.47 (m, 2H), 1.93 - 1.86(m, 2H), 1.81 - 1.74 (m, 1H), 1.71 - 1.61 (m, 5H), 1.56 (d, J = 6.8, 6H), 1.49 - 1.38 (m, 3H), 1.36 - 1.28 (m, 2H), 1.26 - 1.17 (m, 1H), 1.03 - 0.96 (m, 1H). MS (ESI): m/z 485.2 $[M+H]^+$.

**Example E16: 7-(4-chlorobenzyl)-8-cyclopentyl-1-(3-methoxypropyl)-3-methyl-3,7-dihydro-1H-purine-2,6-dione**

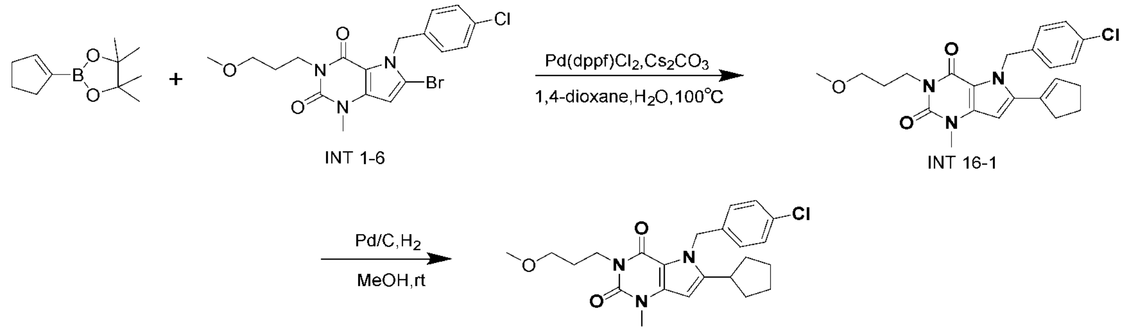


INT 1-6 INT 16-1

E16

INT 1-6 (100 mg, 0.227 mmol), 1-cyclopenteneboronic acid pinacol ester (88.1 mg, 0.454 mmol), Pd(dppf)$Cl_2$ (14.3 mg, 0.019 mmol), $Cs_2CO_3$ (191.1 mg, 0.588 mmol), 1,4-dioxane (10 mL) and $H_2O$ (3 mL) were added to a pressure tube, degassed with nitrogen for 3 min, the pressure tube was sealed, and the mixture was heated and stirred at 100 °C for 2 h. After cooling, it was diluted with ethyl acetate, the insoluble matter was filtered out, and the filtrate was evaporated to dryness under reduced pressure to obtain a crude product. Then, it was separated by a flash silica gel column, eluted with MeOH/DCM as the mobile phase, and the target product was collected and evaporated to dryness to obtain 80 mg of a jelly, namely the intermediate INT 16-1.

INT 16-1 (80 mg, 0.187 mmol) was dissolved in 5 ml methanol, 15 mg Pd/C was added, and the mixture was reacted at room temperature for 2 h after hydrogen substitution. After filtration, the filtrate was concentrated to dryness to obtain 80 mg of a colloid, which was purified by a preparative liquid chromatography column with acetonitrile/water as the mobile phase for elution. The target product was collected and evaporated to dryness to obtain 50 mg of a white solid, i.e., Example E16. $^1$H NMR (500 MHz, Chloroform-*d*) δ 7.28 (d, J = 9 Hz, 2H), 6.95 (d, *J* = 8.3 Hz, 2H), 5.84 (s, 1H), 5.68 (s, 2H), 4.09 (t, *J* = 7.65 Hz, 2H), 3.49 (s, 3H), 3.45 (t, *J* = 6.5 Hz, 2H), 3.32 (s, 3H), 2.95 (p, *J* = 8.1 Hz, 1H), 2.00 - 1.90 (m, 4H), 1.85 - 1.77 (m, 2H), 1.69 - 1.59 (m, 4H). MS (ESI): m/z 431.2 $[M+H]^+$.

**Example E17: 7-(4-chlorobenzyl)-8-(1-fluoro-3-phenylcyclobutyl)-1-(3-hydroxypropyl)-3-methyl-3,7-dihydro-1H-purine-2,6-dione**

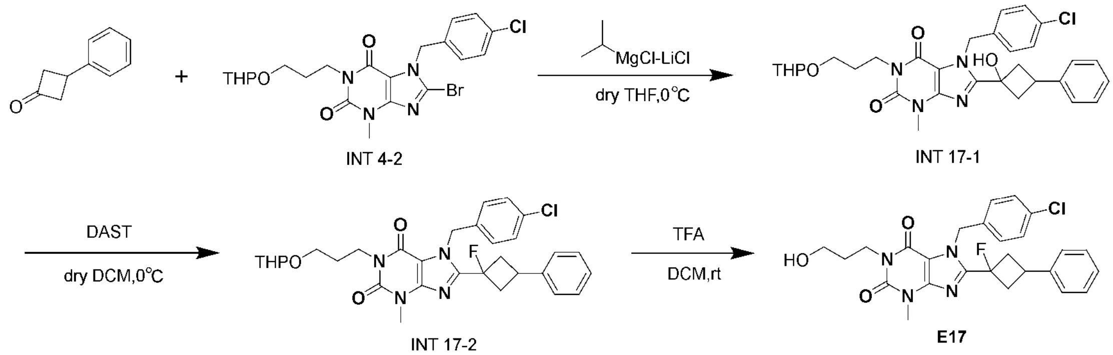

INT 4-2
INT 17-1
INT 17-2
E17

INT 4-2 (250 mg, 0.49 mmol) was dissolved in 5 ml of ultra-dry tetrahydrofuran, and isopropylmagnesium chloride lithium chloride complex (490 μL, 0.735 mmol) was added dropwise at 0 °C, stirred for 10 min, and 3-phenylcyclobutanone (130 μL, 0.98 mmol) was added, and stirred at 0 °C for 2 h. LC-MS showed that the reaction was complete, and the solvent was evaporated under reduced pressure. Then, the reaction mixture was separated by a flash silica gel column, eluted with MeOH/DCM as the mobile phase, and the target product was collected and evaporated to dryness to obtain 100 mg of a jelly, namely the intermediate INT 17-1.

Intermediate INT 17-1 (100 mg, 0.173 mmol) was dissolved in 3 ml of ultra-dry DCM, and DAST (113 μL, 0.865 mmol) was added dropwise at 0 °C., and stirred for 30 min at 0 °C. LC-MS showed that the reaction was complete, and the mixture was evaporated to dryness to obtain 80 mg of a gum, namely intermediate INT 17-2.

INT 17-2 (80 mg, 0.138 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 50 mg of a white solid, i.e., Example E17. $^1$H NMR (500 MHz, Chloroform-*d*) δ 7.41 - 7.35 (m, 3H), 7.34 - 7.30 (m, 4H), 7.21 (d, J = 8.2 Hz, 2H), 5.68 (s, 2H), 4.23 (t, J = 6.0 Hz, 2H), 3.68 (s, 3H), 3.60 - 3.52 (m, 2H), 3.27 (p, J = 8.9 Hz, 1H), 3.16 - 3.08 (m, 2H), 2.79 - 2.66 (m, 2H), 1.97 - 1.88 (m, 2H). MS (ESI): m/z 497.2 $[M+H]^+$.

**Example E18: (E)-7-(4-chlorobenzyl)-1-(3-hydroxypropyl)-3-methyl-2,6-dioxo-2,3,6,7-tetrahydro-1H-purine-8-carbaldehyde-O-(tert-butyl)oxime**

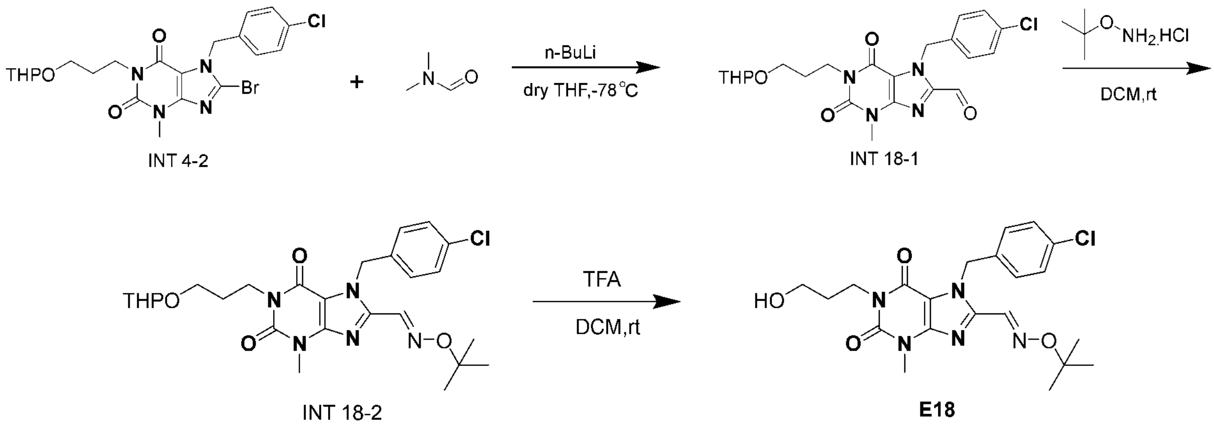

INT 4-2
INT 18-1
INT 18-2
E18

INT 4-2 (250 mg, 0.49 mmol) was dissolved in 5 ml of ultra-dry tetrahydrofuran, 2.5 M n-butyl lithium (235 μL, 0.588 mmol) was added dropwise at -78 °C, stirred for 30 min, ultra-dry DMF (57 μL, 0.735 mmol) was added, and stirred at -78 °C for 2 h. LC-MS showed that the reaction was complete, and the solvent was evaporated under reduced pressure. Then, the residue was separated by a flash silica gel column, eluted with MeOH/DCM as the mobile phase, and the target

product was collected and evaporated to dryness to obtain 80 mg of a jelly, namely the intermediate INT 18-1.

Intermediate INT 18-1 (80 mg, 0.174 mmol) and O-tert-butylhydroxylamine hydrochloride (65.6 mg, 0.522 mmol) were dissolved in 3 ml of DCM and stirred for 12 h. TLC showed that the reaction was complete, and the solvent was evaporated under reduced pressure. Then, the product was separated by flash silica gel chromatography, eluted with MeOH/DCM as the mobile phase, and the target product was collected and evaporated to dryness to obtain 40 mg of a jelly, namely, intermediate INT 18-2.

NT 17-2 (40 mg, 0.075 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 30 mg of a white solid, Example E18. $^1$H NMR (500 MHz, Chloroform-d) δ 8.22 (s, 1H), 7.38 - 7.34 (m, 2H), 7.22 (d, J = 8.5 Hz, 2H), 6.03 (s, 1H), 4.28 - 4.20 (m, 2H), 3.67 (s, 3H), 3.61 - 3.54 (m, 2H), 3.48 - 3.25 (m, 1H), 2.07 - 1.83 (m, 2H), 1.31 (s, 9H).MS (ESI): m/z 448.2 [M+H]$^+$.

**Example E19: 7-(4-chlorobenzyl)-8-(1-fluoro-4-isopropoxycyclohexyl)-1-(3-hydroxypropyl)-3-methyl-3,7-dihydro-1H-purine-2,6-dione**

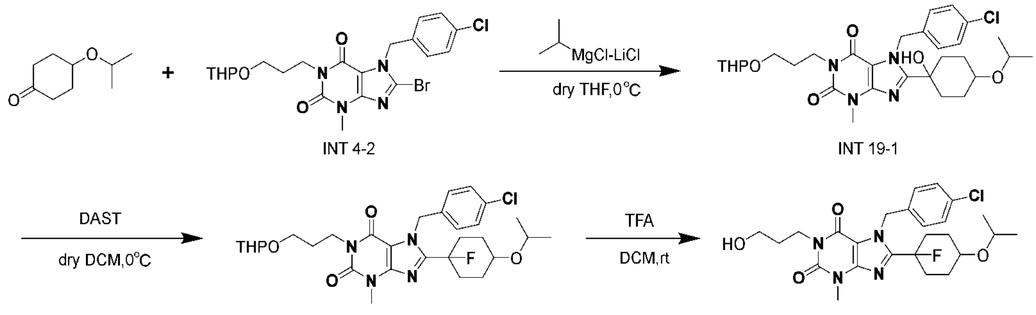


INT 4-2 INT 19-1

INT 19-2 E19

The synthesis method of Example E19 was the same as that of Example E17, except that 4-isopropoxycyclohexanone was used as the starting material instead of 3-phenylcyclobutanone. $^1$H NMR (500 MHz, Chloroform-*d*) δ 7.34 - 7.31 (m, 2H), 7.20 - 7.09 (m, 2H), 5.76 (s, 2H), 4.21 - 4.13 (m, 2H), 3.80 - 3.64 (m, 2H), 3.64 - 3.56 (m, 3H), 3.55 - 3.37 (m, 2H), 2.50 - 2.28 (m, 2H), 2.18 - 2.00 (m, 4H), 1.97 - 1.79 (m, 4H), 1.22 - 1.14 (ms, 6H). MS (ESI): m/z 507.2 [M+H]$^+$.

**Example E20: 7-(4-chlorobenzyl)-1-(3-hydroxypropyl)-8-(1H-inden-2-yl)-3-methyl-3,7-dihydro-1H-purine-2,6-dione**

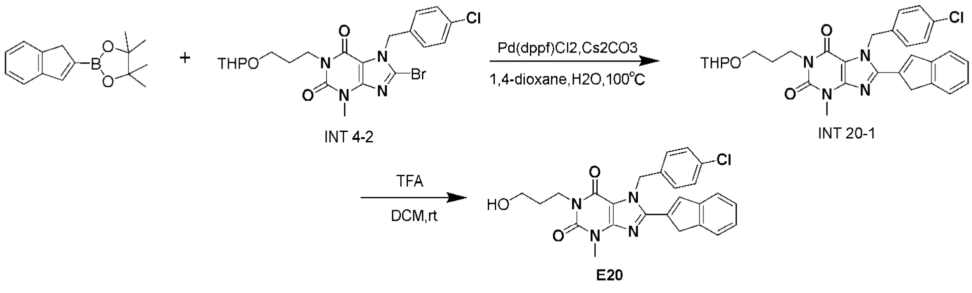


INT 4-2 INT 20-1

E20

The synthesis method of Example E20 was the same as that of Example E6, except that 1H-indene-2-boronic acid pinacol ester was used as the starting material instead of 4,4,5,5-tetramethyl-2-(1,2,3,6-tetrahydro-[1,1'-biphenyl]-4-yl)-1,3,2-dioxaborane. $^1$H NMR (500 MHz, Chloroform-d) δ 7.58 - 7.52 (m, 1H), 7.47 - 7.42 (m, 1H), 7.39 - 7.32 (m, 4H), 7.25 (s, 1H), 7.11(d, J = 8.5 Hz, 2H), 5.86 (s, 2H), 4.20 (t, *J* = 6.0 Hz, 2H), 4.02 (s, 2H), 3.70 (s, 3H), 3.54 (t, *J* = 5.4 Hz, 2H), 1.96 - 1.88 (m, 2H). MS (ESI): m/z 463.2 [M+H]$^+$.

**Example 21: 7-(4-chlorobenzyl)-8-(1-fluoro-4-(trifluoromethyl)cyclohexyl)-1-(3-hydroxypropyl)-3-methyl-3,7-dihydro-1H-purine-2,6-dione**

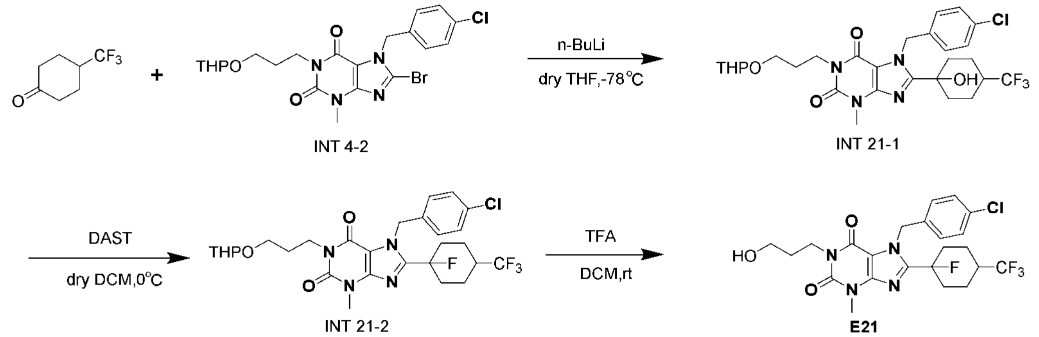


INT 4-2 (250 mg, 0.49 mmol) was dissolved in 5 ml of ultra-dry tetrahydrofuran, 2.5 M n-butyl lithium (235 μL, 0.588 mmol) was added dropwise at -78 °C, stirred for 30 min, 4-(trifluoromethyl) cyclohexanone (122 mg, 0.735 mmol) was added, and stirred at -78 °C for 2 h. LC-MS showed that the reaction was complete, and the solvent was evaporated under reduced pressure. Then, the residue was separated by a flash silica gel column, eluted with MeOH/DCM as the mobile phase, and the target product was collected and evaporated to dryness to obtain 120 mg of a jelly, namely the intermediate INT 21-1.

Intermediate INT 21-1 (120 mg, 0.201 mmol) was dissolved in 3 ml of ultra-dry DCM, and DAST (53 μL, 0.402 mmol) was added dropwise at 0 °C, and stirred for 30 min at 0 °C. LC-MS showed that the reaction was complete, and the mixture was evaporated to dryness to obtain 80 mg of a gum, namely, intermediate INT 21-2.

INT 21-2 (80 mg, 0.133 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 50 mg of a white solid, namely Example E21. $^1$H NMR (500 MHz, Chloroform-*d*, 3:4 isomer) δ 7.30/7.29 (2 × d, J = 7 Hz, 2H), 7.15/7.09 (2 × d, J = 8.2 Hz, 2H), 5.76/5.75(2 × s, 2H), 4.22 - 4.16 (m, 2H), 3.58/3.61 (2 × s, 3H), 3.56 - 3.50 (m, 2H), 3.42 - 3.24 (m, 1H), 2.46 - 2.36 (m, 1H), 2.31 - 2.17 (m, 1H), 2.17 - 2.08 (m, 1H), 2.07 - 1.85 (m, 7H), 1.82 - 1.71 (m, 1H). MS (ESI): m/z 517.2 $[M+H]^+$.

**Example E22: 7-(4-chlorobenzyl)-1-(3-hydroxypropyl)-8-(1-methoxy-4-(trifluoromethyl)cyclohexyl)-3-methyl-3,7-dihydro-1H-purine-2,6-dione**

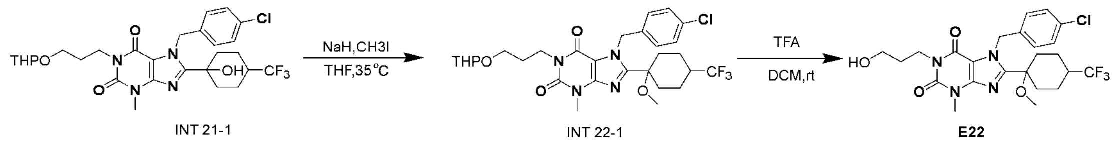


The intermediate INT 21-1 (120 mg, 0.201 mmol) was dissolved in 5 ml of ultra-dry THF, and NaH (16.08 mg, 0.402 mmol) and $CH_3I$ (38 μL, 0.603 mmol) were added, and the reaction was carried out at 35 °C for 12 h. TLC showed that the reaction was complete, and the solvent was evaporated under reduced pressure. Then, the product was separated by a flash silica gel column, eluted with MeOH/DCM as the mobile phase, and the target product was collected and evaporated to dryness to obtain 80 mg of a jelly, namely the intermediate INT 22-1.

INT 22-1 (80 mg, 0.133 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 50 mg of a white solid, Example E22. $^1$H NMR (500 MHz, Chloroform-*d*) δ 7.31 (d, J = 8.5 Hz, 2H), 6.98 (d, J = 8.5 Hz, 2H),5.87 (s, 2H), 4.21 - 4.09 (m, 2H), 3.63 (s, 3H), 3.54 - 3.49 (m, 2H), 3.32 (t, J = 7.1 Hz, 1H), 3.00 (s, 3H), 2.23 - 2.19 (m, 1H), 1.92 - 1.78 (m, 6H), 1.67 (td, J = 12.4, 3.6 Hz, 4H). MS (ESI): m/z 529.2 $[M+H]^+$.

**Example E23: 7-(4-chlorobenzyl)-8-(1-hydroxy-4-(trifluoromethyl)cyclohexyl)-1-(3-hydroxypropyl)-3-methyl-3,7-dihydro-1H-purine-2,6-dione**

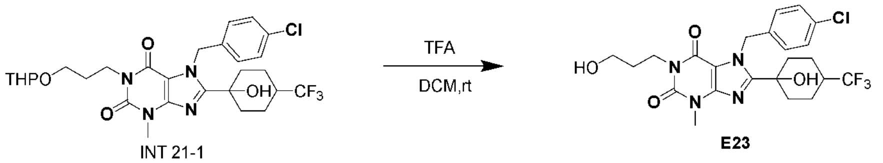

INT 21-1 E23

INT 21-1 (40 mg, 0.067 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 20 mg of a white solid, i.e., Example E23. $^1H$ NMR (500 MHz, Chloroform-*d*) δ 7.29 (d, J = 8.5 Hz, 2H), 7.01 (d, J = 8.5 Hz, 2H), 5.93 (s, 2H), 4.18 (t, J = 6.0 Hz, 2H), 3.61 (s, 3H), 3.51 - 3.46 (m, 2H), 3.38 (t, J = 7.1 Hz, 1H), 2.17 - 2.04 (m, 3H), 1.98 - 1.92 (m, 2H), 1.90 - 1.83 (m, 4H), 1.81 - 1.69 (m, 2H). MS (ESI): m/z 515.2 $[M+H]^+$.

**Example E24: 7-(4-chlorobenzyl)-8-(1-fluoro-4,4-dimethylcyclohexyl)-1-(3-hydroxypropyl)-3-methyl-3,7-dihydro-1H-purine-2,6-dione**

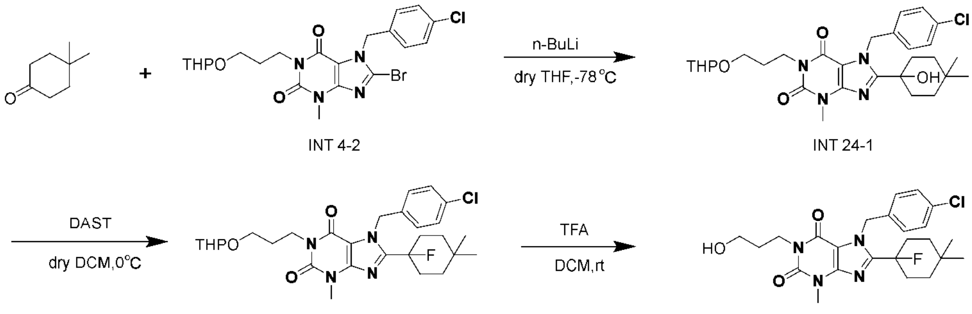

INT 4-2 INT 24-1 INT 24-2 E24

The synthesis method of Example E24 was the same as that of Example E21, except that 4,4-dimethylcyclohexanone was used as the starting material instead of 4-(trifluoromethyl)cyclohexanone. $^1H$ NMR (500 MHz, Chloroform-d) δ 7.28 (d, J = 8.5 Hz, 2H), 7.12 (d, J = 8.5 Hz, 2H), 5.76 (s, 2H), 4.19 (t, *J* = 6.0 Hz, 2H), 3.61 (s, 3H), 3.52 (t, *J* = 5.5 Hz, 2H), 2.26 (td, *J* = 14.1, 4.4 Hz, 2H), 1.93 - 1.86 (m, 4H), 1.64 (dd, *J* = 13.7, 4.0 Hz, 2H), 1.38 (d, *J = 13.7* Hz, 2H), 1.02 (d, *J* = 6.0 Hz, 6H). MS (ESI): m/z 477.2 $[M+H]^+$.

**Example E25: 7-(4-chlorobenzyl)-8-(1-hydroxy-4,4-dimethylcyclohexyl)-1-(3-hydroxypropyl)-3-methyl-3,7-dihydro-1H-purine-2,6-dione**

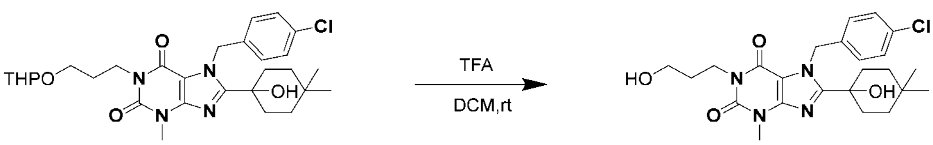

INT 24-1 E25

INT 24-1 (40 mg, 0.072 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 20 mg of a white solid, i.e., Example E25. $^1H$ NMR (500

MHz, Chloroform-*d*) δ 7.30 (d, *J* = 8 Hz, 2H), 7.04 (d, J = 8.5 Hz, 2H), 5.92 (s, 2H), 4.16 (t, *J* = 6.0 Hz, 2H), 3.62 (s, 3H), 3.54 - 3.47 (m, 2H), 3.46 - 3.41 (m, 1H), 2.23 (td, *J* = 13.7, 4.2 Hz, 2H), 1.91 - 1.83(m, 2H), 1.71 (d, *J* = 14.2 Hz,2H), 1.57 (td, *J* = 13.5, 3.9 Hz, 2H), 1.41 - 1.29 (m, 2H), 1.00 (s, 6H). MS (ESI): m/z 475.2 $[M+H]^+$.

**Example E26: 7-(4-chlorobenzyl)-1-(3-hydroxypropyl)-8-(1-methoxy-4,4-dimethylcyclohexyl)-3-methyl-3,7-dihydro-1H-purine-2,6-dione**

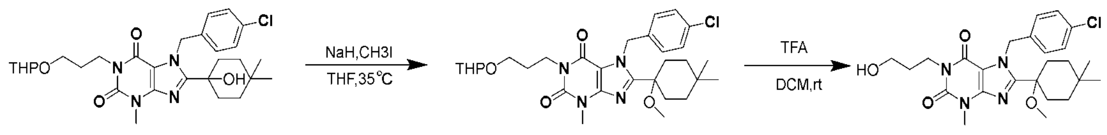


INT 24-1 INT 26-1 **E26**

The synthesis method of Example E26 was the same as that of E22, except that INT 24-1 was used as the starting material instead of INT 21-1. $^1$H NMR (500 MHz, Chloroform-*d*) δ 7.30 (d, *J* = 8.6 Hz, 2H), 6.97 (d, *J* = 9 Hz, 2H, 2H), 5.88 (s, 2H), 4.27 - 4.03 (m, 2H), 3.65 (s, 3H), 3.56 - 3.48 (m, 2H), 2.97 (s, 3H), 2.03 - 1.97 (m, 2H), 1.94 - 1.85 (m, 4H), 1.50 (td, *J* = 13.0, 3.8 Hz, 2H), 1.31 - 1.23 (m, 2H), 0.97 (d, *J* = 3.7 Hz, 6H). MS (ESI): m/z 489.2 $[M+H]^+$.

**Example E27: (E)-8-(1-(tert-butoxyimino)ethyl)-7-(4-chlorobenzyl)-1-(3-hydroxypropyl)-3-methyl-3,7-dihydro-1H-purine-2,6-dione**

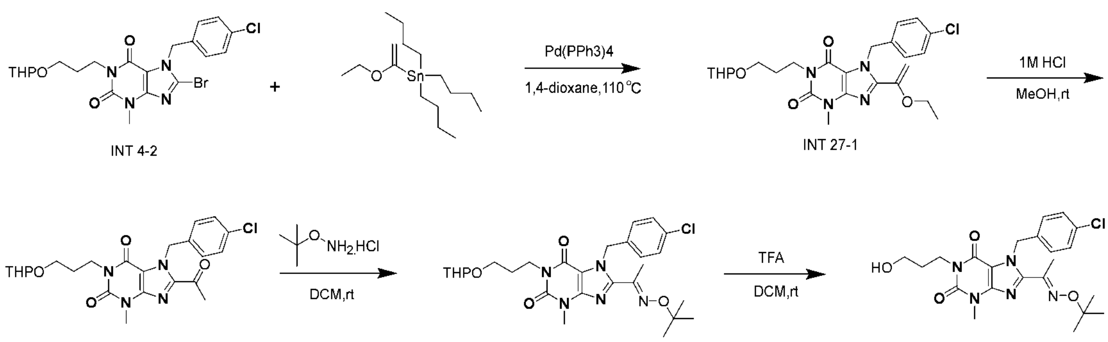


INT 4-2 INT 27-1 INT 27-2 INT 27-3 **E27**

INT 4-2 (200 mg, 0.392 mmol) was dissolved in 5 ml of 1,4-dioxane, and tributyl (1-ethoxyethylene) tin (267 μL, 0.784 mmol) and $Pd(PPh_3)_4$ (45 mg, 0.039 mmol) were added under the protection of N2 , and stirred at 110 °C for 12 h. TLC showed that the reaction was complete, and the solvent was evaporated under reduced pressure. Then, the reaction was separated by a flash silica gel column, and MeOH/DCM was used as the mobile phase for elution. The target product was collected and evaporated to dryness to obtain 180 mg of a jelly, which was the intermediate INT27-1.

INT 27-1 (180 mg, 0.358 mmol) was dissolved in 3 ml of methanol, 1 ml of 1N HCl was added dropwise at 0 °C, and the mixture was stirred at room temperature for 12 h. TLC showed that the reaction was complete, and the solvent was evaporated under reduced pressure. Then, the mixture was separated by flash silica gel chromatography, eluted with MeOH/DCM as the mobile phase, and the target product was collected and evaporated to dryness to obtain 140 mg of a jelly, which was the intermediate INT27-2.

INT 27-2 (140 mg, 0.295 mmol) and O-tert-butylhydroxylamine hydrochloride (74.3 mg, 0.59 mmol) were dissolved in 3 ml of DCM and stirred for 12 h. TLC showed that the reaction was complete, and the solvent was evaporated under reduced pressure. Then, the product was separated by flash silica gel chromatography, eluted with MeOH/DCM as the mobile phase, and the target product was collected and evaporated to dryness to obtain 90 mg of a jelly, namely the intermediate INT 18-2.

NT 17-2 (90 mg, 0.165 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase to collect the target product, which was then evaporated to dryness to obtain 50 mg of a white solid, Example E27. $^1$H NMR (500 MHz, Chloroform-*d*) δ 7.28 (d, J = 6.5 Hz, 2H), 7.10 (d, J = 8.5 Hz, 2H), 6.00 (s, 2H), 4.17 (t, J = 6.0 Hz, 2H), 3.64 (s, 3H), 3.51

(t, J = 5.6 Hz, 2H), 2.38 (s, 3H), 1.91 - 1.83(m, 2H), 1.21 (s, 9H). MS (ESI): m/z 462.2 [M+H]+.

**Example E28: 7-(4-chlorobenzyl)-8-(2-fluoro-6,6-dimethylbicyclo[3.1.1]heptan-2-yl)-1-(3-hydroxypropyl)-3-methyl-3,7-dihydro-1H-purine-2,6-dione**

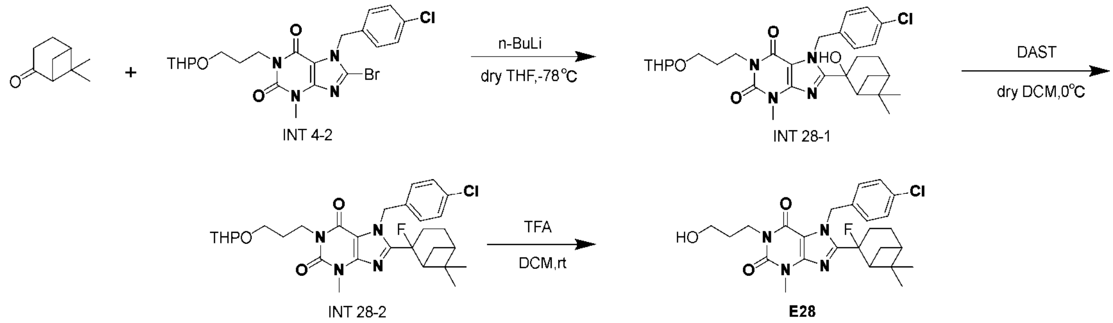


The synthesis method of Example E28 was the same as that of Example E21, except that (1R,5S)-6,6-dimethylbicyclo[3.1.1]heptan-2-one was used as the starting material instead of 4-(trifluoromethyl)cyclohexanone. $^1$H NMR (500 MHz, Chloroform-*d*) δ 7.33 (d, J = 8.5 Hz, 2H), 7.07 (d, J = 8.5 Hz, 2H), 5.69 - 5.50 (m, 2H), 4.33 - 3.97 (m, 2H), 3.63 (s, 3H), 3.55 - 3.49 (m, 2H), 2.64 - 2.58 (m, 1H), 2.41 - 2.29 (m, 2H), 2.06 - 2.00 (m, 4H), 1.92 - 1.84 (m, 3H), 0.96 - 0.86 (m, 6H). MS (ESI): m/z 489.2 [M+H]+.

**Example E29: 7-(4-chlorobenzyl)-8-(2-fluoro-6,6-dimethylbicyclo[3.1.1]heptan-2-yl)-1-(3-hydroxypropyl)-3-methyl-3,7-dihydro-1H-purine-2,6-dione**

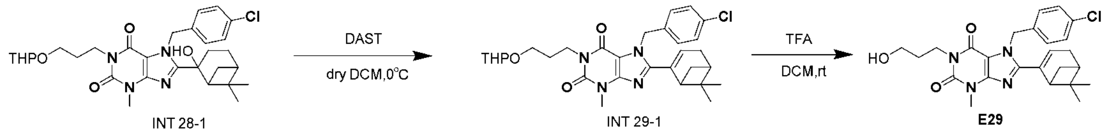


Intermediate INT 28-1 (80 mg, 0.14 mmol) was dissolved in 3 ml of ultra-dry DCM, and DAST (37 μL, 0.28 mmol) was added dropwise at 0 °C, and stirred for 30 min at 0 °C. LC-MS showed that the reaction was complete, and 20 mg of a jelly was obtained by evaporation to dryness, namely, intermediate INT 29-1.

INT 29-1 (20 mg, 0.036 mmol) was dissolved in 4 mL DCM, and then 1 mL TFA was added and stirred at room temperature for 1 h. TLC showed that the reaction was complete. The solvent was evaporated under reduced pressure, and the residue was purified by preparative liquid chromatography using acetonitrile/water as the mobile phase. The target product was collected and evaporated to dryness to obtain 10 mg of a white solid, i.e., Example E29. $^1$H NMR (500 MHz, Chloroform-d) δ 7.33 (d, J = 8.5Hz, 2H), 7.11 - 6.92 (m, 2H), 6.18 - 6.05 (m, 1H), 5.58 (s, 2H), 4. 21 - 4.15 (m, 2H), 3.63 (s, 3H), 3.55 - 3.44 m, 2H), 2.64 - 2.51 (m, 1H), 2.44 - 2.33 (m, 1H), 2.29 - 2.21 (m, 1H), 2.07 - 2.22 (m, 1H), 1.95 - 1.83 (m, 2H), 1.81 - 1.77 (m, 2H), 0.98 - 0.81 (m, 6H).MS (ESI): m/z 469.2 [M+H]+.

**Example E30: 7-(4-chlorobenzyl)-8-(4-ethyl-1-fluorocyclohexyl)-1-(3-hydroxypropyl)-3-methyl-3,7-dihydro-1H-purine-2,6-dione**

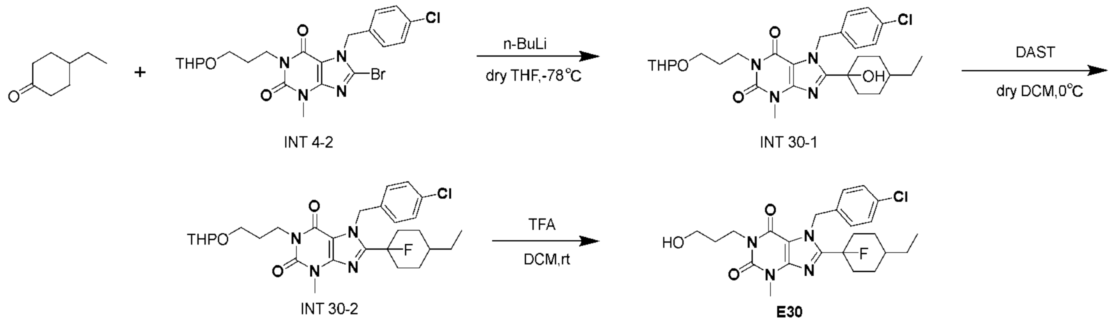


The synthesis method of Example E30 was the same as that of Example E21, except that 4-ethylcyclohexanone was used as the starting material instead of 4-(trifluoromethyl)cyclohexanone. $^1$H NMR (500 MHz, Chloroform-*d*, 3:1 isomer) δ 7.31 (d, J = 8.5 Hz, 2H), 7.16/7.11 (2 × d, J = 8.2 Hz, 2H), 5.76/5.75(2 × s, 2H), 4.21 - 4.15 (m, 2H), 3.59/3.62 (2 × s, 3H), 3.52 (t, J = 6.1 Hz, 2H), 3.45 - 3.36 (m, 1H), 2.15 - 1.99 (m, 4H), 1.93 - 1.86(m, 3H), 1.78 - 1.72 (m, 2H), 1.37 - 1.30 (m, 4H), 0.96 - 0.89 (m, 3H).MS (ESI): m/z 477.2 $[M+H]^+$.

**Example E31: 7-(4-chlorobenzyl)-8-(1-fluoro-4-isopropylcyclohexyl)-1-(3-hydroxypropyl)-3-methyl-3,7-dihydro-1H-purine-2,6-dione**

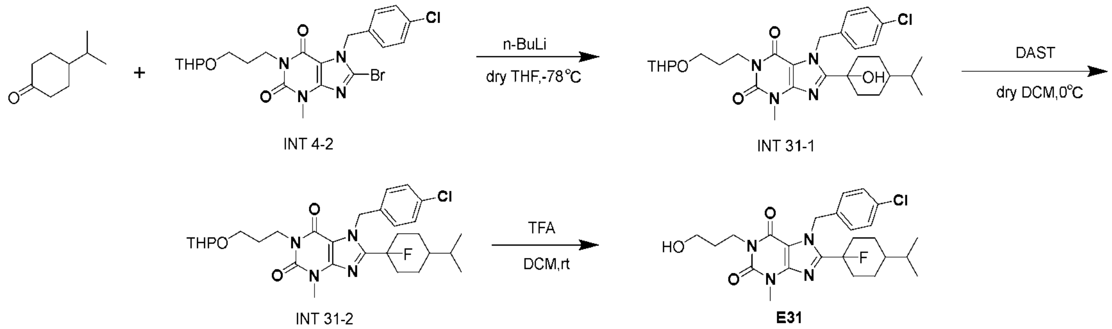


The synthesis method of Example E31 was the same as that of Example E21, except that 4-isopropylcyclohexanone was used as the starting material instead of 4-(trifluoromethyl)cyclohexanone. $^1$H NMR (500 MHz, Chloroform-d, 3:1 isomer) δ 7.31 (d, J = 8.5 Hz, 2H), 7.16/7.10 (2 × d, J = 8.2 Hz, 2H), 5.76 /5.75(2 × s, 2H), 4.21 - 4.13 (m, 2H), 3.59/3.62 (2 × s, 3H), 3.55 - 3.46 (m, 2H), 2.17 - 1.98 (m, 4H), 1.94 - 1.84 (m, 2H), 1.75 - 1.67 (m, 2H), 1.55 - 1.48 (m, 1H), 1.47 - 1.39 (m, 1H), 1.38 - 1.31 (m, 2H), 0.92 (2 × d, J = 7 Hz, 6H). MS (ESI): m/z 491.2 $[M+H]^+$.

**Example E32: 7-(4-chlorobenzyl)-8-(1-fluoro-4-methoxycyclohexyl)-1-(3-hydroxypropyl)-3-methyl-3,7-dihydro-1H-purine-2,6-dione**

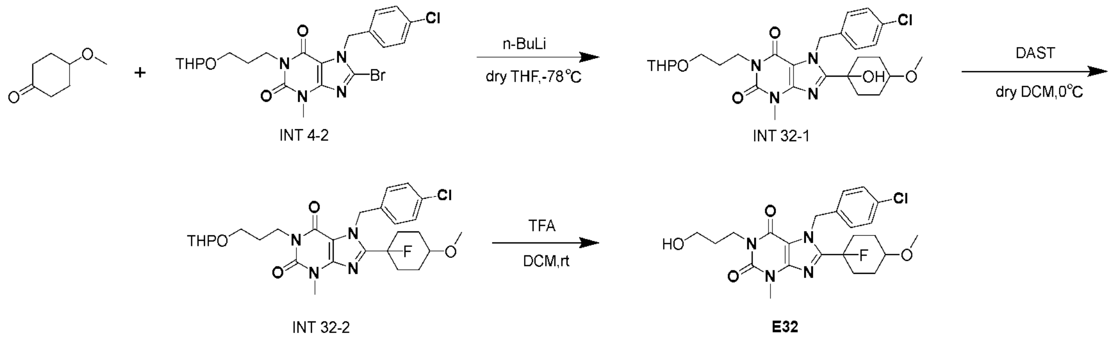


The synthesis method of Example E32 was the same as that of Example E21, except that 4-methoxycyclohexanone was used as the starting material instead of 4-(trifluoromethyl)cyclohexanone. $^1$H NMR (500 MHz, Chloroform-*d*) δ 7.31 (d, *J* = 8.5 Hz, 2H), 7.14 (d, *J* = 8.4 Hz, 2H), 5.76 (s, 2H), 4.18 (t, *J* = 6 Hz, 2H), 3.61 (s, 3H), 3.56 (s, 1H), 3.54 - 3.49 (m, 2H), 3.42 - 3.39 (m, 1H), 3.38 (s, 3H), 2.46 - 2.29 (m, 2H), 2.08 - 1.90 (m, 4H), 1.88 - 1.78 (m, 4H). MS (ESI): m/z 479.2 $[M+H]^+$.

**Example E33: 7-(4-chlorobenzyl)-8-(1-hydroxy-4-methoxycyclohexyl)-1-(3-hydroxypropyl)-3-methyl-3,7-dihydro-1H-purine-2,6-dione**

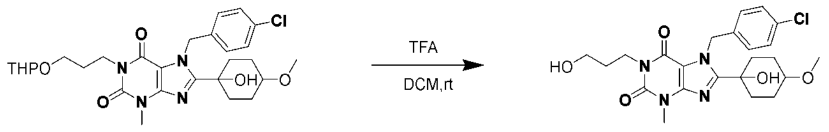


INT 32-1 E33

The synthesis method of Example E33 was the same as that of E25, except that INT 32-1 was used as the starting material instead of INT 24-1. $^1$H NMR (500 MHz, Chloroform-*d*) δ 7.31 (d, *J* = 8.5 Hz, 2H), 7.05 (d, *J* = 8.5 Hz, 2H), 5.93 (s, 2H), 4.16 (t, J = 6.0 Hz, 2H), 3.61 (s, 3H), 3.53 - 3.47 (m, 2H), 3.39 (s, 3H), 3.32 - 3.25 (m, 1H), 2.14 (td, J = 13.5, 3.9 Hz, 2H), 2.06 - 2.03 (m, 1H), 2.01 - 1.97 (m, 1H), 1.97 - 1.90 (m, 2H), 1.91 - 1.83(m, 2H), 1.70 - 1.64 (m, 2H). MS (ESI): m/z 477.2 $[M+H]^+$.

**Example E34: 7-(4-chlorobenzyl)-8-(1-fluoro-4,4-dimethylcyclohex-2-en-1-yl)-1-(3-hydroxypropyl)-3-methyl-3,7-dihydro-1H-purine-2,6-dione**

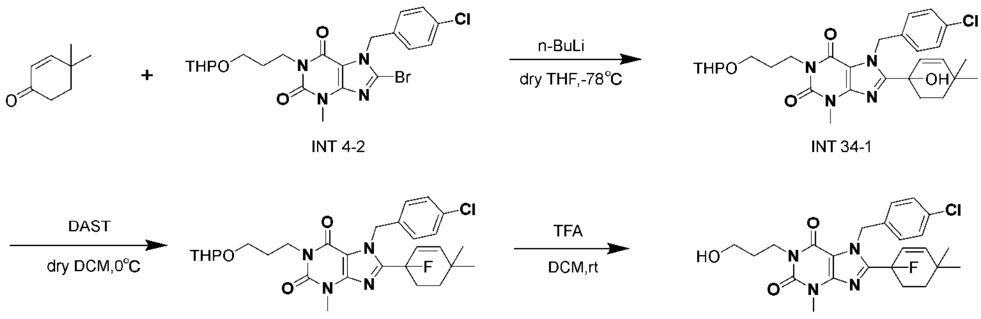


INT 34-2 E34

The synthesis method of Example E34 was the same as that of Example E21, except that 4,4-dimethyl-2-cyclohexyl-1-one was used as the starting material instead of 4-(trifluoromethyl)cyclohexanone. $^1$H NMR (500 MHz, Chloroform-d) δ 7.33 (d, *J* = 8.5 Hz, 2H), 7.06 (d, *J* = 8.5 Hz, 2H), 6.00 (d, J = 3.4 Hz, 1H), 5.64 (d, J = 3.2 Hz2H), 4.74 - 4.64 (m, 1H), 4.20 (t, J = 6 Hz, 2H), 3.63 (s, 3H), 3.57 - 3.51 (m, 2H), 3.42 (t, J = 7.1 Hz, 1H), 2.54 - 2.33 (m, 2H), 2.07 - 2.04 (m, 2H), 1.91 - 1.83(m, 2H), 1.03 (s, 3H), 0.94 (s, 3H).MS (ESI): m/z 475.2 $[M+H]^+$.

**Example E35: 7-(4-chlorobenzyl)-8-(1-hydroxy-4,4-dimethylcyclohex-2-en-1-yl)-1-(3-hydroxypropyl)-3-methyl-3,7-dihydro-1H-purine-2,6-dione**

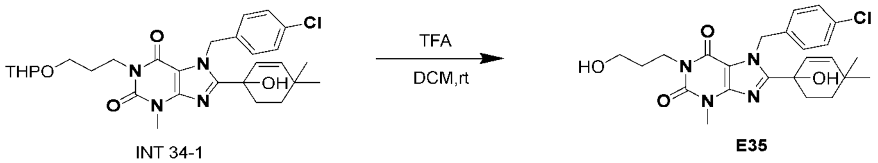


INT 34-1 E35

The synthesis method of Example E35 was the same as that of Example E25, except that INT 34-1 was used as

the starting material instead of INT 24-1. $^1$H NMR (500 MHz, Chloroform-*d*) δ 7.32 (d, *J* = 8.5 Hz, 2H), 7.06 (d, *J* = 8.5 Hz, 2H), 6.01 - 5.98 (m, 1H), 5.64 (d, *J* = 3.2 Hz, 2H), 5.42 - 5.30 (m, 1H), 4.19 (t, *J* = 5.5 Hz, 2H), 3.97 - 3.93 (m, 1H), 3.63 (s, 3H), 3.55 - 3.50 (m, 2H), 3.42 (t, J = 7 Hz, 1H), 2.38 - 2.43 (m, 2H), 2.28 - 2.20 (m, 1H), 2.07 - 2.01 (m, 1H), 1.91 - 1.83(m, 2H), 1.03 (s, 3H), 0.94 (s, 3H). MS (ESI): m/z 473.2 [M+H]$^+$.

**Example E36: 7-(4-chlorobenzyl)-8-(4,4-difluorocyclohexyl)-1-(3-hydroxypropyl)-3-methyl-3,7-dihydro-1H-purine-2,6-dione**

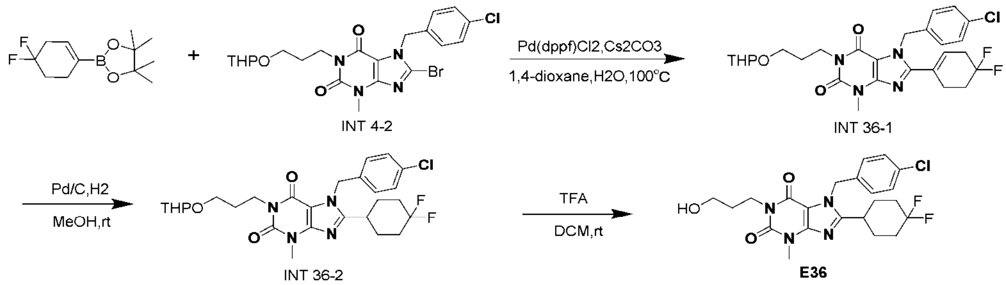


The synthesis method of Example E36 was the same as that of Example E10, except that 2-(4,4-difluorocyclohex-1-en-1-yl)boronic acid pinacol ester was used as the starting material instead of 4,4-(dimethylcyclohexen-1-yl) boronic acid pinacol ester. $^1$H NMR (500 MHz, Chloroform-*d*) δ 7.35 (d, *J* = 8.5 Hz, 2H), 7.10 (d, *J* = 8.5 Hz, 2H), 5.58 (s, 2H), 4.20 (t, *J* = 6.0 Hz, 2H), 3.62 (s, 3H), 3.57 - 3.51 (m, 2H), 3.49 - 3.45 (m, 1H), 2.82 - 2.73 (m, 1H), 2.32 - 2.20 (m, 2H), 2.10 - 2.02 (m, 2H), 1.93 - 1.86(m, 2H), 1.86 - 1.72 (m, 4H). MS (ESI): m/z 467.2 [M+H]$^+$.

**Example E37: 7-(4-chlorobenzyl)-8-(4,4-difluorocyclohex-1-en-1-yl)-1-(3-hydroxypropyl)-3-methyl-3,7-dihydro-1H-purine-2,6-dione**

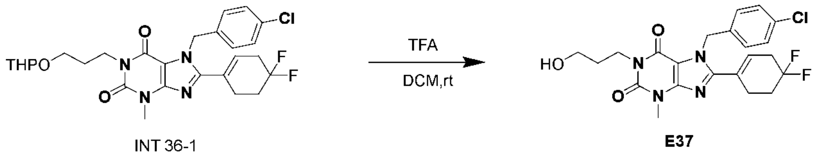


The synthesis method of Example E37 was the same as that of Example E25, except that INT 36-1 was used as the starting material instead of INT 24-1. $^1$H NMR (500 MHz, Chloroform-*d*) δ 7.33 (d, *J* = 8.5 Hz, 2H), 7.09 (d, J = 8.1 Hz, 2H), 5.91 (s, 1H), 5.59 (s, 2H), 4.21 (t, J = 6.0 Hz, 2H), 3.62 (s, 3H), 3.56 - 3.51 (m, 2H), 3.43 - 3.34 (m, 1H), 2.82 - 2.61 (m, 4H), 2.27 - 2.15 (m, 2H), 1.93 - 1.86(m, 2H). MS (ESI): m/z 465.1 [M+H]$^+$.

**Example E38: 7-(4-chlorobenzyl)-1-(3-hydroxypropyl)-3-methyl-8-(1,4,4-trifluorocyclohexyl)-3,7-dihydro-1H-purine-2,6-dione**

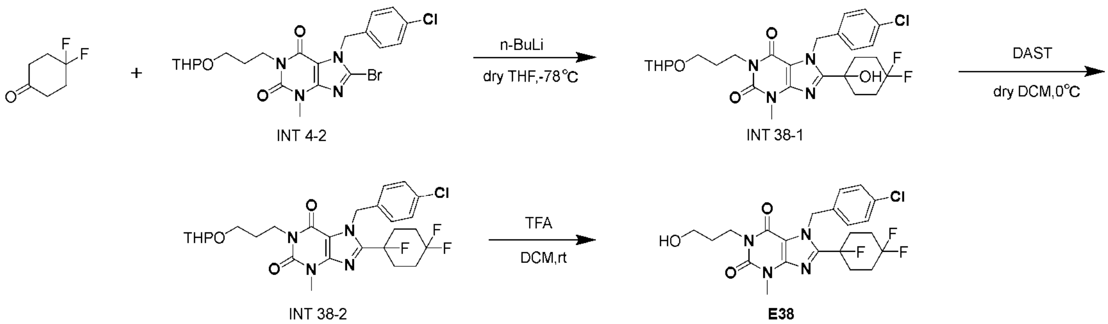

The synthesis method of Example E38 was the same as that of Example E21, except that 4,4-difluorocyclohexanone was used as the starting material instead of 4-(trifluoromethyl)cyclohexanone. $^1$H NMR (500 MHz, Chloroform-*d*) δ 7.33 (d, J = 8.5 Hz, 2H), 7.14 (d, J = 8.2 Hz, 2H), 5.78 (s, 2H), 4.20 (t, J = 6.0 Hz, 2H), 3.60 (s, 3H), 3.54 (t, J = 5.6 Hz, 2H), 2.49 - 2.33 (m, 2H), 2.22 - 2.09 (m, 6H), 1.93 - 1.86(m, 2H). MS (ESI): m/z 485.2 $[M+H]^+$.

**Example E39: 7-(4-chlorobenzyl)-8-(1-fluoro-4-methylcyclohexyl)-1-(3-hydroxypropyl)-3-methyl-3,7-dihydro-1H-purine-2,6-dione**

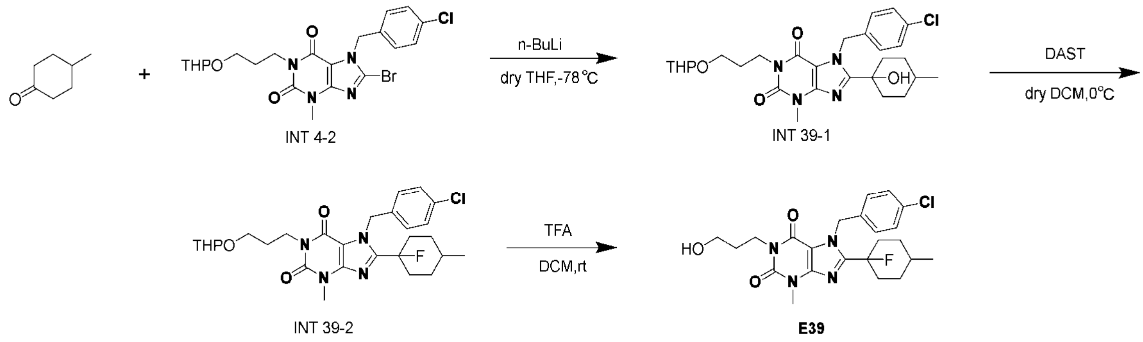


INT 4-2 INT 39-1 INT 39-2 E39

The synthesis method of Example E39 was the same as that of Example E21, except that 4-methylcyclohexanone was used as the starting material instead of 4-(trifluoromethyl)cyclohexanone. $^1$H NMR (500 MHz, Chloroform-d, 8:1 isomer) δ 7.31 (d, J = 8.5 Hz, 2H), 7.10/7.16 (2 × d, J = 8.2 Hz, 2H), 5.75/5.76(2 × s, 2H), 4.26 - 4.08 (m, 2H), 3.62/3.60 (2 × s, 3H), 3.51 (t, J = 5.5 Hz, 2H), 3.45 - 3.27 (m, 1H), 2.41 - 2.24 (m, 2H), 2.12 - 1.97 (m, 1H), 1.93 - 1.75 (m, 6H), 1.52 - 1.42 (m, 2H), 1.02 (d, J = 6.7 Hz, 3H). MS (ESI): m/z 463.2 $[M+H]^+$.

**Example E40: 4-(7-(4-chlorobenzyl)-1-(3-hydroxypropyl)-3-methyl-2,6-dioxo-2,3,6,7-tetrahydro-1H-purin-8-yl)-4-fluorocyclohexane-1-carbonitrile**

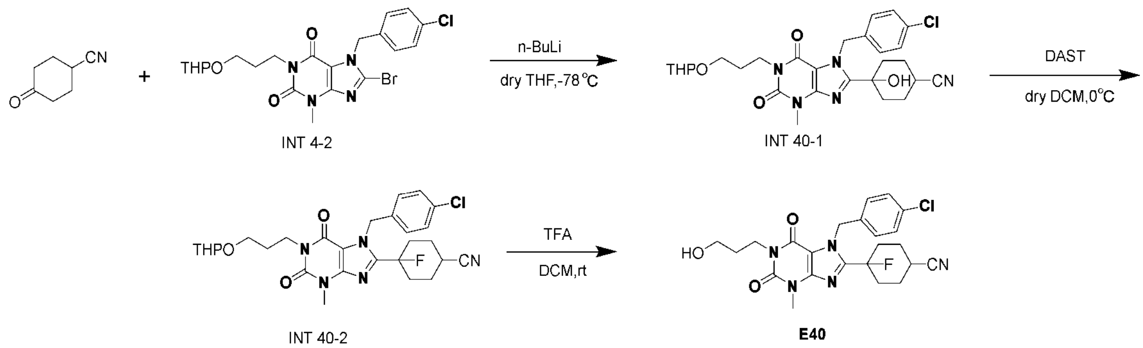


INT 4-2 INT 40-1 INT 40-2 E40

The synthesis method of Example E40 was the same as that of Example E21, except that 4-oxocyclohexanecarbonitrile was used as the starting material instead of 4-(trifluoromethyl)cyclohexanone. $^1$H NMR (500 MHz, Chloroform-*d*, 7:10 isomer) δ 7.33/7.31 (2 × d, J = 8.6 Hz, 2H), 7.14/7.12 (2 × d, J = 8.2 Hz, 2H), 5.76 (s, 2H), 4.19 (t, J = 6.0 Hz, 2H), 3.58/3.62 (2 × s, 3H), 3.53 (t, J = 5.5 Hz, 2H), 3.06 (p, J = 3.9 Hz, 1H), 2.52 - 2.36 (m, 1H), 2.18 - 2.04 (m, 5H), 2.03 - 1.99 (m, 2H). 1.91 - 1.83(m, 2H). MS (ESI): m/z 474.2 $[M+H]^+$.

**Example E41: 7-(4-chlorobenzyl)-1-(3-hydroxypropyl)-3-methyl-8-(4-(trifluoromethyl)cyclohex-1-en-1-yl)-3,7-dihydro-1H-purine-2,6-dione**

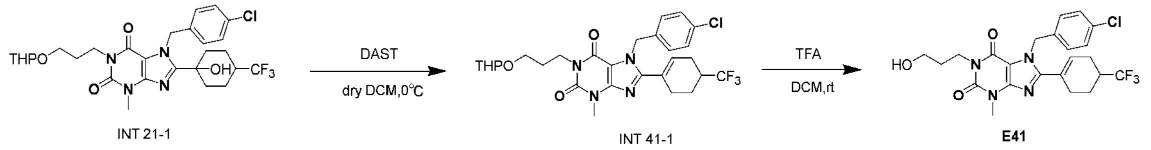


The synthesis method of Example E41 was the same as that of E29, except that INT 21-1 was used as the starting material instead of INT 28-1. $^1$H NMR (500 MHz, Chloroform-*d*) δ 7.33 (d, J = 8.5 Hz, 2H), 7.03 (d, J = 8.5 Hz, 2H), 6.08 - 6.04 (m, 1H), 5.65 - 5.47 (m, 2H), 4.18 (t, *J* = 6.0 Hz, 2H), 3.62 (s, 3H), 3.52 (t, *J* = 5.5 Hz, 2H), 3.42 - 3.34 (m, 1H), 2.67 - 2.59 (m, 1H), 2.54 - 2.44 (m, 1H), 2.44 - 2.24 (m, 3H), 2.20 - 2.12 (m, 1H), 1.91 - 1.83 (m, 2H), 1.75 - 1.70 (m, 1H). MS (ESI): m/z 497.15 [M+H]$^+$.

**Example E42: 7-(4-chlorobenzyl)-1-(3-hydroxypropyl)-3-isopropyl-8-(1,4,4-trifluorocyclohexyl)-3,7-dihydro-1H-purine-2,6-dione**

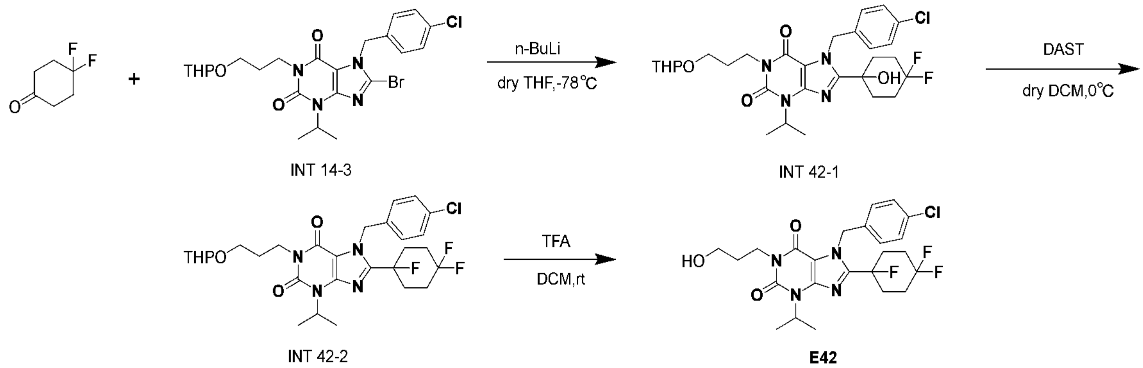


The synthesis method of Example E42 was the same as that of E38, except that INT 14-3 was used as the starting material instead of INT 4-2. $^1$H NMR (500 MHz, Chloroform-*d*) δ 7.33 (d, J = 8.5 Hz, 2H), 7.17 (d, *J* = 8.2 Hz, 2H), 5.77 (s, 2H), 5.19 (hept, *J* = 6.8 Hz, 1H), 4.17 (t, *J* = 6.0 Hz, 2H), 3.57 - 3.48 (m, 2H), 3.45 - 3.36 (m, 1H), 2.54 - 2.24 (m, 2H), 2.21 - 2.03 (m, 6H), 1.91 - 1.83(m, 2H)), 1.59 (d, *J* = 7.0 Hz, 6H). MS (ESI): m/z 513.2 [M+H]$^+$.

**Example E43: 7-(4-chloro-3-fluorobenzyl)-1-(3-hydroxypropyl)-3-methyl-8-(1,4,4-trifluorocyclohexyl)-3,7-dihydro-1H-purine-2,6-dione**

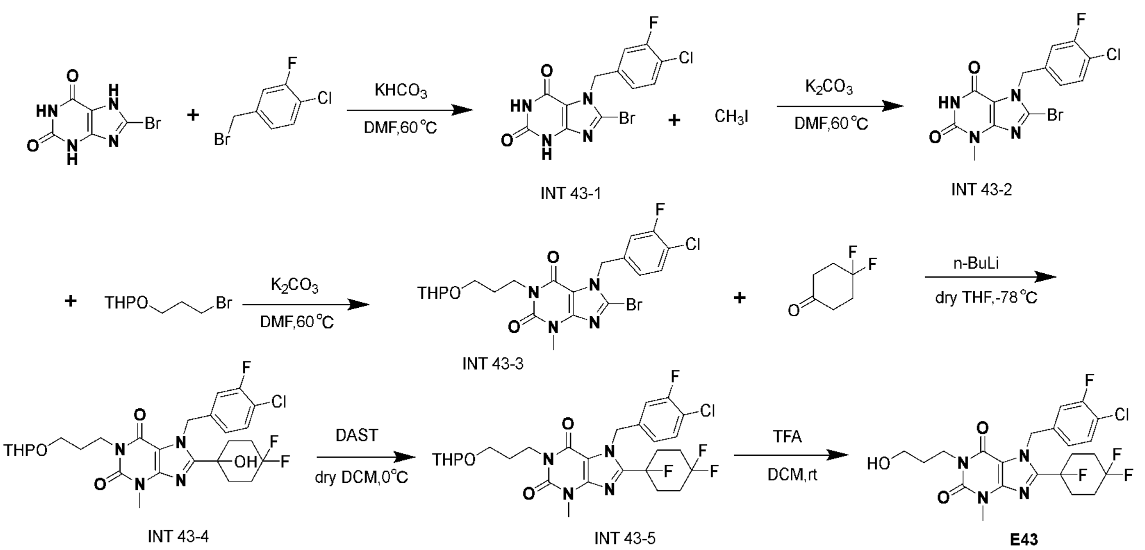

The synthesis method of Example E43 was the same as that of Example E38, except that INT 43-3 was used as the raw material instead of INT 4-2. The synthesis method of INT 43-3 was the same as that of INT 4-2, except that 4-chloro-3-fluorobenzyl bromide was used as the raw material instead of 4-chlorobenzyl bromide. $^{1}$H NMR (500 MHz, Chloroform-*d*) δ 7.41 - 7.34 (m, 2H), 7.01 - 6.90 (m, 2H), 5.76 (s, 2H), 4.19 (t, *J* = 6.0 Hz, 2H), 3.59 (s, 3H), 3.56 - 3.48 (m, 2H), 3.28 (s, 1H), 2.55 - 2.32 (m, 2H), 2.25 - 2.08 (m, 6H), 1.92 - 1.84 (m, 2H). MS (ESI): m/z 503.1 [M+H]$^{+}$.

**Example E44: 7-(3,4-difluorophenyl)-1-(3-hydroxypropyl)-3-methyl-8-(1,4,4-trifluorocyclohexyl)-3,7-dihydro-1H-purine-2,6-dione**

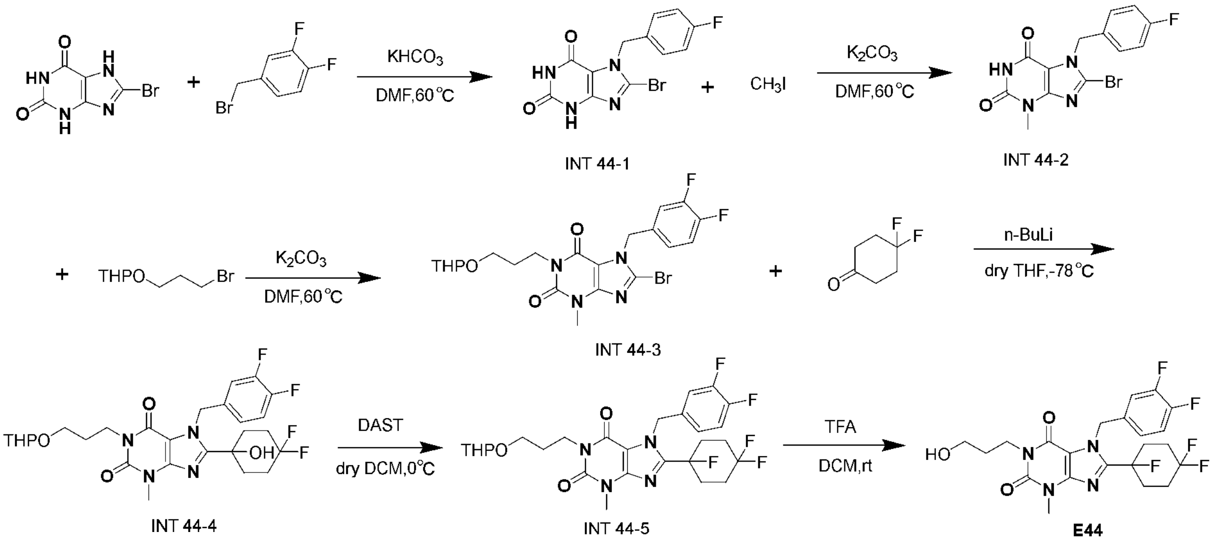


The synthesis method of Example E44 was the same as that of Example E43, except that 3,4-difluorobenzyl bromide was used as the starting material instead of 4-chloro-3-fluorobenzyl bromide. $^{1}$H NMR (500 MHz, Chloroform-*d*) δ 7.16 - 7.09 (m, 1H), 7.03 (ddd, *J* = 10.5, 7.5, 2.3 Hz, 1H), 6.99 - 6.95 (m, 1H), 5.72 (s, 2H), 4.17 (t, *J* = 6.5 Hz, 2H), 3.57 (s, 3H), 3.55 - 3.47 (m, 2H), 3.28 (s, 1H), 2.49 - 2.31 (m, 2H), 2.23 - 2.05 (m, 6H), 1.93 - 1.84 (m, 2H). MS (ESI): m/z 487.2 [M+H]$^{+}$.

**Example E45: 7-(4-fluorobenzyl)-1-(3-hydroxypropyl)-3-methyl-8-(1,4,4-trifluorocyclohexyl)-3,7-dihydro-1H-purine-2,6-dione**

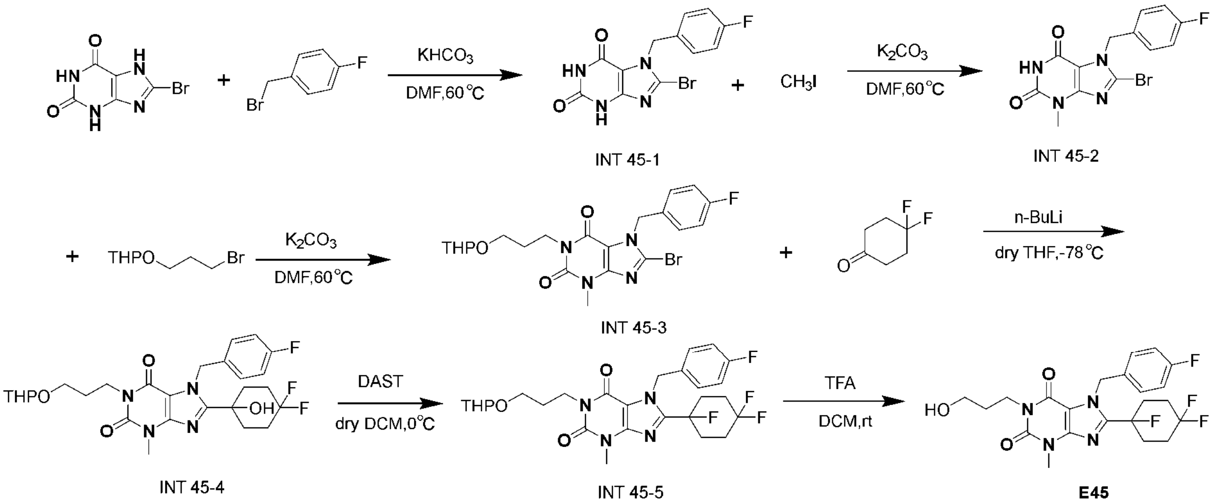

The synthesis method of Example E45 was the same as that of Example E43, except that 4-fluorobenzyl bromide was used as the starting material instead of 4-chloro-3-fluorobenzyl bromide. $^1$H NMR (500 MHz, Chloroform-*d*) δ 7.23 - 7.13 (m, 2H), 7.04 - 6.97 (m, 2H), 5.75 (s, 2H), 4. 17 (t, *J* = 6 Hz, 2H), 3.57 (s, 3H), 3.53 - 3.46 (m, 2H), 3.33 (s, 1H), 2.47 - 2.27 (m, 2H), 2.19 - 2.04 (m, 6H), 1.92 - 1.83 (m, 2H). MS (ESI): m/z 469.2 [M+H]$^+$.

**Example E46: 7-(4-fluorobenzyl)-1-(3-hydroxypropyl)-3-isopropyl-8-(1,4,4-trifluorocyclohexyl)-3,7-dihydro-1H-purine-2,6-dione**

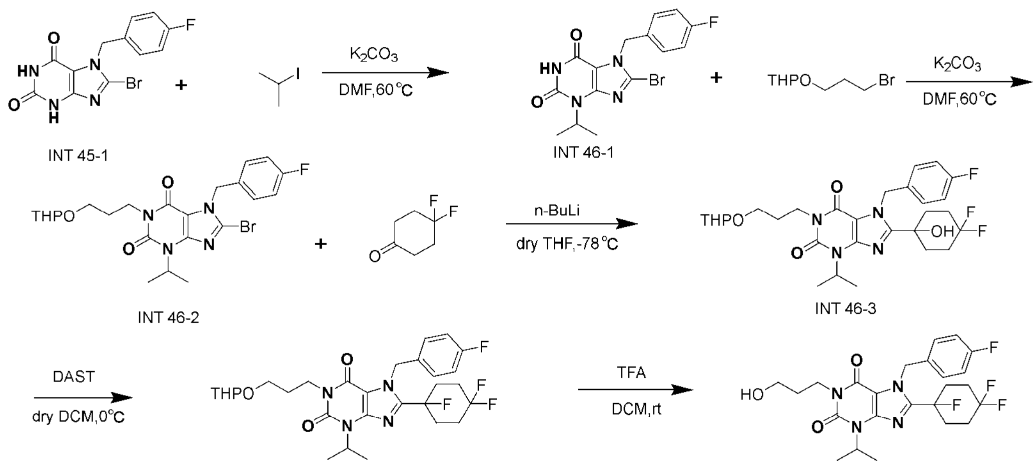


INT 45-1 INT 46-1 INT 46-2 INT 46-3 INT 46-4 **E46**

The synthesis method of Example E46 was the same as that of Example E45, except that 2-iodopropane was used as the starting material instead of iodomethane. $^1$H NMR (500 MHz, Chloroform-d) δ 7.24 - 7.18 (m, 2H), 7.06 - 6.98 (m, 2H), 5.75 (s, 2H), 5.16 (h, *J* = 6.9 Hz, 1H), 4.16 (t, *J* = 6.0 Hz, 2H), 3.54 - 3.47 (m, 2H), 3.40 (t, *J* = 7.1 Hz, 1H), 2.46 - 2.27 (m, 2H), 2.20 - 2.06 (m, 6H), 1.91 - 1.83 (m, 2H), 1.56 (d, *J* = 7.0 Hz, 6H). MS (ESI): m/z 497.2 [M+H]$^+$.

**Example E47: 7-(3,4-difluorophenyl)-1-(3-hydroxypropyl)-3-isopropyl-8-(1,4,4-trifluorocyclohexyl)-3,7-dihydro-1H-purine-2,6-dione**

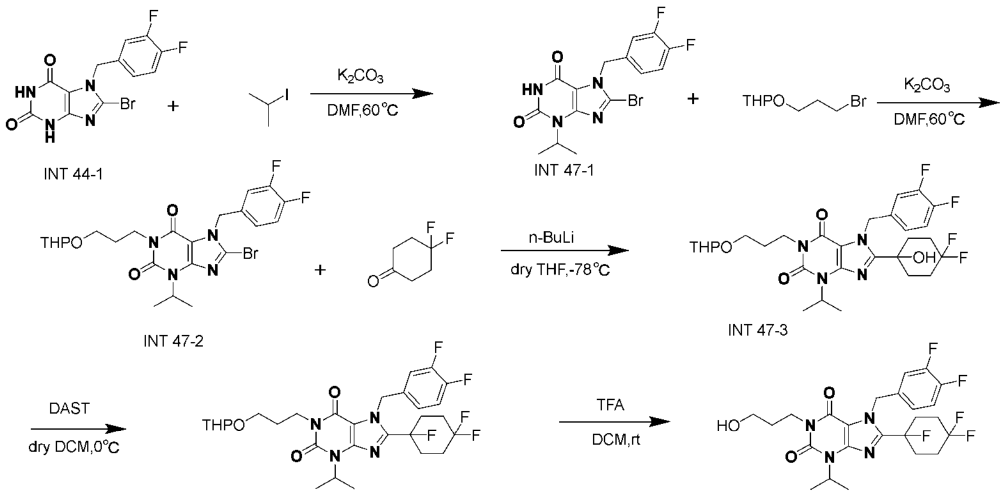


INT 44-1 INT 47-1 INT 47-2 INT 47-3 INT 47-4 **E47**

The synthesis method of Example E47 was the same as that of E46, except that INT 47-2 was used as the starting material instead of INT 46-2. $^1$H NMR (500 MHz, Chloroform-d) δ 7.19 - 7.12 (m, 1H), 7.07 (ddd, *J* = 10.6, 7.5, 2.2 Hz, 1H), 7.04 - 6.99 (m, 1H), 5.74 (s, 2H), 5.19 (hept, *J* = 6.9 Hz, 1H), 4.17 (t, *J* = 6 Hz, 2H), 3.53 (t, *J* = 5.5 Hz, 2H), 3.40 (s, 1H), 2.50 -

2.31 (m, 2H), 2.28 - 2.07 (m, 6H), 1.94 - 1.84 (m, 2H), 1.59 (d, *J* = 6.9 Hz, 6H). MS (ESI): m/z 515.2 $[M+H]^+$.

**Example E48: 7-(4-chlorobenzyl)-8-(4-(dimethylamino)-1-fluorocyclohexyl)-1-(3-hydroxypropyl)-3-methyl-3,7-dihydro-1H-purine-2,6-dione**

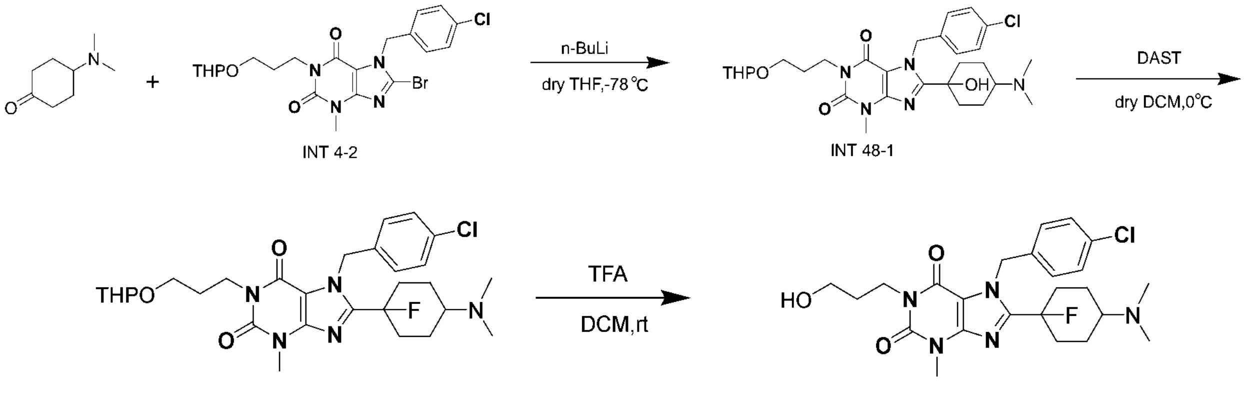


INT 4-2 INT 48-1 INT 48-2 **E48**

The synthesis method of Example E48 was the same as that of Example E21, except that 4-dimethylaminocyclohexanone was used as the starting material instead of 4-(trifluoromethyl)cyclohexanone. $^1$H NMR (500 MHz, Chloroform-*d*) δ 7.28 (d, *J* = 8.5 Hz, 2H), 7.09 (d, *J* = 8.4 Hz, 2H), 5.74 (s, 2H), 4.15 (t, *J* = 6.0 Hz, 2H), 3.59 (s, 3H), 3.52 - 3.46 (m, 2H), 3.36 (s, 1H), 2.48 - 2.32 (m, 2H), 2.27 (s, 6H), 2.22 - 2.16 (m, 1H), 1.94 - 1.79 (m, 8H). MS (ESI): m/z 492.2 $[M+H]^+$.

**Example E49: 7-(4-chlorobenzyl)-8-(4-(dimethylamino)cyclohex-1-en-1-yl)-1-(3-hydroxypropyl)-3-methyl-3,7-dihydro-1H-purine-2,6-dione**

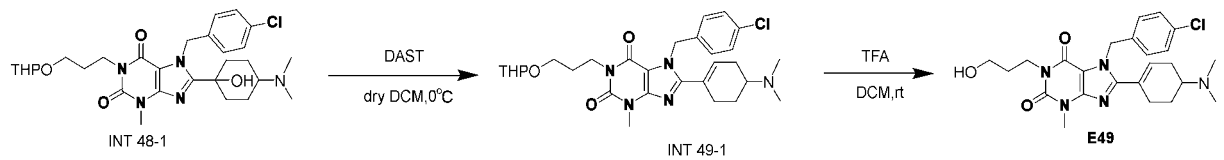


INT 48-1 INT 49-1 **E49**

The synthesis method of Example E49 was the same as that of E29, except that INT 48-1 was used as the starting material instead of INT 28-1. $^1$H NMR (500 MHz, Chloroform-*d*) δ 7.32 (d, *J* = 8.5 Hz, 2H), 6.99 (d, *J* = 8.3 Hz, 2H), 5.98 - 5.94 (m, 1H), 5.66 - 5.46 (m, 2H), 4.15 (t, *J* = 6.1 Hz, 2H), 3.59 (s, 3H), 3.50 (t, *J* = 5.5 Hz, 3H), 3.44 - 3.33 (m, 1H), 2.89 - 2.81 (m, 6H), 2.73 - 2.63 (m, 1H), 2.57 - 2.42 (m, 5H), 2.35 - 2.27 (m, 1H), 1.91 - 1.81 (m, 2H). MS (ESI): m/z 472.2 $[M+H]^+$.

**Example E50: 7-(4-chlorobenzyl)-1-(3-hydroxypropyl)-3-methyl-8-(4-morpholinocyclohex-1-en-1-yl)-3,7-dihydro-1H-purine-2,6-dione**

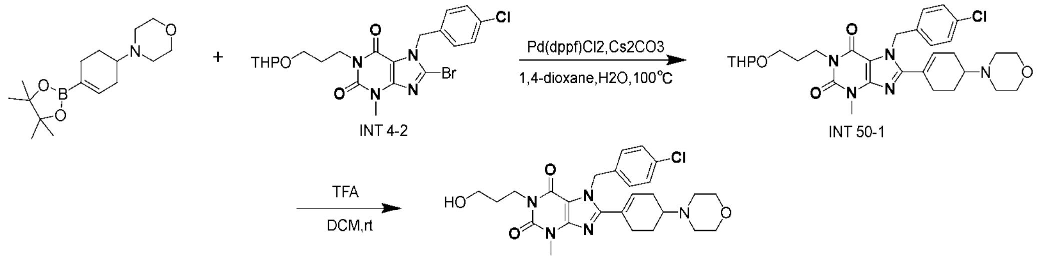


INT 4-2 INT 50-1 **E50**

The synthesis method of Example E50 was the same as that of Example E6, except that 4-(4-(4,4,5,5-tetramethyl-1,3,2-dioxaborolan-2-yl)-3-cyclohexene)morpholine was used as the starting material instead of 4,4,5,5-tetramethyl-2-(1,2,3,6-tetrahydro-[1,1'-biphenyl]-4-yl)-1,3,2-dioxaborolan. $^1$H NMR (500 MHz, Chloroform-*d*) δ 7.30 (d, *J* = 8.5 Hz, 2H), 7.05 (d, *J* = 8.3 Hz, 2H), 6.05 - 6.01 (m, 1H), 5.61 - 5.47 (m, 2H), 4.20 - 4.12 (m, 2H), 3.78 - 3.72 (m, 4H), 3.60 (s, 3H), 3.54 - 3.46 (m, 2H), 3.41 (t, *J* = 7.1 Hz, 1H), 2.66 - 2.50 (m, 6H), 2.45 - 2.30 (m, 2H), 2.27 - 2.17 (m, 1H), 2.14 - 2.07 (m, 1H), 1.91 - 1.84 (m, 2H), 1.57 - 1.53 (m, 1H). MS (ESI): m/z 514.2 [M+H]$^+$.

**Example E51: 7-(4-chloro-3-fluorobenzyl)-3-ethyl-8-(1-fluoro-4-(trifluoromethyl)cyclohexyl)-1-(3-hydroxypropyl)-3,7-dihydro-1H-purine-2,6-dione**

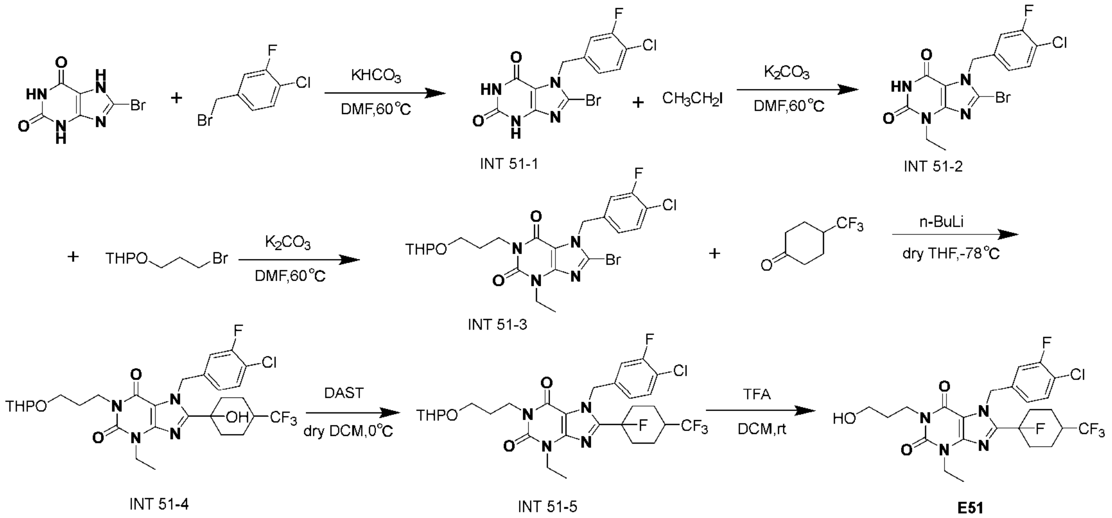


The synthesis method of Example E51 was the same as that of Example E43, except that 2-iodopropane was used as the starting material instead of iodoethane. $^1$H NMR (500 MHz, Chloroform-*d*, 5:11 isomer) δ 7.41 - 7.34 (m, 1H), 7.03 - 6.90 (m, 2H), 5.75/5.73 (2 × s, 2H), 4.28 - 4.07 (m, 4H), 3.60 - 3.46 (m, 2H), 2.49 - 2.39 (m, 1H), 2.35 - 2.09 (m, 2H), 2.07 - 1.82 (m, 7H), 1.81 - 1.74 (m, 1H), 1.37/1.36 (2 × t, *J* = 7Hz, 3H). MS (ESI): m/z 549.2 [M+H]$^+$.

**Example E52: 7-(3,4-difluorophenyl)-8-(1-fluoro-4-(trifluoromethyl)cyclohexyl)-1-(3-hydroxypropyl)-3,7-dihydro-1H-purine-2,6-dione**

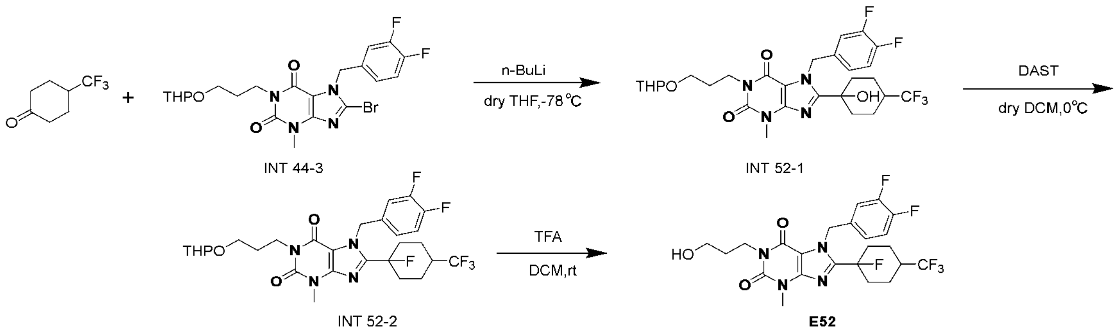


The synthesis method of Example E52 was the same as that of E21, except that INT 44-3 was used as the starting material instead of INT 4-2.$^1$H NMR (500 MHz, Chloroform-*d*, 9:13 isomer) δ 7.16 - 7.08 (m, 1H), 7.07 - 6.88 (m, 2H), 5.70/5.72 (2 × s, 2H), 4.17/4.16 (2 × t, *J* = 7.5 Hz, 2H), 3.57 (2 × s, 3H), 3.54 - 3.47 (m, 2H), 3.35 - 3.26 (m, 1H), 2.46 - 2.36 (m, 1H), 2.32 - 2.19 (m, 1H), 2.18 -2.11 (m, 1H), 2.05 - 1.82 (m, 7H), 1.82 - 1.71 (m, 1H). MS (ESI): m/z 519.2 [M+H]$^+$.

**Example E53: 7-(4-chlorobenzyl)-8-(1-fluoro-4-(trifluoromethyl)cyclohexyl)-1-(3-hydroxypropyl)-3-isopropyl-3,7-dihydro-1H-purine-2,6-dione**

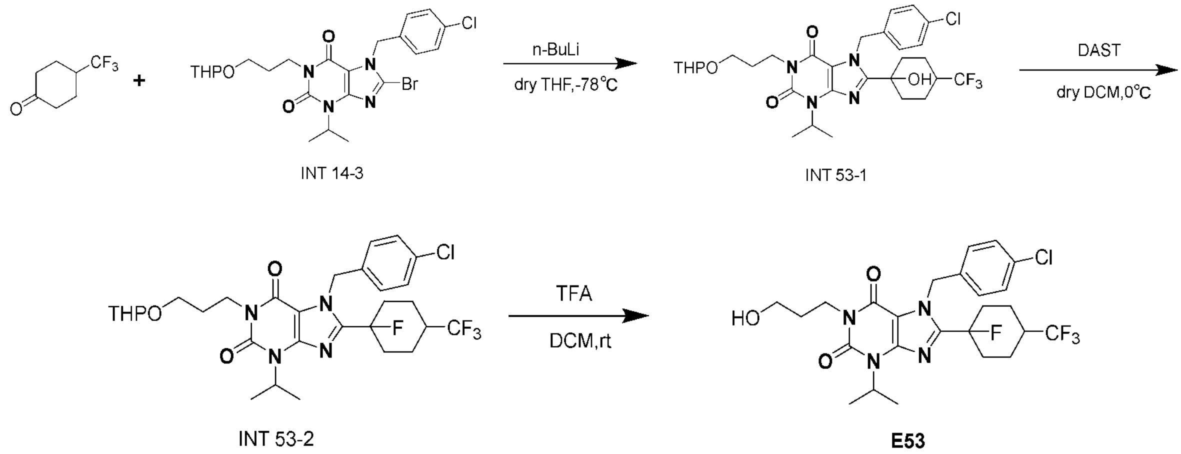


The synthesis method of Example E53 was the same as that of Example E42, except that 4-(trifluoromethyl) cyclohexanone was used as the raw material instead of 4,4-difluorocyclohexanone. $^1$H NMR (500 MHz, Chloroform-*d*, 8:1 isomer) δ 7.32 (d, *J* = 8.5 Hz, 2H), 7.19/7.13 (2 × d, *J* = 8.2 Hz, 2H), 5.76 (s, 2H), 5.25 - 5.12 (m, 1H), 4.17/4.16 (2 × t, *J* = 6 Hz, 2H), 3.58 - 3.49 (m, 2H), 3.48 - 3.39 (m, 1H), 2.32 - 2.11 (m, 4H), 2.11 - 2.00 (m, 1H), 2.00 - 1.92 (m, 2H), 1.91 - 1.85 (m, 2H), 1.84 - 1.72 (m, 2H), 1.58 (d, *J* = 7.0 Hz, 6H). MS (ESI): m/z 545.2 [M+H]$^+$.

**Example E54: 7-(4-chlorobenzyl)-8-(1-fluoro-4-phenylcyclohexyl)-1-(3-hydroxypropyl)-3-methyl-3,7-dihydro-1H-purine-2,6-dione**

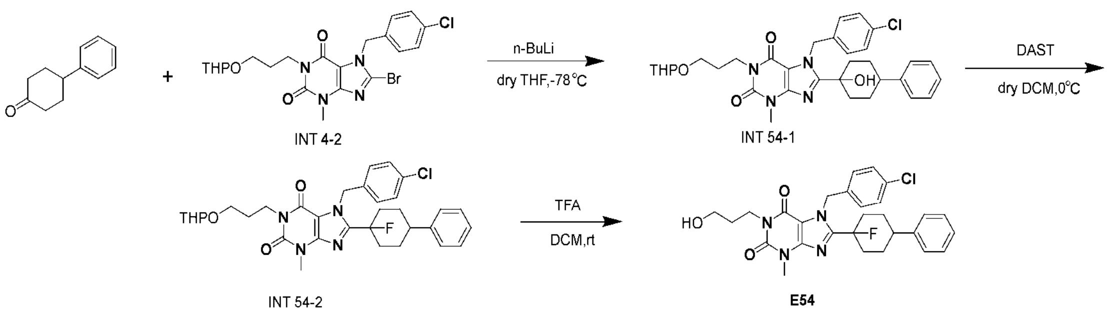


The synthesis method of Example E54 was the same as that of Example E21, except that 4-phenylcyclohexanone was used as the starting material instead of 4-(trifluoromethyl)cyclohexanone. $^1$H NMR (500 MHz, Chloroform-d, 3:1 isomer) δ 7.38 - 7.30 (m, 4H), 7.28 - 7.22 (m, 3H), 7.19/7.09 (2 × d, *J* = 8.2 Hz, 2H), 5.79/5.77 (2 × s, 2H), 4.22 -4.15(m, 2H), 3.61/3.65 (2 × s, 3H), 3.56 - 3.49 (m, 2H), 2.83 - 2.64 (m, 1H), 2.51 - 2.43 (m, 1H), 2.35 - 2.24 (m, 1H), 2.22 - 2.15 (m, 2H), 1.99 - 1.86 (m, 6H). MS (ESI): m/z 525.2 [M+H]$^+$.

**Example E55: 7-(4-chlorobenzyl)-8-(1-fluoro-4-(pyrrolidin-1-yl)cyclohexyl)-1-(3-hydroxypropyl)-3-methyl-3,7-dihydro-1H-purine-2,6-dione**

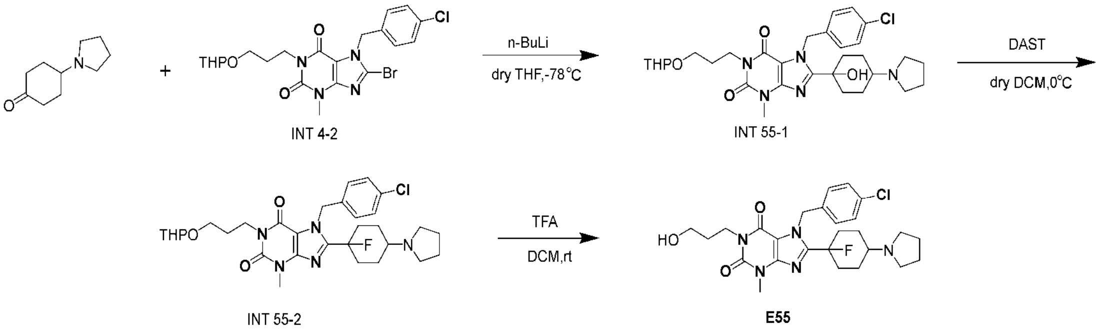


The synthesis method of Example E55 was the same as that of Example E21, except that 4-(1-pyrrolidinyl) cyclohexanone was used as the starting material instead of 4-(trifluoromethyl)cyclohexanone. $^1H$ NMR (500 MHz, Chloroform-*d*) δ 7.31 (d, *J* = 8.5 Hz, 2H), 7.08 (d, *J* = 8.2 Hz, 2H), 5.73 (s, 2H), 4.17 (t, *J* = 5.9 Hz, 2H), 3.60 (s, 3H), 3.51 (t, *J* = 5.5 Hz, 2H), 3.09 - 2.99 (m, 1H), 2.87 - 2.75 (m, 2H), 2.64 - 2.51 (m, 2H), 2.35 - 2.27 (m, 2H), 2.21 - 2.11 (m, 4H), 2.09 - 2.01 (m, 4H), 1.99 - 1.92 (m, 2H), 1.93 - 1.85 (m, 2H). MS (ESI): m/z 518.2 $[M+H]^+$.

**Example E56: 7-(4-chloro-3-fluorobenzyl)-1-(3-hydroxypropyl)-3-isopropyl-8-(1,4,4-trifluorocyclohexyl)-3,7-dihydro-1H-purine-2,6-dione**

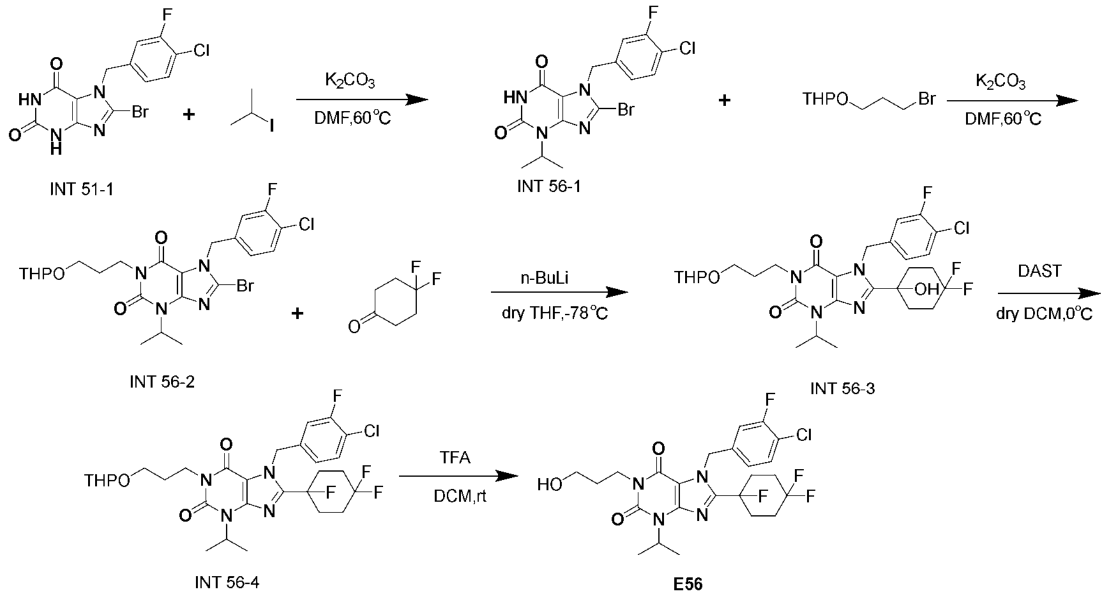


The synthesis method of Example E56 was the same as that of Example E47, except that INT 51-1 was used as the starting material instead of INT 44-1. $^1H$ NMR (500 MHz, Chloroform-d) δ 7.39 - 7.35 (m, 1H), 7.05 - 6.95 (m, 2H), 5.75 (s, 2H), 5.19 (hept, *J* = 7.0 Hz, 1H), 4.17 (t, *J* = 6.0 Hz, 2H), 3.53 (t, *J* = 5.5 Hz, 2H), 2.51 - 2.30 (m, 2H), 2.24 - 2.11 (m, 6H), 1.93 - 1.86 (m, 2H), 1.59 (d, *J* = 6.9 Hz, 6H). MS (ESI): m/z 531.2 $[M+H]^+$.

**Example E57: 7-(4-chloro-3-fluorobenzyl)-3-ethyl-1-(3-hydroxypropyl)-8-(4-(trifluoromethyl)cyclohex-1-en-1-yl)-3,7-dihydro-1H-purine-2,6-dione**

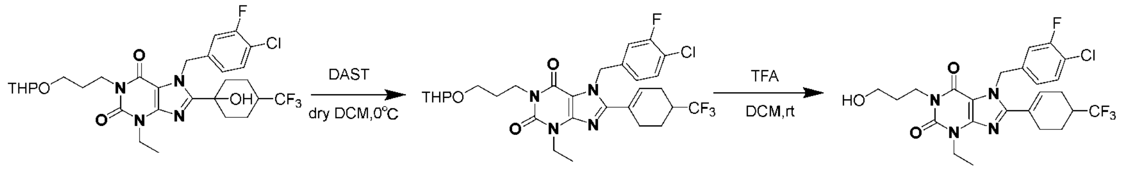


INT 51-4 INT 57-1 **E57**

The synthesis method of Example E57 was the same as that of Example E29, except that INT 51-4 was used as the starting material instead of INT 28-1. $^1$H NMR (500 MHz, Chloroform-*d*) δ 7.42 - 7.37 (m, 1H), 6.88 (ddd, *J* = 12.3, 8.9, 2.1 Hz, 2H), 6.05 (s, 1H), 5.63 - 5.49 (m, 2H), 4.28 - 4.09 (m, 4H), 3.52 (t, *J* = 5.5 Hz, 2H), 2.72 - 2.63 (m, 1H), 2.55 - 2.26 (m, 4H), 2.23 - 2.13 (m, 1H), 1.96 - 1.83 (m, 3H), 1.39 (t, *J* = 7.0 Hz, 3H). MS (ESI): m/z 529.2 [M+H]$^+$.

**Example E58: 7-(3,4-difluorophenyl)-1-(3-hydroxypropyl)-3-methyl-8-(4-(trifluoromethyl)cyclohex-1-en-1-yl)-3,7-dihydro-1H-purine-2,6-dione**

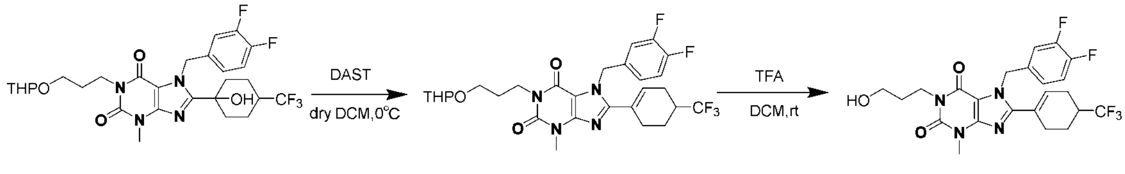


INT 51-4 INT 58-1 **E58**

The synthesis method of Example E58 was the same as that of Example E29, except that INT 52-1 was used as the starting material instead of INT 28-1. $^1$H NMR (500 MHz, Chloroform-*d*) δ 7.21 - 7.12 (m, 1H), 6.92 (ddd, *J* = 10.3, 7.4, 2.3 Hz, 1H), 6.88 - 6.83 (m, 1H), 6.10 - 6.04 (m, 1H), 5.64 - 5.49 (m, 2H), 4.27 - 4.12 (m, 2H), 3.62 (s, 3H), 3.57 - 3.47 (m, 2H), 3.38 (t, *J* = 7.0 Hz, 1H), 2.69 - 2.61 (m, 1H), 2.56 - 2.27 (m, 4H), 2.23 - 2.13 (m, 1H), 1.94 - 1.86 (m, 3H). MS (ESI): m/z 499.2 [M+H]$^+$.

**Example E59: 8-(1-Fluoro-4-(trifluoromethyl)cyclohexyl)-7-(4-fluorobenzyl)-1-(3-hydroxypropyl)-3-methyl-3,7-dihydro-1H-purine-2,6-dione**

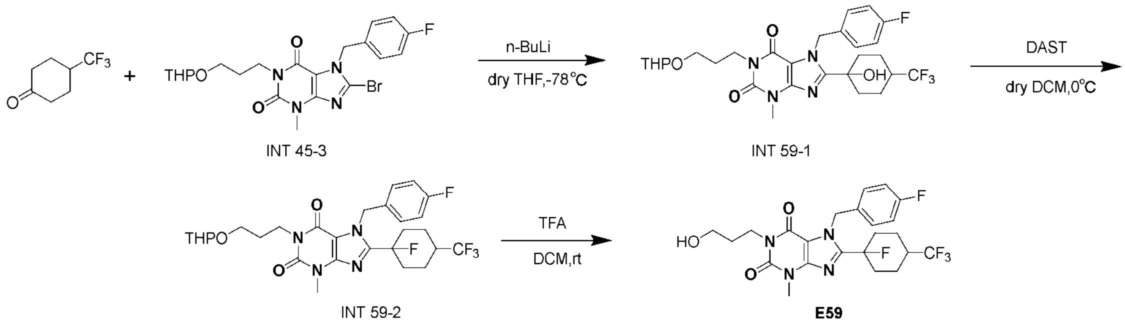


INT 45-3 INT 59-1

INT 59-2 **E59**

The synthesis method of Example E59 was the same as that of Example E45, except that 4-(trifluoromethyl)cyclohexanone was used as the starting material instead of 4,4-difluorocyclohexanone. $^1$H NMR (500 MHz, Chloroform-*d*, 3:1 isomer) δ 7.23/7.16 (2 × m, 2H), 7.06 - 7.00 (m, 2H), 5.77/5.75(2 × s, 2H), 4.23 - 4.15 (m, 2H), 3.58/3.61 (2 × s, 3H), 3.56 - 3.49 (m, 2H), 3.39 - 3.30 (m, 1H), 2.48 - 2.35 (m, 1H), 2.31 - 2.09 (m, 3H), 2.08 - 2.01 (m, 1H), 2.00 - 1.85 (m, 5H), 1.83 - 1.74 (m, 1H). MS (ESI): m/z 501.2 [M+H]$^+$.

**Example E60: 1-(3-hydroxypropyl)-3-methyl-8-(1,4,4-trifluorocyclohexyl)-7-(4-(trifluoromethyl)benzyl)-3,7-dihydro-1H-purine-2,6-dione**

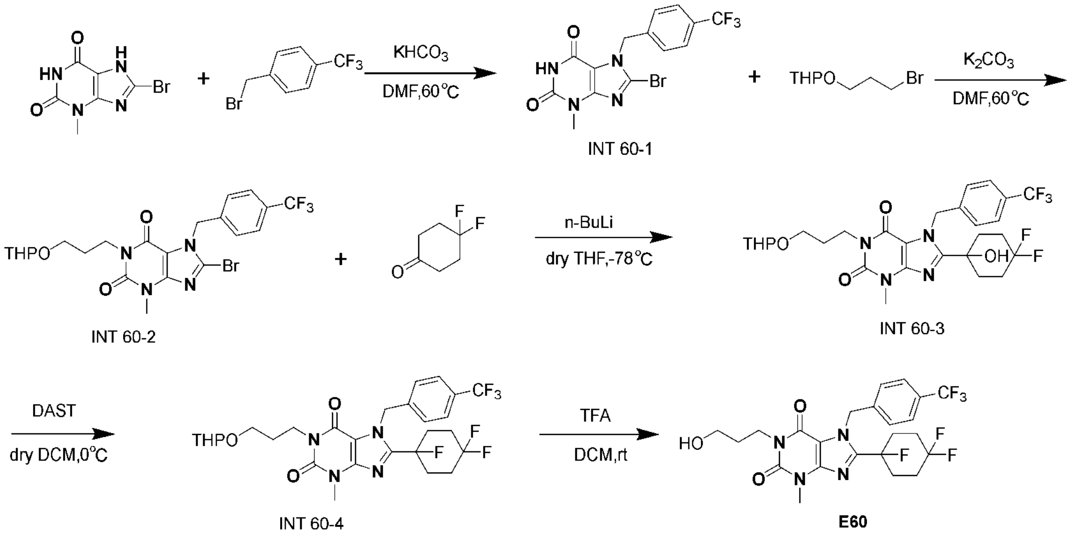


8-bromo-3-methyl-3,7-dihydro-purine-2,6-dione (2 g, 8.13 mmol) was dissolved in 10 ml DMF, $KHCO_3$ (1.298g, 12.987mmol) and 4-(trifluoromethyl)benzyl bromide (1.8g, 7.531mmol) were added, and the mixture was stirred at 60 °C for 2 h. TLC showed that the reaction was complete, 10 ml $H_2O$ was added, and the mixture was filtered and dried to obtain 2.5 g of a white solid, which was the intermediate INT 60-1. INT 60-1 (350mg, 0.87mmol) was dissolved in 5ml DMF, $K_2CO_3$ (244mg, 1.768mmol) and 2-(3-bromopropoxy)tetrahydro-2H-pyran (236.5mg, 1.06mmol) were added, and the mixture was stirred at 60°C for 2h. TLC showed that the reaction was complete. The reaction solution was poured into a saturated NaCl aqueous solution, extracted with ethyl acetate three times, the organic phases were combined, and then washed with a saturated NaCl aqueous solution three times. After dried with anhydrous $MgSO_4$, it was separated by a flash silica gel column, and eluted with MeOH/DCM as the mobile phase. The target product was collected to obtain 300 mg of a colorless oily liquid to obtain the intermediate INT 60-2.

The subsequent synthesis method of Example E60 was the same as that of Example E38, except that INT 60-2 was used as the starting material instead of 4-2. $^1H$ NMR (500 MHz, Chloroform-*d*) δ 7.59 (d, *J* = 8.1 Hz, 2H), 7.29 - 7.24 (m, 2H), 5.84 (s, 2H), 4.15 (t, *J* = 6.0 Hz, 2H), 3.58 (s, 3H), 3.53 - 3.48 (m, 2H), 3.26 (s, 1H), 2.51 - 2.31 (m, 2H), 2.22 - 2.06 (m, 6H), 1.91 - 1.83 (m, 2H). MS (ESI): m/z 519.2 $[M+H]^+$.

**Example E61: 7-(3,5-difluorobenzyl)-1-(3-hydroxypropyl)-3-methyl-8-(1,4,4-trifluorocyclohexyl)-3,7-dihydro-1H-purine-2,6-dione**

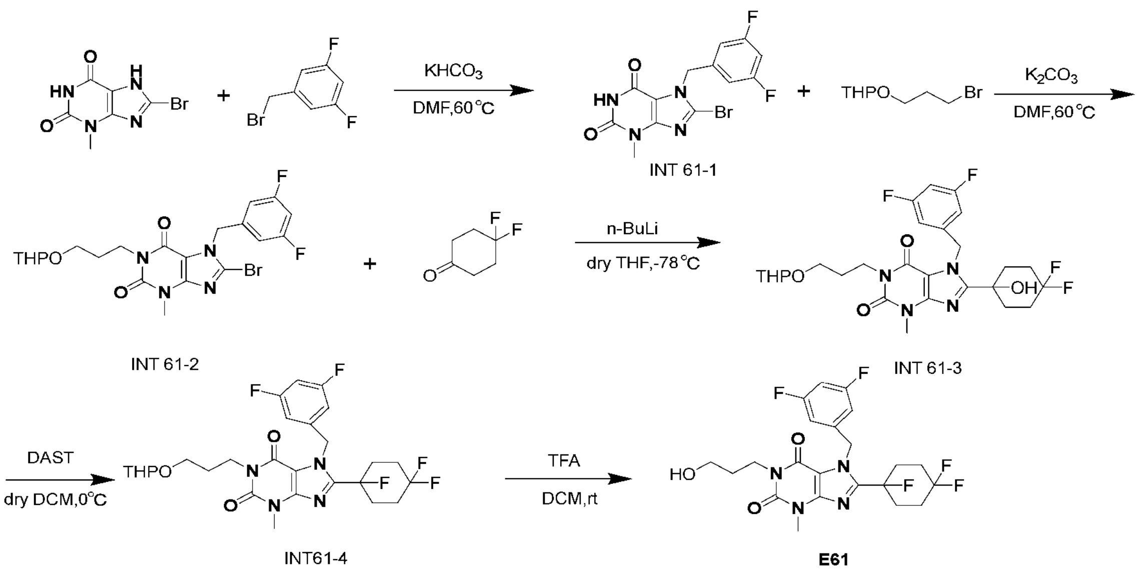


The synthesis method of Example E61 was the same as that of Example E60, except that 3,5-difluorobenzyl

bromide was used as the starting material instead of 4-(trifluoromethyl)benzyl bromide. $^1$H NMR (500 MHz, Chloroform-*d*) δ 6.74 (tt, *J* = 2.3, 8.7 Hz, 1H), 6.70 - 6.64 (m, 2H), 5.75 (s, 2H), 4.16 (t, *J* = 6.1 Hz, 2H), 3.58 (s, 3H), 3.54 - 3.48 (m, 2H), 3.21 (t, *J* = 7.0 Hz, 1H), 2.51 - 2.32 (m, 2H), 2.21 - 2.06 (m, 6H), 1.93 - 1.83 (m, 2H). MS (ESI): m/z 487.2 [M+H]$^+$.

**Example E62: 7-benzyl-1-(3-hydroxypropyl)-3-methyl-8-(1,4,4-trifluorocyclohexyl)-3,7-dihydro-1H-purine-2,6-dione**

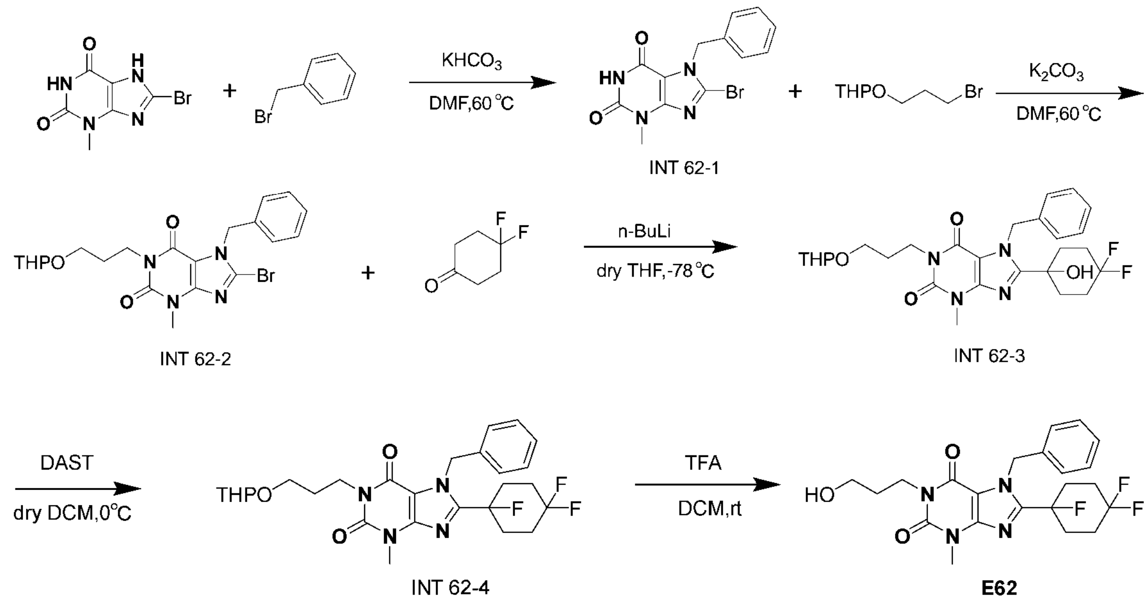

INT 62-1
INT 62-2
INT 62-3
INT 62-4
**E62**

The synthesis method of Example E62 was the same as that of Example E60, except that benzyl bromide was used as the starting material instead of 4-(trifluoromethyl)benzyl bromide. $^1$H NMR (500 MHz, Chloroform-*d*) δ 7.36 - 7.28 (m, 3H), 7.18 - 7.11 (m, 2H), 5.80 (s, 2H), 4.17 (t, *J* = 6.0 Hz, 2H), 3.57 (s, 3H), 3.54 - 3.48 (m, 2H), 2.43 - 2.26 (m, 2H), 2.17 - 2.03 (m, 6H), 1.91 - 1.84 (m, 2H). MS (ESI): m/z 451.2 [M+H]$^+$.

**Example E63: 7-benzyl-8-(1-fluoro-4-(trifluoromethyl)cyclohexyl)-1-(3-hydroxypropyl)-3-methyl-3,7-dihydro-1H-purine-2,6-dione**

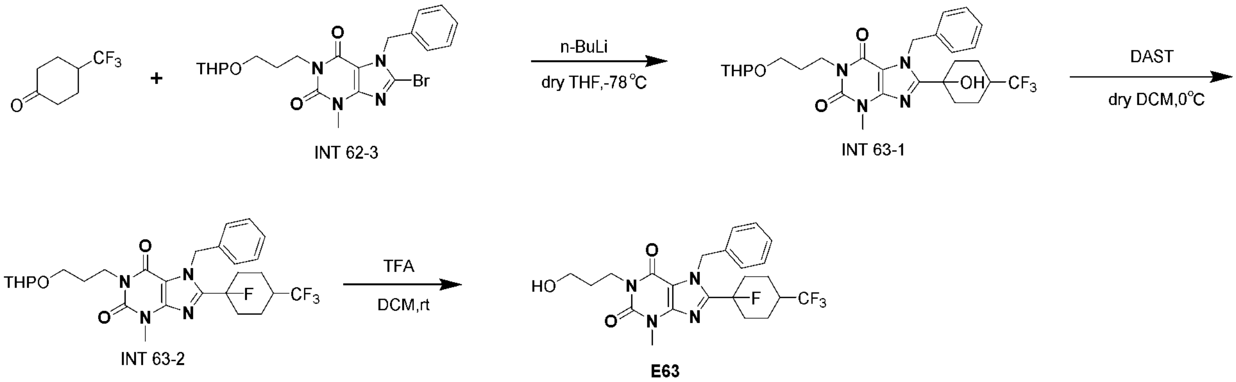

INT 62-3
INT 63-1
INT 63-2
**E63**

The synthesis method of Example E63 was the same as that of Example E62, except that 4-(trifluoromethyl) cyclohexanone was used as the starting material instead of 4,4-difluorocyclohexanone. $^1$H NMR (500 MHz, Chloroform-d) δ 7.36 - 7.28 (m, 3H), 7.13 - 7.06 (m, 2H), 5.79 (s, 2H), 4.15 (t, *J* = 6.0 Hz, 2H), 3.60 (s, 3H), 3.52 - 3.45 (m, 2H), 3.33 (t, *J* = 7.1 Hz, 1H), 2.45 - 2.34 (m, 2H), 2.29 - 2.18 (m, 1H), 1.99 - 1.91 (m, 4H), 1.89 - 1.82 (m, 4H). MS (ESI): m/z 483.2 [M+H]$^+$.

**Example E64: 7-(2,4-difluorobenzyl)-1-(3-hydroxypropyl)-3-methyl-8-(1,4,4-trifluorocyclohexyl)-3,7-dihydro-1H-purine-2,6-dione**

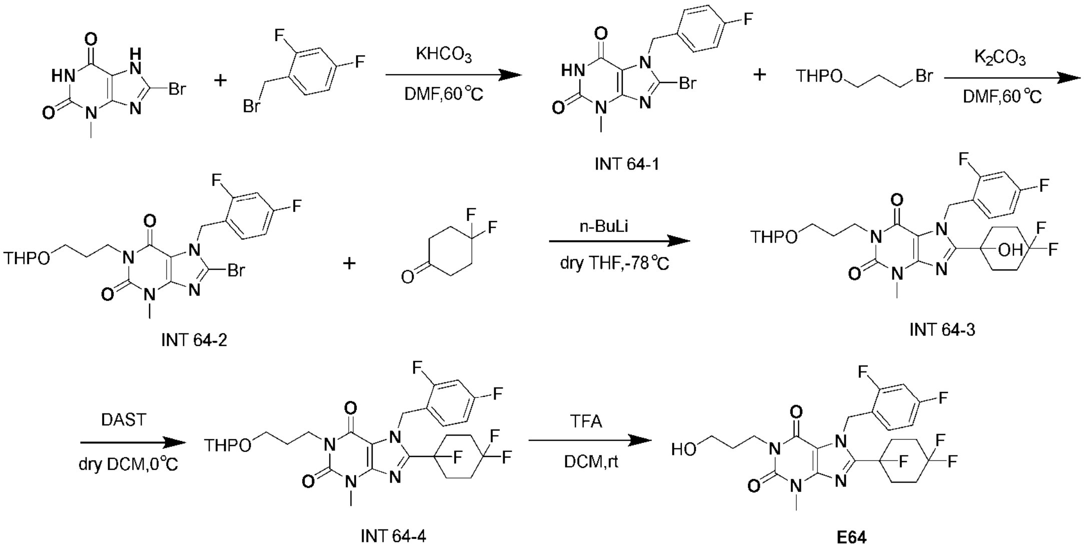

INT 64-1 INT 64-2 INT 64-3 INT 64-4 E64

The synthesis method of Example E64 was the same as that of Example E60, except that 2,4-difluorobenzyl bromide was used as the starting material instead of 4-(trifluoromethyl)benzyl bromide. $^1$H NMR (500 MHz, Chloroform-*d*) δ 6.89 - 6.84 (m, 1H), 6.83 - 6.73 (m, 2H), 5.82 (s, 2H), 4.16 (t, *J* = 6.0 Hz, 2H), 3.58 (s, 3H), 3.53 - 3.47 (m, 2H), 2.47 - 2.30 (m, 2H), 2.19 - 2.04 (m, 6H), 1.91 - 1.83 (m, 2H). MS (ESI): m/z 487.2 [M+H]$^+$.

**Example E65: 7-(2,4-difluorobenzyl)-8-(1-fluoro-4-(trifluoromethyl)cyclohexyl)-1-(3-hydroxypropyl)-3-methyl-3,7-dihydro-1H-purine-2,6-dione**

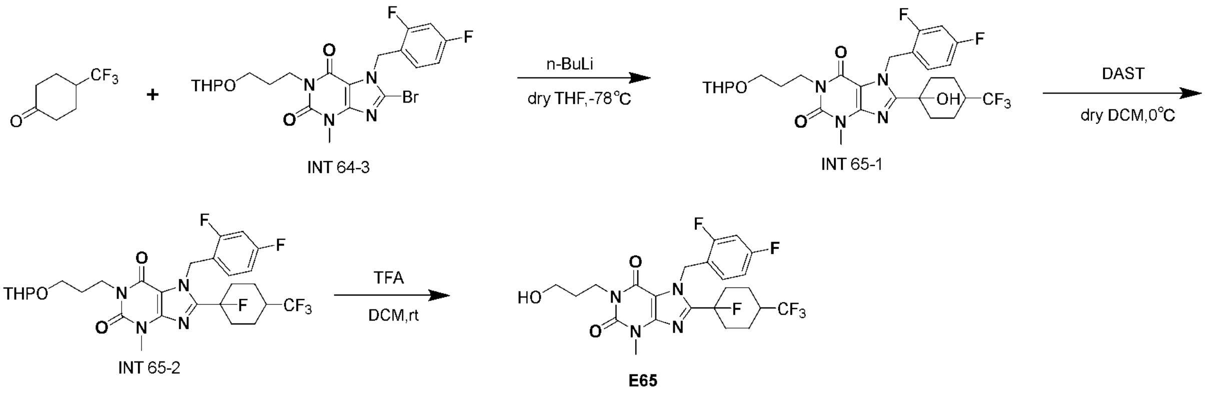

INT 64-3 INT 65-1 INT 65-2 E65

The synthesis method of Example E65 was the same as that of Example E64, except that 4-(trifluoromethyl) cyclohexanone was used as the starting material instead of 4,4-difluorocyclohexanone. $^1$H NMR (500 MHz, Chloroform-*d*) δ 6.89 - 6.83 (m, 1H), 6.82 - 6.75 (m, 1H), 6.72 - 6.64 (m, 1H), 5.79 (s, 2H), 4.14 (t, *J* = 6.0 Hz, 2H), 3.60 (s, 3H), 3.52 - 3.44 (m, 2H), 2.45 - 2.33 (m, 2H), 2.31 - 2.21 (m, 1H), 2.02 - 1.82 (m, 8H). MS (ESI): m/z 519.2 [M+H]$^+$.

**Example E66: 7-(2-chloro-4-fluorobenzyl)-1-(3-hydroxypropyl)-3-methyl-8-(1,4,4-trifluorocyclohexyl)-3,7-dihydro-1H-purine-2,6-dione**

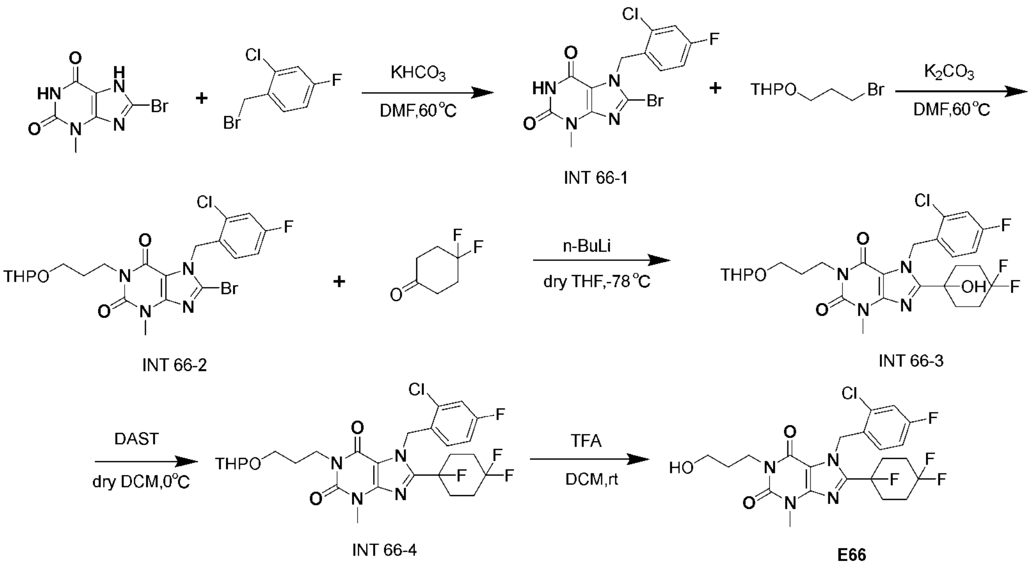


The synthesis method of Example E66 was the same as that of Example E60, except that 2-chloro-4-fluorobenzyl bromide was used as the starting material instead of 4-(trifluoromethyl)benzyl bromide. $^1H$ NMR (500 MHz, Chloroform-*d*) δ 7.19 (dd, *J* = 2.6, 8.2 Hz, 1H), 6.92 - 6.85 (m, 1H), 6.47 - 6.42 (m, 1H), 5.82 (s, 2H), 4.13 (t, *J* = 6.0 Hz, 2H), 3.60 (s, 3H), 3.52 - 3.45 (m, 2H), 2.50 - 2.31 (m, 2H), 2.21 - 2.05 (m, 6H), 1.89 - 1.82 (m, 2H). MS (ESI): m/z 503.1 $[M+H]^+$.

**Example E67: 7-(2-chloro-4-fluorobenzyl)-8-(1-fluoro-4-(trifluoromethyl)cyclohexyl)-1-(3-hydroxypropyl)-3-methyl-3,7-dihydro-1H-purine-2,6-dione**

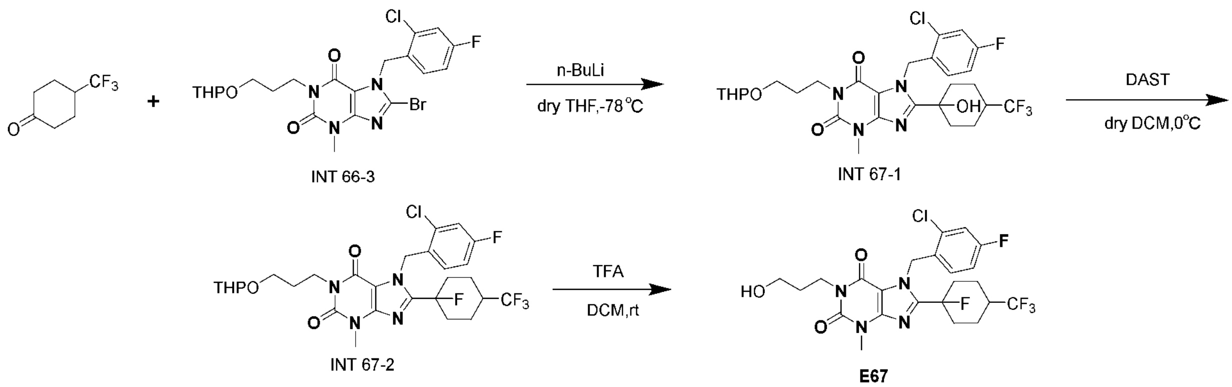


The synthesis method of Example E67 was the same as that of Example E66, except that 4-(trifluoromethyl)cyclohexanone was used as the starting material instead of 4,4-difluorocyclohexanone. $^1H$ NMR (500 MHz, Chloroform-*d*) δ 7.19 (dd, *J* = 2.6, 8.2 Hz, 1H), 6.92 - 6.84 (m, 1H), 6.47 - 6.42 (m, 1H), 5.80 (s, 2H), 4.12 (t, *J* = 6.0 Hz, 2H), 3.62 (s, 3H), 3.52 - 3.44 (m, 2H), 3.17 (s, 1H), 2.48 - 2.37 (m, 2H), 2.30 - 2.19 (m, 1H), 2.01 - 1.88 (m, 6H), 1.87 - 1.82 (m, 2H). MS (ESI): m/z 535.1 $[M+H]^+$.

**Example E68: 7-(3-chloro-4-fluorobenzyl)-1-(3-hydroxypropyl)-3-methyl-8-(1,4,4-trifluorocyclohexyl)-3,7-dihydro-1H-purine-2,6-dione**

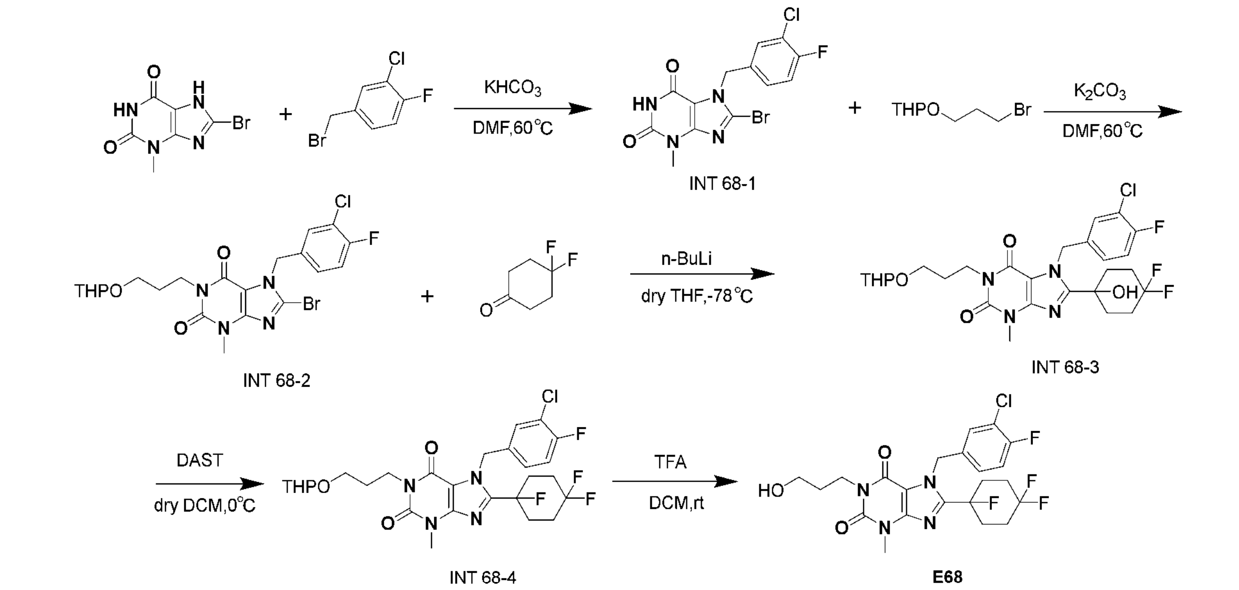


The synthesis method of Example E68 was the same as that of Example E60, except that 3-chloro-4-fluorobenzyl bromide was used as the starting material instead of 4-(trifluoromethyl)benzyl bromide. $^1$H NMR (500 MHz, Chloroform-d) δ 7.29 - 7.26 (m, 1H), 7.10 (d, J = 6.9 Hz, 2H), 5.72 (s, 2H), 4.17 (t, J = 6.0 Hz, 2H), 3.57 (s, 3H), 3.54 - 3.47 (m, 2H), 2.49 - 2.32 (m, 2H), 2.24 - 2.07 (m, 6H), 1.92 - 1.84 (m, 2H). MS (ESI): m/z 503.1 [M+H]$^+$.

**Example E69: 7-(3-chloro-4-fluorobenzyl)-8-(1-fluoro-4-(trifluoromethyl)cyclohexyl)-1-(3-hydroxypropyl)-3-methyl-3,7-dihydro-1H-purine-2,6-dione**

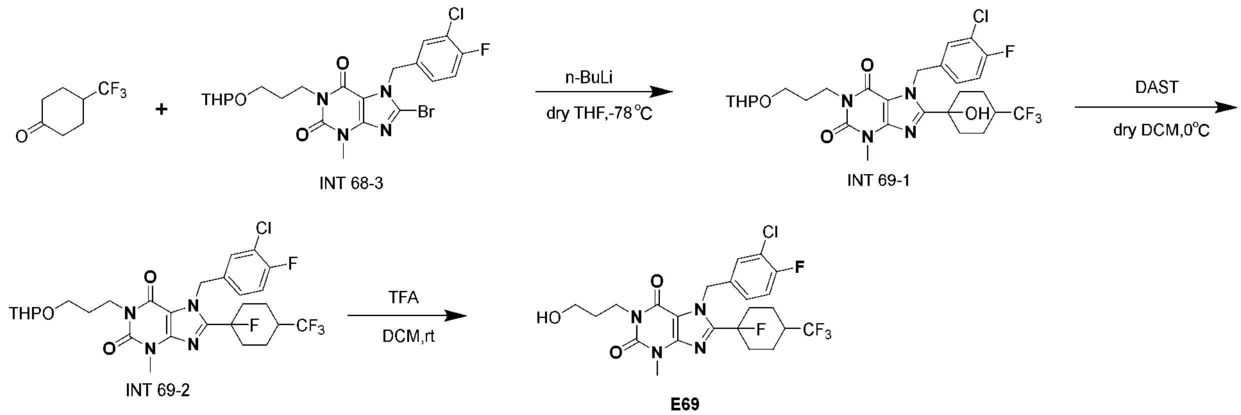


The synthesis method of Example E69 was the same as that of Example E68, except that 4-(trifluoromethyl) cyclohexanone was used as the starting material instead of 4,4-difluorocyclohexanone. $^1$H NMR (500 MHz, Chloroform-*d*) δ 7.25 - 7.21 (m, 1H), 7.13 - 7.04 (m, 2H), 5.69 (s, 2H), 4.16 (t, *J* = 6.0 Hz, 2H), 3.59 (s, 3H), 3.53 - 3.47 (m, 2H), 3.25 (t, *J* = 7.0 Hz, 1H), 2.48 - 2.35 (m, 2H), 2.32 - 2.21 (m, 1H), 2.11 - 1.96 (m, 4H), 1.94 - 1.83 (m, 4H). MS (ESI): m/z 535.1 [M+H]$^+$.

**Example E70: 7-(4-chloro-2-fluorobenzyl)-1-(3-hydroxypropyl)-3-methyl-8-(1,4,4-trifluorocyclohexyl)-3,7-dihydro-1H-purine-2,6-dione**

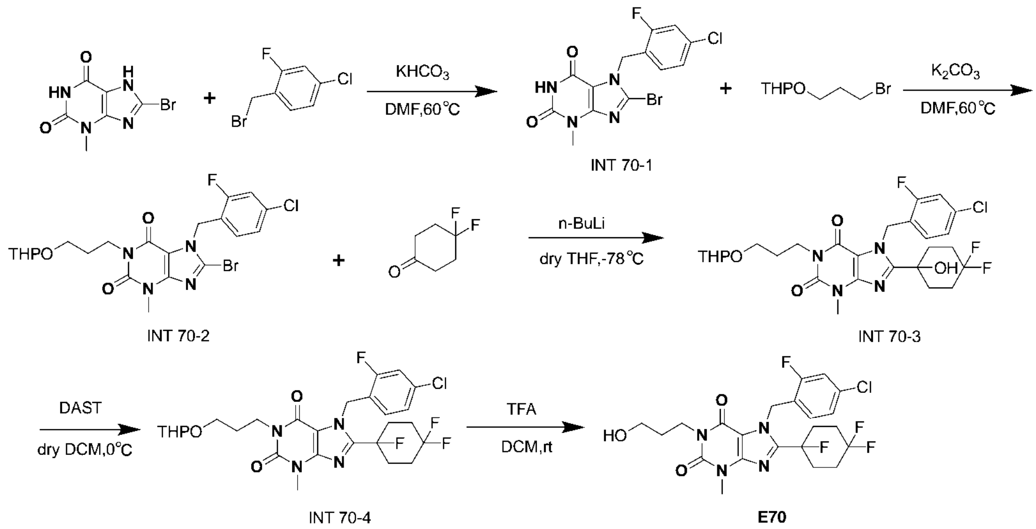


The synthesis method of Example E70 was the same as that of Example E60, except that 4-chloro-2-fluorobenzyl bromide was used as the starting material instead of 4-(trifluoromethyl)benzyl bromide. $^1$H NMR (500 MHz, Chloroform-d) δ 7.17 - 7.12 (m, 1H), 7.08 - 7.03 (m, 1H), 6.71 - 6.65 (m, 1H), 5.82 (s, 2H), 4.14(t, *J* = 6.0 Hz, 2H), 3.58 (s, 3H), 3.53 - 3.47 (m, 2H), 2.48 - 2.31 (m, 2H), 2.19 - 2.05 (m, 6H), 1.90 - 1.82 (m, 2H). MS (ESI): m/z 503.1 [M+H]$^+$.

**Example E71: 7-(4-chloro-2-fluorobenzyl)-8-(1-fluoro-4-(trifluoromethyl)cyclohexyl)-1-(3-hydroxypropyl)-3-methyl-3,7-dihydro-1H-purine-2,6-dione**

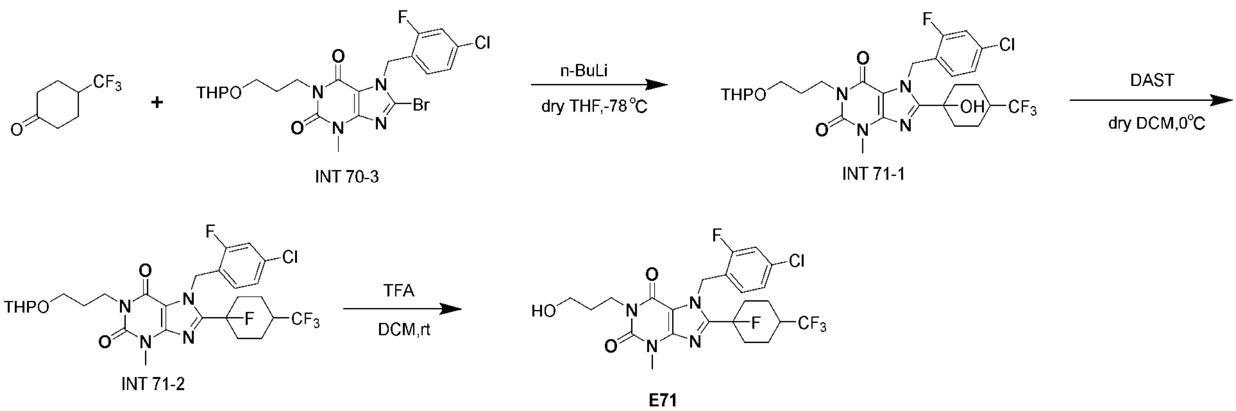


The synthesis method of Example E71 was the same as that of Example E70, except that 4-(trifluoromethyl) cyclohexanone was used as the starting material instead of 4,4-difluorocyclohexanone. $^1$H NMR (500 MHz, Chloroform-d) δ 7.13 (dd, *J* = 2.1, 9.8 Hz, 1H), 7.08 - 7.03 (m, 1H), 6.71 - 6.66 (m, 1H), 5.82 (s, 2H), 4.15 (t, *J* = 6.0 Hz, 2H), 3.58 (s, 3H), 3.51 - 3.46 (m, 2H), 2.26 - 2.10 (m, 4H), 2.09 - 2.01 (m, 1H), 1.97 - 1.91 (m, 2H), 1.90 - 1.83 (m, 2H), 1.79 - 1.67 (m, 2H). MS (ESI): m/z 535.1 [M+H]$^+$.

**Example E72: 4-((1-(3-hydroxypropyl)-3-methyl-2,6-dioxo-8-(1,4,4-trifluorocyclohexyl)-1,2,3,6-tetrahydro-7H-purin-7-yl)methyl)benzonitrile**

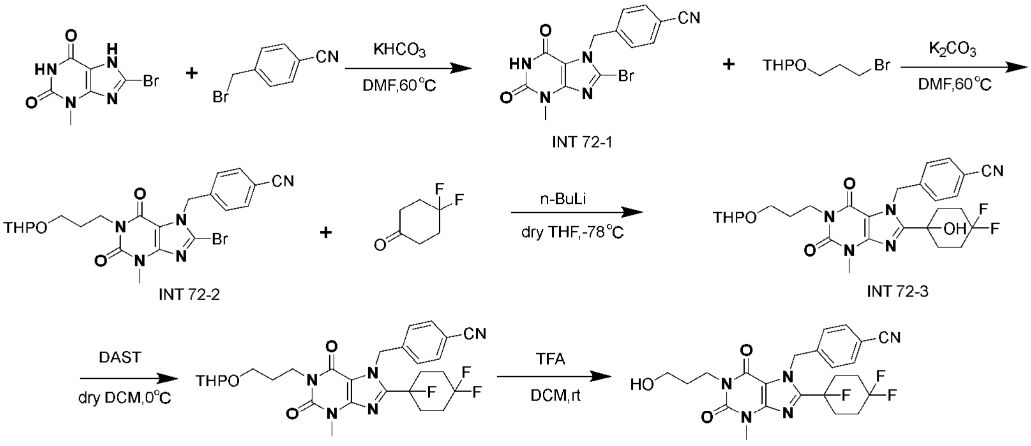


INT 72-1

INT 72-2

INT 72-3

INT 72-4

**E72**

The synthesis method of Example E72 was the same as that of Example E60, except that 4-cyanobenzyl bromide was used as the starting material instead of 4-(trifluoromethyl)benzyl bromide. $^1H$ NMR (500 MHz, Chloroform-d) δ 7.63 (d, *J* = 8.2 Hz, 2H), 7.25 (d, *J* = 8.7 Hz, 2H), 5.83 (s, 2H), 4.15 (t, *J* = 6.1 Hz, 2H), 3.58 (s, 3H), 3.52 - 3.44 (m, 2H), 3.17 (s, 1H), 2.50 - 2.32 (m, 2H), 2.20 - 2.03 (m, 6H), 1.91 - 1.81 (m, 2H). MS (ESI): m/z 476.2 $[M+H]^+$.

**Example E73: 4-((8-(1-fluoro-4-(trifluoromethyl)cyclohexyl)-1-(3-hydroxypropyl)-3-methyl-2,6-dioxo-1,2,3,6-tetrahydro-7H-purin-7-yl)methyl)benzonitrile**

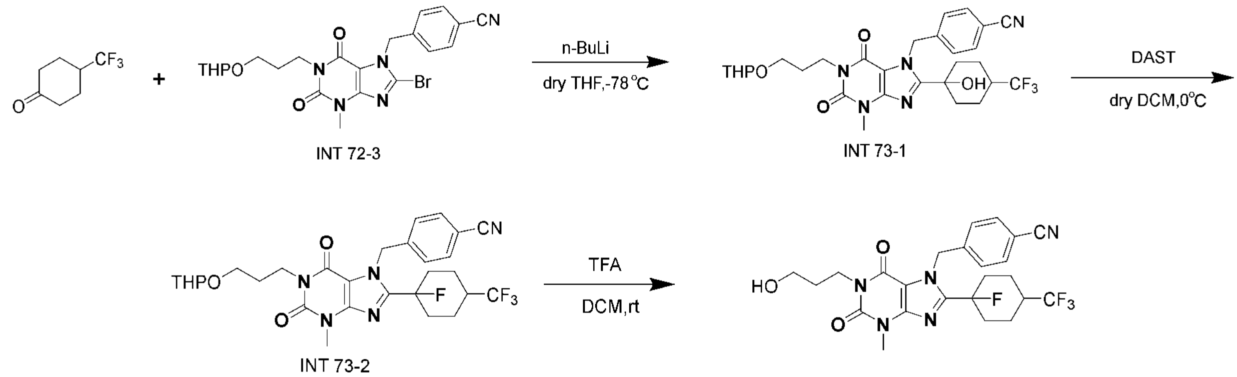


INT 72-3

INT 73-1

INT 73-2

**E73**

The synthesis method of Example E73 was the same as that of Example E72, except that 4-(trifluoromethyl) cyclohexanone was used as the starting material instead of 4,4-difluorocyclohexanone. $^1H$ NMR (500 MHz, Chloroform-d) δ 7.62 (d, *J* = 8.0 Hz, 2H), 7.22 (d, *J* = 8.0 Hz, 2H), 5.81 (s, 2H), 4.13 (t, *J* = 6.0 Hz, 2H), 3.60 (s, 3H), 3.52 - 3.47 (m, 2H), 3.19 (s, 1H), 2.47 - 2.36 (m, 2H), 2.33 - 2.21 (m, 1H), 2.04 - 1.80 (m, 8H). MS (ESI): m/z 508.2 $[M+H]^+$.

## II. Pharmacological Examples

### 1. Evaluation of ion channel activity

#### 1.1 TRPC3/4/5 inhibitory activity assay

HEK-293 cells expressing hTRPC3, hTRPC4 or hTRPC5 were seeded into black-bottomed 96-well plates coated with poly-lysine (PDL), with $2\times10^4$ cells per well. After 8 h of culture, the original culture medium was discarded, and 60 μL of Fluo-4/AM dye with a final concentration of 4 μM was added. The plates were incubated at 37 °C for 60 min, and then rinsed five times with calcium flow detection buffer. The cell plates were placed in FLIPR®TETRA (Molecular Devices, Sunnyvale, CA, USA) preheated at 30 °C, excited at a wavelength of 488 nm, and the fluorescence signals were continuously recorded in the range of 515-535 nm with a sampling frequency of 1 s. After recording for 60 seconds, solvent control, test compounds and positive inhibitor HC608 (compound C31 in WO2014143799, final concentration of 100nM)

were added, and the signal was collected for 300 s. Then, the agonist Englerin A (EA) (MedChemExpress, Shanghai, China, final concentration of 0.3nM) was added, and the fluorescence signal was collected for 600 seconds. The fluorescence signal of the trajectory diagram is represented by $F/F_0$, wherein F is the fluorescence signal at different time points, and $F_0$ is the basic fluorescence signal, that is, the average value of the fluorescence signal at the first 10 time points. The dose-effect diagram first took $F/F_0$ =1 as the baseline. The area under the curve of the change in fluorescence intensity after adding EA was calculated. $IC_{50}$ value was calculated using log[Inhibitor] vs. response - Variable slope, taking into account the area under the curve and the log value of the concentration of the compound.

**1.2 TRPC5 agonist activity assay**

HEK-293 cells expressing hTRPC5 were seeded into PDL-coated black-walled 96-well plates, with $2\times10^4$ cells per well. After 8 hours of culture, the original culture medium was discarded, and 60 μL of 4 μM Fluo-4/AM dye was added immediately. The cells were incubated at 37 °C for 60 minutes, and then rinsed with calcium flow detection buffer 5 times. The cell plates were placed in FLIPR®TETRA (Molecular Devices, Sunnyvale, CA, USA) preheated at 30 °C, excited at a wavelength of 488 nm, and the fluorescence signals were continuously recorded in the range of 515-535 nm with a sampling frequency of 1 s. After recording for 360 seconds, solvent control and test compounds were added, and the fluorescence signal was collected for another 600 seconds. The fluorescence signal of the trajectory diagram is represented by $F/F_0$, wherein F is the fluorescence signal at different time points, and $F_0$ is the basic fluorescence signal, that is, the average value of the fluorescence signal at the first 10 time points. The dose-effect diagram first took $F/F_0$ =1 as the baseline. The area under the curve of the change in fluorescence intensity after adding test compound was calculated. $IC_{50}$ value was calculated using log[agonist] vs. response - Variable slope, taking into account the area under the curve and the log value of the concentration of the compound.

**1.3 hERG channel inhibition assay**

(1) Cell preparation

CHO cells expressing hERG protein were cultured in $175cm^2$ culture flasks. When the cell density grew to 60-80%, the culture medium was removed, and the cells were washed with 7mL PBS, and then 3mL Detachin was added for digestion. After digestion was complete, 7mL culture medium was added for neutralization, and then centrifuged, the supernatant was aspirated, and 5mL culture medium was added for resuspending to ensure that the cell density was $2\text{-}5\times10^6$/mL.

(2) Solution preparation

| Agents | Extracellular fuid (mM) | Intracellular fuid (mM) |
|---|---|---|
| $CaCl_2$ | 2 | 5.374 |
| $MgCl_2$ | 1 | 1.75 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity-305 mOsm | 7.25 (adjusted with KOH), Osmolarity-290 mOsm |

(3) Electrophysiological recording process

The single-cell high-impedance sealing and whole-cell pattern formation process were all automatically completed by the Qpatch instrument. After obtaining the whole-cell recording mode, the cell was clamped at -80 mV. Before giving a 5-second +20 mV depolarizing stimulus, a 50-millisecond -50 mV pre-voltage was given, and then repolarization was maintained at -50 mV for 5 seconds, and then -80 mV voltage was given. This voltage stimulus was applied every 15 seconds. After recording for 2 minutes, the extracellular solution was recorded for 2 minutes, and then the drug administration process began. The compound concentration started from the lowest test concentration, and each test

concentration was given for 2 minutes. After all concentrations were given continuously, the positive control compound 10 μM Cisapride was given. At least 3 cells (n≥3) were tested for each concentration.

(4) Preparation of compounds

The stock solution of compound was diluted with extracellular solution, 5 μL of 20 mM compound stock solution was added to 2495 μL of extracellular solution, 500 times diluted to 40 μM, and then 3 times serial dilution was performed in the extracellular solution containing 0.2% DMSO to obtain the final concentration to be tested. The highest test concentration of the compound was 40 μM, and the concentrations were 40, 13.33, 4.44, 1.48, 0.49 and 0.16 μM, respectively. The highest test concentration of the positive compound Cisapride was 3 μM, and the concentrations were 3, 1, 0.333, 0.111, 0.037 and 0.012 μM, respectively. The DMSO content in the final test concentration did not exceed 0.2%, and this concentration of DMSO had no effect on the hERG potassium channel.

(5) Data analysis: The experimental data were analyzed by XLFit software.

**1.4 Liver microsome stability test**

First, 0.1 M Tris buffer (pH 7.4) was prepared, and then, a 100mM $MgCl_2$ solution and a 10 mM coenzyme factor NADPH solution were prepared with this buffer. The test compound was first prepared as a mother solution from DMSO, and then diluted with water and 0.1% BSA to a working concentration when used. During the assay, the liver microsomes in TRIS buffer (final concentration of 0.33 mg/mL microsomal protein), $MgCl_2$ solution (final concentration of 5mM), the test compound solution (final concentration of 1 μM) and the NADPH solution (final concentration of 1mM) were incubated at 37 °C, and methanol was added at 0, 7, 17, 30 and 60 minutes to terminate the reaction. The residual concentration of the test compound was determined by LC/MS/MS method.

Calculation of half-life:

$$T_{1/2}=0.693/k_e;$$

Calculation of Intrinsic Clearance:

$$Cl_{int} = \frac{1000 \times slope}{P};$$

Calculation of In vivo clearance:

$$Cl = \frac{Cl_{int} \times Houston \times LW}{1000};$$

Calculation of Hepatic clearance:

$$Cl_{hep} = \frac{HBF \times fu \times Cl}{HBF + (fu \times Cl)};$$

Calculation of Metabolic utilization rate:

$$\%MF = 100 - \frac{Cl_{hep} \times 100}{HBF}.$$

$k_e$: slope of the linear regression line (absolute value of slope) when plotting semi-logarithmically, taking the semi-logarithm of the remaining percentage of substrate and plotting the semi-logarithmic graph against the reaction time. P: microsomal protein concentration (mg/mL); Houston: Houston factor (45 mg microsomal protein/g liver); LW: liver weight (g); HBF: liver blood flow (mL/min); fu: unbound fraction (usually fu=1).

## 1.5 Test Results

(1) As shown in Table 1, the compounds of the present invention have moderate to strong inhibitory effects on TRPC5 channels, and the activity of some compounds exceeds that of the reference compound HC-070. Therefore, the compounds of the present invention have potential application value in treating a disease related to excessive activation of TRPC5 channels or overexpression of TRPC5 proteins.

**Table 1: Inhibitory activity of compounds on TRPC5 channels**

| Example | FLIPR $IC_{50}$ (nM) | Example | FLIPR $IC_{50}$ (nM) | Example | FLIPR $IC_{50}$ (nM) |
|---|---|---|---|---|---|
| E1 | 69.36 | E2 | 112.2 | E3 | 50-100 |
| E4 | 22.3 | E8 | 19.31 | E9 | 21 |
| E10 | 28.45 | E11 | 8.79 | E12 | 13.45 |
| E13 | 82.13 | E15 | 76 | E18 | 43.81 |
| E20 | 83.75 | E21 | 5.10 | E22 | 198 |
| E23 | 322 | E24 | 18.55 | E25 | 63.37 |
| E27 | 64.73 | E28 | 455.9 | E29 | 30.21 |
| E30 | 280.5 | E32 | 10.15 | E34 | 49.15 |
| E36 | 12.57 | E38 | 3.59 | E39 | 4.63 |
| E40 | 3.70 | E41 | 55.54 | E42 | 3.84 |
| E43 | 6.12 | E44 | 4.09 | E45 | 6.69 |
| E46 | 1.18 | E47 | 1.67 | E48 | 100 |
| E51 | 3.19 | E52 | 1.30 | E53 | 41.45 |
| E56 | 4.64 | E57 | 52.88 | E60 | 6.87 |
| E61 | 6.97 | E62 | 4.05 | E63 | 0.86 |
| E64 | 5.97 | E65 | 2.85 | E67 | 18.9 |
| E68 | 0.58 | E69 | 0.37 | E70 | 1.2 |
| E71 | 0.27 | E72 | 24.8 | E73 | 1.88 |
| HC-070 | 9.3 | | | | |

(2) As shown in Table 2, some compounds of the present invention have moderate agonist effects on TRPC5 channels. The structures of some compounds with agonist activity and other compounds with inhibitory activity are very similar, such as the comparison of compound Example 14 with Example 15 and Example 9, and the comparison of Example 7 and Example 17 with Example 8, but such subtle structural differences unexpectedly cause significant changes in biological activity. Therefore, the compounds of the present invention can be used to treat a disease related to the inhibition of TRPC5 channels and the loss of TRPC5 protein, or can activate TRPC5 channels to cause calcium ion overload and induce cell death, thereby being used to treat certain types of tumors.

**Table 2: Agonist activity of compounds on TRPC5 channels**

| Example | FLIPR $EC_{50}$ (nM) | Example | FLIPR $EC_{50}$ (nM) | Example | FLIPR $EC_{50}$ (nM) |
|---|---|---|---|---|---|
| E5 | 140.8 | E7 | 36.05 | E14 | 104.5 |
| E17 | 241.9 | E19 | 74.48 | E26 | 100-200 |
| E31 | 102.5 | E37 | 100-200 | E54 | 170 |
| E55 | 700 | | | | |

(3) As shown in Table 3, the compounds of the present invention also have a good inhibitory effect on TRPC4, which is consistent with the biological characteristics of the high homology of TRPC4 and TRPC5. Therefore, the compounds of the present invention also have potential application value in the treatment of a disease related to excessive activation of TRPC4 channels or overexpression of TRPC4 proteins.

On the other hand, compared with HC-070, the compounds of the present invention have lower inhibitory activity on TRPC3 channels, higher relative selectivity for TRPC5 channels, and lower off-target risk.

On the other hand, compared with HC-070, the compounds of the present invention have lower inhibitory activity on hERG channels and higher relative selectivity on TRPC5 channels.

In the field of medicinal chemistry, hERG channel blockade is considered an indicator of cardiac toxicity risk and should be avoided as much as possible. Compared with the prior art, the distinguishing structural feature of the compounds of the present invention is that the 8-position of xanthine is substituted by a "cycloalkyl" rather than an aryl, aryloxy, alkyl, alkoxy, cycloalkoxy and the like. Unexpectedly, this structural difference significantly weakens the inhibitory effect of the compound on the hERG channel, indicating that the compounds of the present invention have a lower risk of cardiac toxicity.

**Table 3: Inhibitory activity and selectivity of compounds on TRPC3, TRPC4, and hERG channels**

| Example | inhibitory activity $IC_{50}$ (μM) | | | | relative selectivity | |
|---|---|---|---|---|---|---|
| | TRPC3 | TRPC4 | TRPC5 | hERG | TRPC3/ TRPC5 | hERG/ TRPC5 |
| E32 | > 10 | 0.0184 | 0.0102 | 7.52 | > 980 | 737 |
| E21 | 2.83 | 0.0083 | 0.0051 | > 40 | 555 | > 7843 |
| E38 | 8.40 | 0.0032 | 0.0036 | 6.54 | 2333 | 1816 |
| E43 | 2.00 | 0.0036 | 0.0061 | 13.01 | 327 | 2133 |
| E44 | > 10 | 0.0067 | 0.0041 | > 40 | > 2439 | > 9756 |
| E45 | > 10 | 0.0040 | 0.0067 | > 40 | > 1492 | > 5970 |
| E52 | 2.53 | 0.0057 | 0.0013 | > 40 | 1946 | > 30769 |
| E60 | | | 0.0069 | 13.35 | | 1935 |
| E61 | | | 0.0070 | > 40 | | > 5714 |
| E62 | | | 0.0041 | > 40 | | > 9756 |
| E64 | | | 0.0060 | > 40 | | > 6667 |
| E68 | | | 0.00058 | > 40 | | > 68965 |
| E70 | | | 0.0012 | > 40 | | > 33333 |
| HC-070 | 2.47 | 0.0162 | 0.0093 | 1.90[a] | 266 | 204 |
| Note: [a], literature value (PLoS ONE. 2018, 13, e0191225.) | | | | | | |

4) As shown in Table 4, the reference compound HC-070 has moderate metabolic stability in human liver microsomes, but is rapidly metabolized in mouse liver microsomes with a short half-life. Compared with HC-070, the compound of the present invention has a longer half-life and a higher metabolic utilization rate, indicating that it is more stable in metabolic properties and has better drug-like properties.

**Table 4: Metabolic stability of compounds in liver microsomes**

| Example | Species | Half-life $T_{1/2}$ (min) | MF % | Stability Evaluation |
|---|---|---|---|---|
| HC-070 | Human | 117 | 52.2 | Medium |
| | Mouse | 11.7 | 19.8 | Worse |
| E38 | Human | 109 | 50.5 | Medium |
| | Mouse | 34.4 | 42.1 | Medium |
| E44 | Human | 216 | 66.8 | Medium |
| | Mouse | 63.8 | 57.5 | Medium |
| E45 | Human | 218 | 67.0 | Medium |
| | Mouse | 68.4 | 59.1 | Medium |

(continued)

| Example | Species | Half-life $T_{1/2}$ (min) | MF % | Stability Evaluation |
|---|---|---|---|---|
| E61 | Human | 366 | 77.4 | Better |
| E62 | Human | 999 | 90.3 | Better |
| E63 | Human | 311 | 74.4 | Better |
| E64 | Human | 282 | 72.5 | Better |
| | Mouse | 61.1 | 56.4 | Medium |
| E68 | Human | 132 | 55.2 | Medium |
| | Mouse | 46 | 49.3 | Medium |
| E72 | Human | 214 | 66.7 | Medium |
| Note: MF%, indicates metabolic bioavailability; metabolic stability criteria: MF% >70%, better metabolic stability; 30%<MF%<70%, medium metabolic stability; MF%<30%, worse metabolic stability. | | | | |

### 2. Evaluation of the inhibitory effect of compounds on hepatic stellate cell activation

#### 2.1 Experimental methods

After primary cultured mouse hepatic stellate cells (HSC) were plated on 96-well black-bottom permeable plates for 24 hours, they were pre-incubated with 1 μM compound E21 and solvent control 0.1% DMSO for 1 hour, and then TGF-β was added to a final concentration of 10 ng/mL, and continued to be cultured in a $CO_2$ incubator for 24 hours. Then the culture medium was aspirated, the plates were washed with 37 °C PBS solution for 3 times, and 4% paraformaldehyde solution was added for 30 minutes for fixation. Then 0.15% Triton X-100 solution was added for permeabilization for 10 minutes; then 5% BSA solution was added for blocking at room temperature for 1 hour; then anti-α-SMA solution (1:300) prepared with 5% BSA solution was added and incubated at 4 °C overnight; the next day, Alexa-488 anti-rabbit fluorescent secondary antibody (1:1000) prepared with 5% BSA solution was added and incubated at room temperature for 1 hour in the dark. After the incubation of the secondary antibody, the cells were washed 3 times with PBS solution, Hoechst solution was added, and incubated in the dark for 20 min at room temperature. After the end, the cells were washed 3 times with PBS solution and fluorescence photography was performed. The FITC filter was used to shoot green fluorescence, and the DAPI filter was used to shoot blue fluorescence. ImageJ software was used to count the total number of cells in each field of view, and the number of α-SMA positive cells was manually counted. GraphPad (Version 6.01) software was used for data processing and analysis.

#### 2.2 Experimental Results

As can be seen from Figure 1, under TGF-β stimulation, the proportion of α-SMA-positive hepatic stellate cells increased from 0.3 ± 0.04% to 11.76 ± 1.47% ($P < 0.01$), indicating that the degree of activation of hepatic stellate cells increased; and the TRPC4/5 inhibitor E21 can significantly reduce the proportion of α-SMA-positive cells to 5.20 ± 0.74% ($P < 0.01$), which can significantly inhibit the activation of hepatic stellate cells, indicating that the compounds of the present invention have the potential to be used for the treatment of liver diseases.

### 3. Preliminary pharmacokinetic evaluation

#### 3.1 Experimental methods

6-8 week old male ICR mice (Nanjing Annuokang Biotechnology Co., Ltd.) were housed in an SPF animal room with a light-to-dark time ratio of 1:1, room temperature controlled at 23 ± 2 °C, humidity controlled at 55%, and mice were able to eat and drink freely. The test compound was prepared according to the following solvent: an aqueous solution of DMSO/Tween 80/0.5% (m/v) CMC-Na at a ratio of 2:2:96 (v/v/v), with a drug concentration of 1 mg/mL. Then, the test compound was gavaged to mice at a dosage of 10 mL/kg, with a dose of 10 mg/kg. Each compound was administered to 3 mice. At 1h and 4h after administration, about 50 μL of blood was collected from the orbital venous plexus of the mouse and placed in a centrifuge tube containing EDTA-K2 anticoagulant. After the blood sample was centrifuged at 3,000 rpm for 15 min, the supernatant was taken to obtain a plasma sample, and then the drug concentration in plasma was determined by liquid chromatography-tandem mass spectrometry (LC-MS/MS).

### 3.2 Test Results

As shown in Table 5, compared with HC-070, the compound of the present invention has higher plasma drug concentrations 1 and 4 hours after oral administration to mice, which preliminarily indicates that the compound of the present invention has better oral absorption properties and plasma exposure, and better pharmacokinetic properties.

Table 5: Plasma drug concentrations 1 and 4 hours after oral administration of 10 mg/kg of the compound to mice (Mean ± SD, N = 3)

| Compound | drug concentration (ng/mL) | |
|---|---|---|
| | 1h | 4h |
| HC-070 | 802 ± 164 | 398 ± 57 |
| E46 | 1172 ± 410 | 576 ± 117 |
| E60 | 1308 ± 45 | 1176 ± 163 |
| E62 | 1734 ± 215 | 785 ± 77 |
| E64 | 1963 ± 190 | 1380 ± 312 |
| E63 | 2799 ± 860 | 2055 ± 755 |
| E68 | 2697 ± 54 | 1890 ± 213 |
| E70 | 1264 ± 167 | 1199 ± 110 |

## 4. Evaluation of Pharmacokinetics and Tissue Distribution in Rats

### 4.1 Drug preparation

Intravenous (IV) administration: the drug to be tested was dissolved in DMSO/Solutol/normal saline at a ratio of 5:5:90 (v/v/v), with a concentration of 0.2 mg/mL, and the administration volume was 5 mL/kg. Oral (PO) administration: the drug to be tested was dissolved in DMSO/Tween 80/0.5% CMC-Na solution at a ratio of 2:2:96 (v/v/v), with a concentration of 1 mg/mL, and the administration volume was 10 mL/kg.

### 4.2 Experimental methods

Male SD rats weighing 180-280g, 3 rats in each group, fasted for 12h before the experiment, had free access to water, and ate uniformly 4 h after administration. In the IV group, the rat tail vein was administered with a dose of 1mg/kg, and blood was collected at 0.083, 0.25, 0.5, 1.0, 2.0, 4.0, 6.0, 8.0, 12 and 24 h after administration. PO group, administered by gavage at a dose of 10 mg/kg, blood was collected at 0.25, 0.5, 1.0, 2.0, 4.0, 6.0, 8.0, 12 and 24 h after administration. At the above time points, 0.2 mL of blood was collected from the jugular vein and placed in an anticoagulant tube containing EDTA-K2. After the whole blood was collected, it was temporarily stored in an ice water bath, centrifuged at 11000 rpm for 5 min within 30min, the plasma was separated, and then frozen in a -70 °C refrigerator for testing. Tissue distribution group, oral administration, dose of 10mg/kg, 2h after drug administration, rats were anesthetized, bled through the abdominal aorta, and immediately dissected to collect brain, heart, liver, spleen, lung, kidney, colon, muscle. Part of the whole blood was collected and operated in an ice bath. The whole blood was centrifuged at 11000 rpm for 5 min within 30 min, and plasma (about 200 μL) was separated. After the tissue and plasma were collected, they were stored below -60 °C for testing. After each tissue was accurately weighed, the homogenizing reagent acetonitrile-water (1:1, v/v) was added for homogenization, wherein the raion of tissue to homogenizing reagent was 1:4 (g:mL). After homogenization, the samples were sonicated for 10 min, and the supernatant was taken after centrifugation for detection. The drug concentration in the samples was quantified using LC-MS/MS method. Relevant pharmacokinetic parameters $T_{max}$, $C_{max}$, $AUC_{0-\infty}$, $t_{1/2}$, etc. were calculated using WinNonlin software.

### 4.3 Test Results

As shown in Table 6, after oral administration of 10 mg/kg compound E68 to rats, the maximum blood concentration of compound E68 is 1600 ng/mL, the half-life is 4.88 h, and the bioavailability is 62.2%, showing good oral pharmacokinetic properties. Figure 2 shows the distribution of compound E68 in tissues 2 hours after oral administration of 10 mg/kg compound E68 to rats. It can be seen that E68 has a high concentration in each tissue, which is

significantly higher than that in plasma. Therefore, the compounds of the present invention can be efficiently distributed to target tissues when used to treat mental illness, neurodegenerative diseases, liver diseases, kidney diseases and other diseases.

Table 6: Plasma pharmacokinetic parameters of compound E68 after oral and intravenous injection in rats (Mean ±SD, N=3)

| Dose | $C_{max}$ | $T_{max}$ | $AUC_{0-\infty}$ | CL | $V_{SS}$ | $t_{1/2}$ | F |
|---|---|---|---|---|---|---|---|
| | (ng/mL) | (h) | (h*ng/mL) | (mL/min/kg) | (L/kg) | (h) | % |
| 10 mg/kg (PO) | 1600 ± 261 | 0.50 ± 0 | 7216 ± 1850 | - | - | 4.88 ± 0.80 | 62.2 ± 15.9 |
| 1 mg/kg (IV) | 772 ± 92 | - | 1171 ± 406 | 15.36 ± 4.98 | 2.94 ± 0.79 | 3.77 ± 2.14 | - |
| HC-070 10 mg/kg PO in mice [a] | 444 | 1.0 | - | - | - | 3.3 | 13 |
| Notes: $C_{max}$, maximum plasma drug concentration; $T_{max}$, peak time of drug concentration; $AUC_{0-\infty}$, area under the drug-time curve from zero to infinity; CL, plasma clearance of the drug; $V_{ss}$, volume of distribution of the drug at steady state; $t_{1/2}$, elimination half-life; F, oral bioavailability; -, not calculated; [a], parameters reported in the literature (PLoS ONE. 2018, 13, e0191225.). | | | | | | | |

**5.Antianxiety and antidepression efficacy experiments**

**5.1 Drug preparation**

The drug to be tested was dissolved in DMSO: Tween 80: distilled water at a ratio of 2:2:96 (v/v/v), and then administered to mice by gavage at a volume of 10 mL/kg. Dosage: positive control Fluoxetine, 10 mg/kg; HC-070, 0.1, 0.3 or 1 mg/kg; compound E68, 0.03, 0.1, 0.3 or 1 mg/kg. The blank control group was only given 10 mL/kg of the above solvent.

**5.2 Experimental methods**

(1) Marble burial test

The mouse marble burial test is a behavioral assay used for the screening of antianxiety drugs. It exploits the natural digging behavior of mice in environments such as burrows or escape tunnels and is sensitive to small changes in behavior caused by genetic manipulation, disease, or drug treatment. The test has been shown to be effective in detecting anxiolytic effects in drug interventions and transgenic mouse models, and can accurately and sensitively measure repetitive and compulsive behaviors in rodents.

A 5cm thick sawdust litter was laid in a 33 cm×24 cm×28 cm carton, and 4×5 black marbles (4 cm apart) with a total of 20 black marbles of 14mm in diameter were neatly placed on the litter. The mouse was put into the carton facing the corner for 30 minutes. After 30 minutes, the mouse was carefully taken out and the number of marbles buried by the mouse was counted. If more than 2/3 of the marble was buried, it was considered a whole marble. First, a blank experiment was conducted. Before the experiment, the mouse was taken to the experimental environment to adapt. After 1 hour, the experiment began. After the experiment, the mouse was returned to the cage and the number of marbles buried by the mouse was counted. On the second day, the mouse was taken to the experimental environment to adapt for 1 hour before the drug was administered. After the drug administration, the marble burying experiment was conducted for 1 hour. After the experiment, the mouse was carefully taken out and returned to the cage to prevent the mouse from escaping violently and affecting the experimental results. After counting, the black marbles were taken out and wiped with alcohol, and the litter was turned over again to remove the smell of the previous mouse to prevent it from affecting the experiment of the next mouse. The marbles were neatly placed for the experiment of the next mouse.

(2) Tail suspension test

The mouse tail suspension test (TST) is a widely used experimental method to evaluate the effectiveness of antidepressants and other psychotropic drugs. The principle of the test is to suspend a mouse by its tail and observe its

behavior. The mouse will initially struggle to escape, but when it realizes that escape is impossible, it will eventually stop struggling and enter a state of immobility that indicates despair. This immobility behavior is used to measure depression-like states, and the severity of the condition is assessed by recording the duration of immobility. Antidepressants and stimulants can significantly shorten the duration of this immobility, which is why the TST is used in the initial screening of these drugs. The test is sensitive to most antidepressants and the efficacy is significantly correlated with clinical efficacy, which makes it the initial testing standard for antidepressant drugs.

The same mouse was subjected to the tail suspension test immediately after the marble burying test. A tail suspension test box was built using a black frosted acrylic board, 55 cm high, 15 cm long, and 11.5 cm wide. The three sides and top of the box were frosted blackboards, and one side was used for video recording. The mouse tail was wrapped with yellow tape 2 cm away from the tip of the tail, and the mouse was hung upside down in the middle of the top acrylic board. The tip of the mouse tail was about 3 cm away from the acrylic board, and the body was suspended and could not touch the surrounding board wall. The test time for each mouse lasted for 6 minutes, and the immobility time of the mouse during this time was counted. After the experiment, the mouse was returned to the cage, and the inside of the box and the table were wiped with alcohol to remove the smell of the previous mouse to prevent affecting the next mouse experiment. The criteria for immobility in the tail suspension test: stop struggling, no flexion or twisting of the body, no swinging of the hind limbs, and slight swinging of the forelimbs and head were also counted as immobility time.

### 5.3 Experimental Results

As shown in Figures 3 and 4, in the marble burial test, the number of marbles buried by mice in the blank control group did not change significantly before and after administration; however, after administration of Fluoxetine (10 mg/kg), HC-070 (1 mg/kg) and E68 (0.1-1 mg/kg), the number of marbles buried by mice was significantly reduced, indicating that the drugs have significant antianxiety effects. In the tail suspension test, the immobility time of mice in the blank control group did not change significantly before and after administration; however, after administration of compound E68, HC-070 or Fluoxetine, the immobility time of mice was significantly reduced, indicating that the drugs have significant antidepressant effects.

In another test conducted after reducing the dosage (Figures 5 and 6), oral administration of 0.03 mg/kg of compound E68 can reduce the number of buried marbles, and the effect is more obvious when administered 0.1 mg/kg of E68, and is better than the same dose of HC-070. In addition, oral administration of 0.03 mg/kg of compound E68 can also significantly reduce the immobility time of mice in the tail suspension box, and its effect is comparable to 0.1 mg/kg of HC-070. It can be seen that compared with HC-070, the compound E68 of the present invention has a more potent anti-anxiety and anti-depressant effect.

All documents mentioned in the present invention are cited as references in this application, just as each document is cited as reference individually. In addition, it should be understood that after reading the above teachings of the present invention, those skilled in the art can make various changes or modifications to the present invention, and these equivalent forms also fall within the scope defined by the claims attached to this application.

## Claims

1. A compound represented by general formula (I-a), a stereoisomer and pharmaceutically acceptable salt thereof;

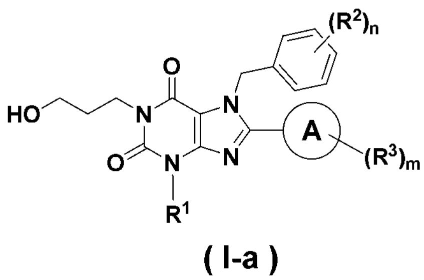

**( I-a )**

wherein, $R^1$ is C1-C6 alkyl or C1-C6 haloalkyl;
n is an integer from 1 to 4, and each $R^2$ is independently H, deuterium, halogen, C1-C6 alkyl, C1-C6 haloalkyl, C1-C6 alkoxy, C1-C6 haloalkoxy, or cyano;
ring A is a saturated or partially unsaturated C3-C10 cyclic hydrocarbyl;
m is an integer from 1 to 6, each $R^3$ is independently selected from H, deuterium, C1-C6 alkyl, C1-C6 haloalkyl, C1-C6 alkoxy, C1-C6 haloalkoxy, C3-C8 cycloalkyl, C6-C10 aryl, 5-8 membered heteroaryl, 4-8 membered

heterocyclyl, halogen, cyano, hydroxyl, or -$NR^4R^5$; or $R^3$ and the connected carbon on the A ring together form a 4-8 membered heterocycle or C6-C10 aryl; the cycloalkyl, aryl, heteroaryl, heterocyclyl is unsubstituted or optionally substituted by 1-4 groups selected from a group consisting of C1-C6 alkyl, C1-C6 haloalkyl, C1-C6 alkoxy, C1-C6 haloalkoxy, halogen, and cyano;

$R^4$ and $R^5$ are each independently H, C1-C6 alkyl, or C3-C8 cycloalkyl.

**2.** The compound of claim 1, wherein ring A is a saturated or partially unsaturated C3-C8 monocyclic hydrocarbyl or bridged cyclic hydrocarbyl.

**3.** The compound of claim 1, wherein $R^1$ is a C1-C4 alkyl.

**4.** The compound of claim 1, wherein n is 1, 2 or 3, and each $R^2$ is independently H, deuterium, halogen, C1-C4 alkyl, C1-C4 haloalkyl, C1-C4 alkoxy, C1-C4 haloalkoxy, or cyano.

**5.** The compound of claim 1, wherein m is 1, 2, 3 or 4, and each $R^3$ is independently selected from H, deuterium, C1-C4 alkyl, C1-C4 haloalkyl, C1-C4 alkoxy, C1-C4 haloalkoxy, C3-C6 cycloalkyl, phenyl, 5-6 membered heteroaryl, 5-6 membered heterocyclyl, F, Cl, Br, cyano, hydroxyl, or -$NR^4R^5$; or $R^3$ and the connected carbon on the A ring together form a 5-6 membered heterocycle or phenyl; the cycloalkyl, phenyl, heteroaryl, heterocyclyl is unsubstituted or optionally substituted with 1-4 groups selected from a group consisting of C1-C6 alkyl, C1-C6 haloalkyl, C1-C6 alkoxy, C1-C6 haloalkoxy, halogen, and cyano.

**6.** The compound of claim 1, wherein it has a structure as shown in general formula (I-b);

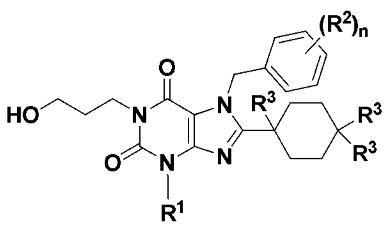

**( I-b )**

wherein, $R^1$ is a C1-C6 alkyl;

n is an integer from 1 to 4, and each $R^2$ is independently H, a halogen atom, C1-C6 haloalkyl or CN;

each $R^3$ is independently selected from H, deuterium, C1-C6 alkyl, C1-C6 haloalkyl, C1-C6 alkoxy, C1-C6 haloalkoxy, C6-C10 aryl, C3-C8 cycloalkyl, 5-8 membered heteroaryl, 4-8 membered heterocyclyl, halogen atom, cyano, hydroxyl, or -$NR^4R^5$; or two $R^3$ and the connected carbon on the A ring together form a 4-8 membered heterocyclic ring;

$R^4$ and $R^5$ are each independently H or C1-C6 alkyl.

**7.** The compound of claim 1, wherein the compound is selected from:

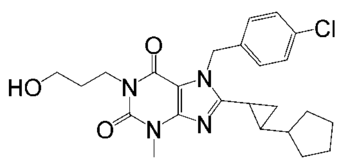 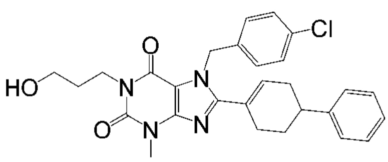 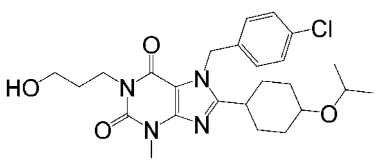

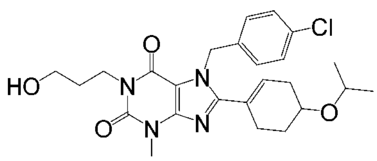 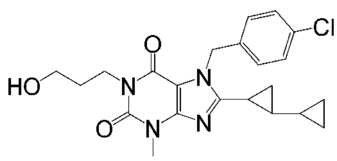 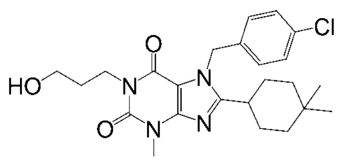

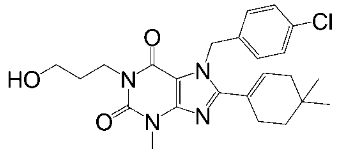 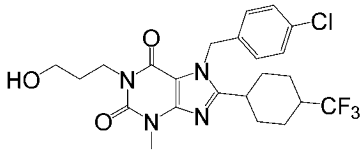 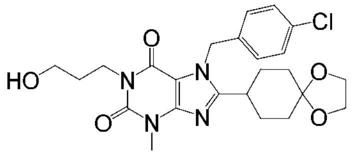

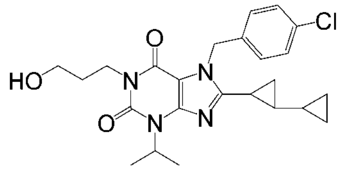 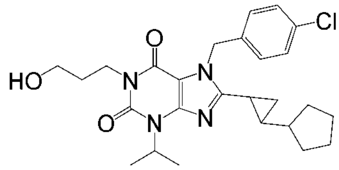 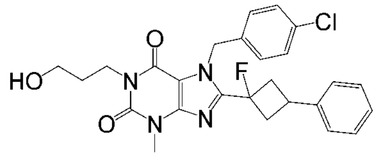

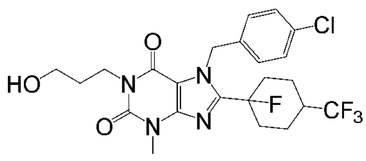 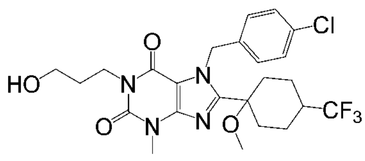 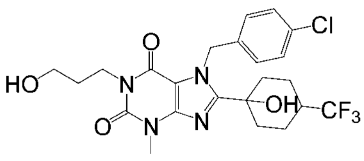

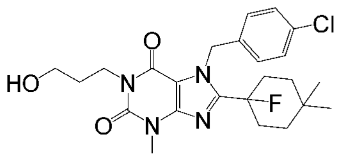 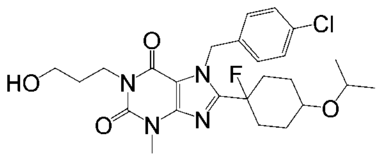 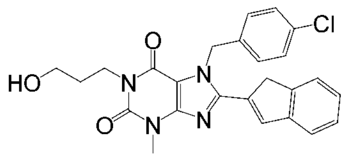

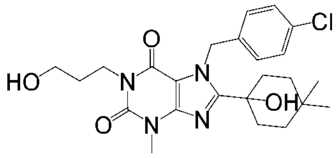 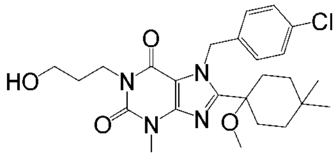 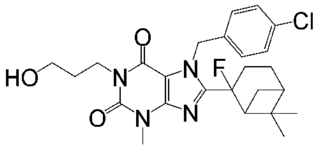

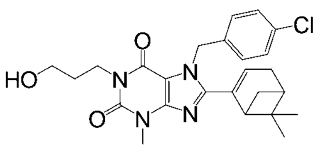 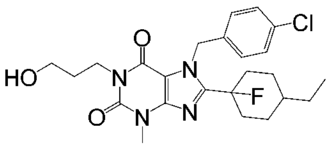 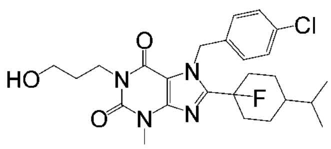

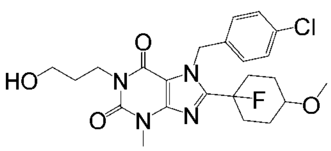 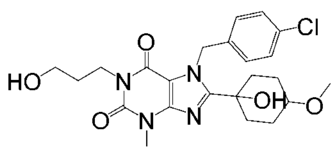 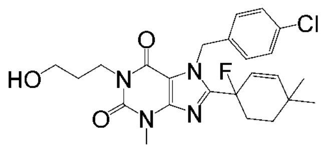

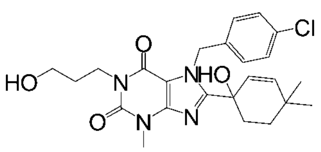 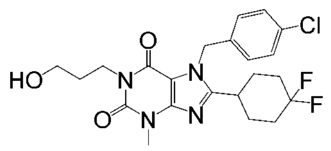 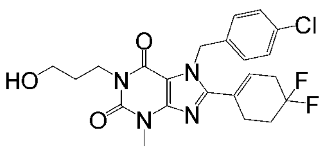

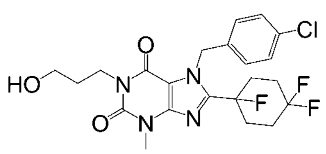 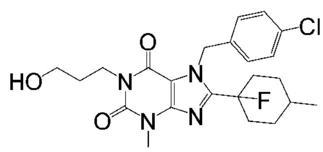 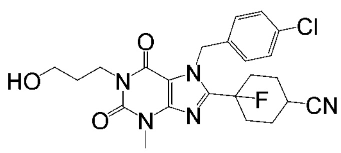

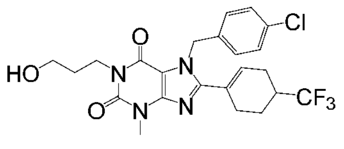
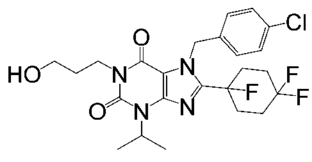
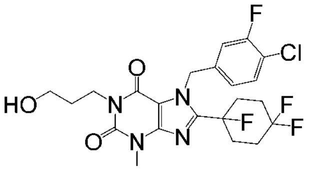

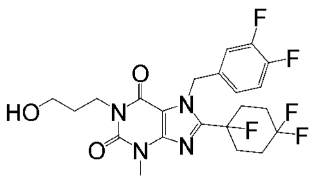
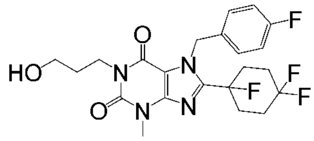
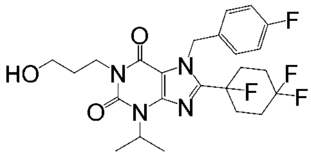

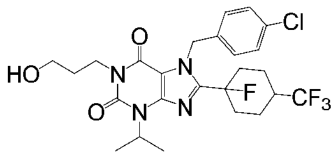
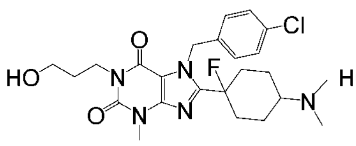
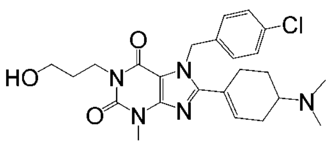

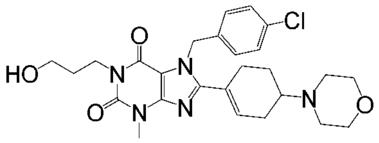
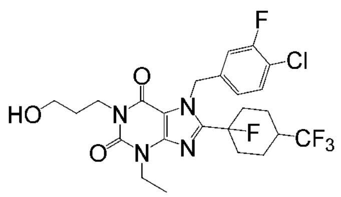
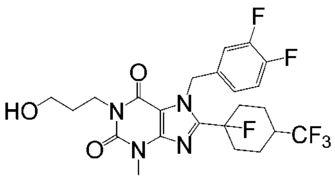

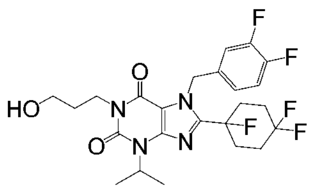
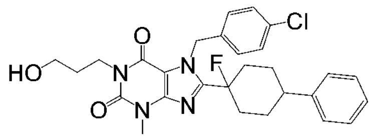
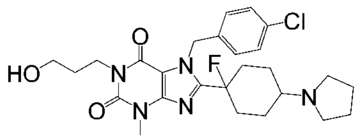

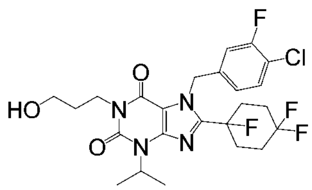
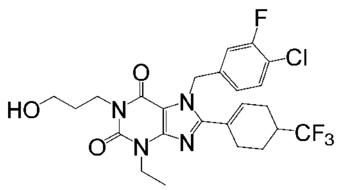
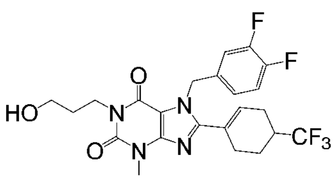

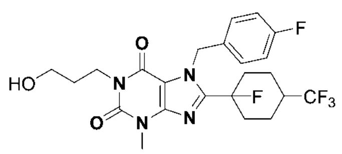
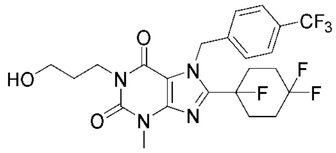
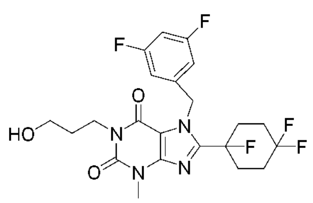

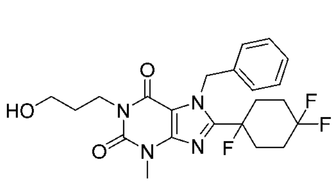
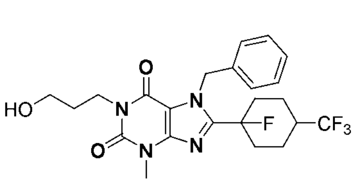
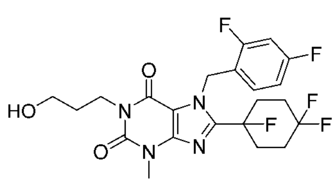

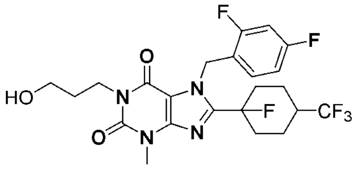
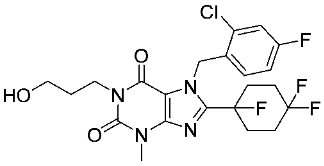
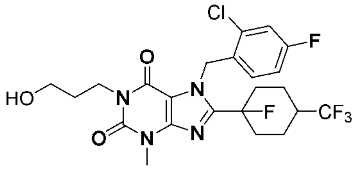

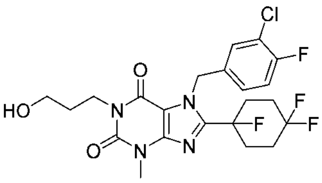
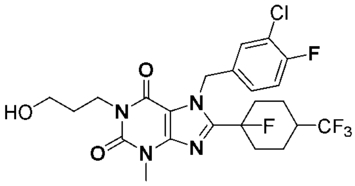
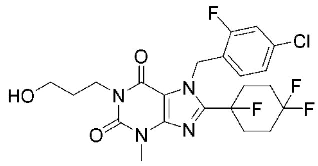

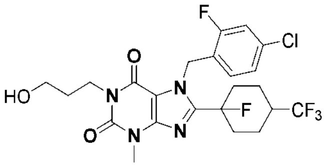
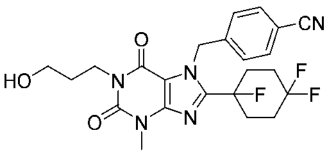
or
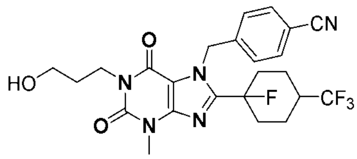
.

**8.** A pharmaceutical composition, comprising the compound of any one of claims 1 to 7, the stereoisomer and pharmaceutically acceptable salt thereof, and a pharmaceutically acceptable carrier.

**9.** Use of the compound of claims 1 to 7 or the pharmaceutical composition of claim 8 for the manufacture of an inhibitor or agonist of TRPC4 and TRPC5 channels, or for the manufacture of a medicament for preventing, delaying or treating a disease related to abnormal function or expression of TRPC4 and/or TRPC5.

**10.** The use of claim 9, wherein the disease includes mental illness (such as borderline personality disorder, depression, dysthymia, postpartum depression, bipolar disorder, post-traumatic stress disorder, panic disorder, agoraphobia, social phobia, generalized anxiety disorder, social anxiety disorder, separation anxiety, schizophrenia, mania, obsessive-compulsive disorder, apathy, neurasthenia, paranoia, etc.), neurodegenerative disease (such as Alzheimer's disease, Parkinson's disease, Huntington's disease, amyotrophic lateral sclerosis, memory impairment, amnesia, aphasia, chronic fatigue syndrome, Creutzfeldt-Jakob disease, dissociative amnesia, fugue amnesia, learning disability, sleep disorder, and other brain diseases caused by trauma or aging), kidney disease (such as kidney damage caused by toxic substances, kidney damage caused by viral or bacterial infection, hypertensive nephropathy, diabetic nephropathy, glomerulonephritis, lupus erythematosus nephritis, IgA nephropathy, nephrotic syndrome, membranous nephropathy, minimal change nephropathy, polycystic kidney disease, focal segmental glomerulosclerosis, etc.), pain (such as nociceptive pain, mechanical pain, inflammatory pain, cancer pain and neuropathic pain), epilepsy, liver disease (such as cholestatic liver disease, alcoholic fatty liver disease, non-alcoholic fatty liver disease, viral hepatitis, autoimmune liver disease, chemical liver injury, liver fibrosis, cirrhosis, hepatocellular carcinoma), cardiovascular disease (such as hypertension, atherosclerosis, coronary heart disease, angina pectoris, myocardial infarction, arrhythmia, stroke, pulmonary hypertension), cancer (such as renal cell carcinoma, breast cancer, colorectal cancer), skin disease (such as psoriasis, ichthyosis, palmoplantar keratoderma, Olmsted's disease, toad skin disease or menopausal keratoderma, etc.), intestinal disease (such as ulcerative colitis, short bowel syndrome, irritable bowel syndrome, intestinal cramp, diarrhea, abdominal pain, etc.); in another preferred embodiment, the disease is borderline personality disorder, depression, anxiety disorder, post-traumatic stress disorder.

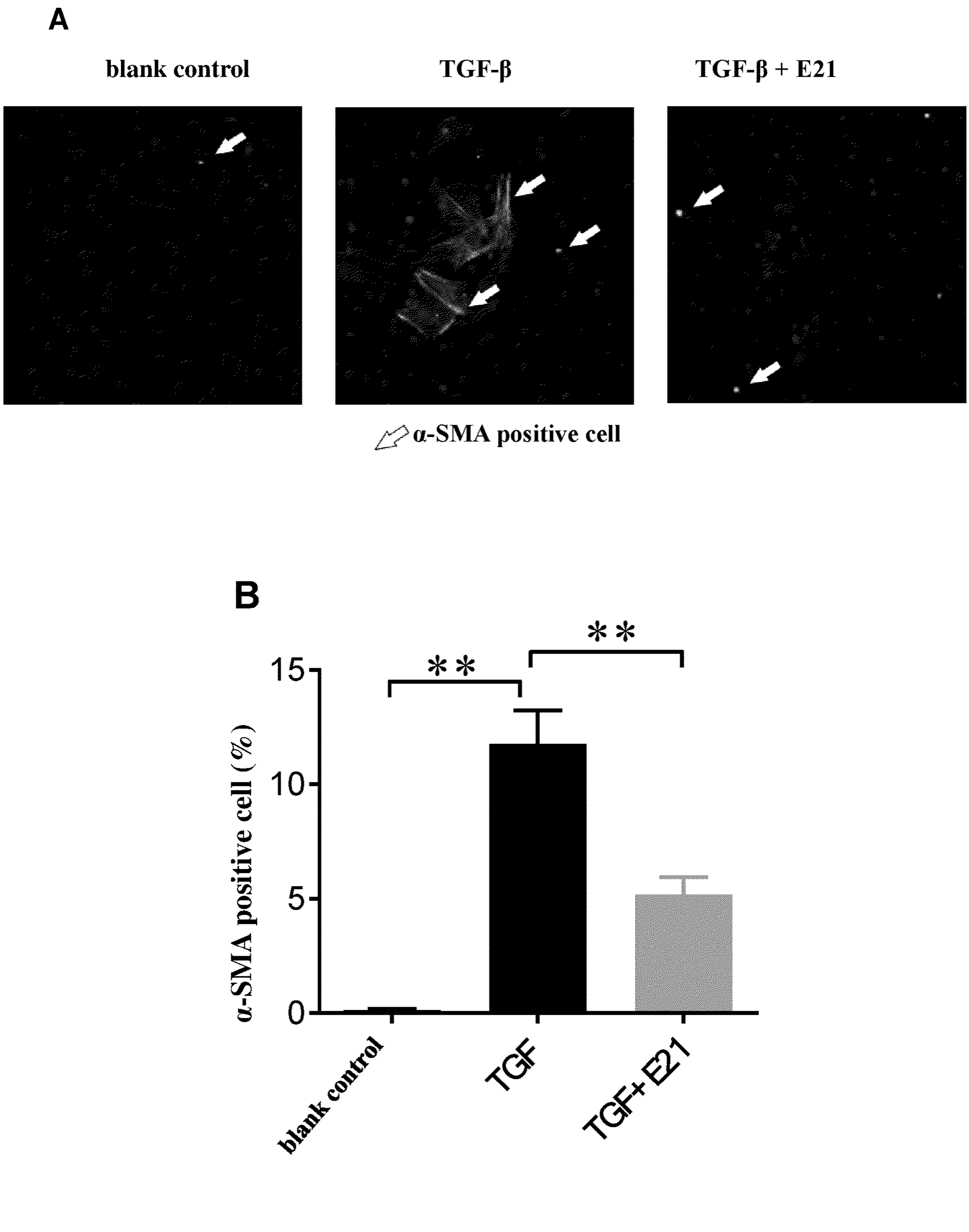
A
blank control
TGF-β
TGF-β + E21
α-SMA positive cell
B
**
**
15
10
5
0
α-SMA positive cell (%)
blank control
TGF
TGF+ E21

Figure 1

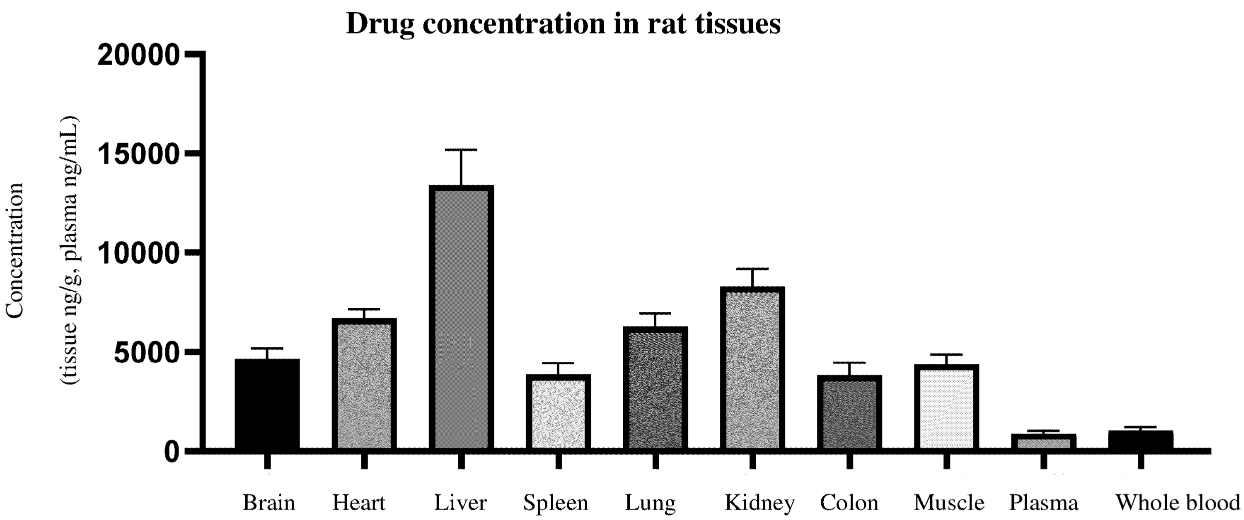
Drug concentration in rat tissues
Concentration
(tissue ng/g, plasma ng/mL)
20000
15000
10000
5000
0
Brain
Heart
Liver
Spleen
Lung
Kidney
Colon
Muscle
Plasma
Whole blood

Figure 2

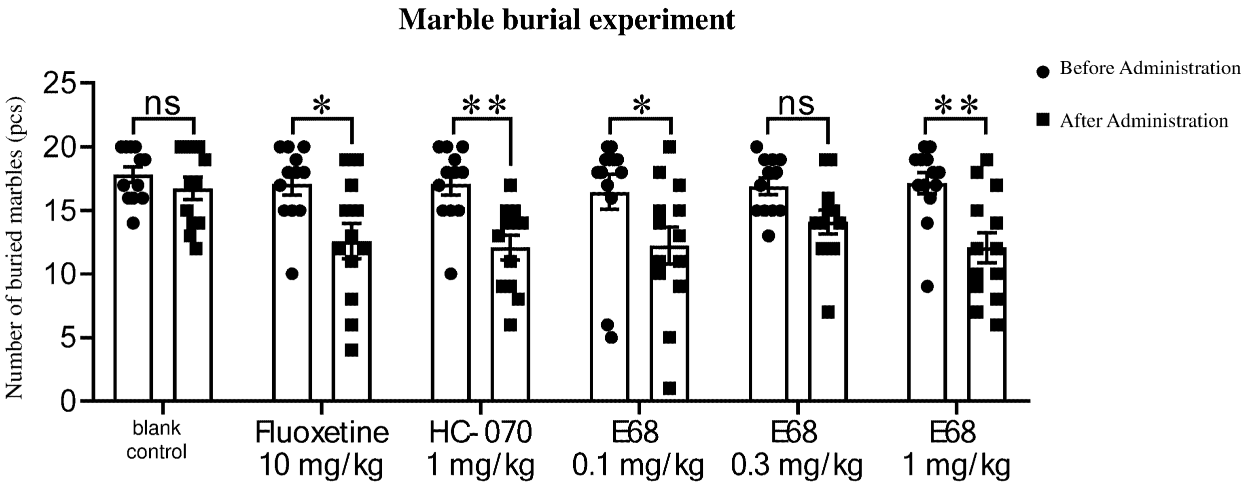
Marble burial experiment
Before Administration
After Administration
Number of buried marbles (pcs)
25
20
15
10
5
0
ns
*
**
*
ns
**
blank control
Fluoxetine 10 mg/kg
HC-070 1 mg/kg
E68 0.1 mg/kg
E68 0.3 mg/kg
E68 1 mg/kg

Figure 3

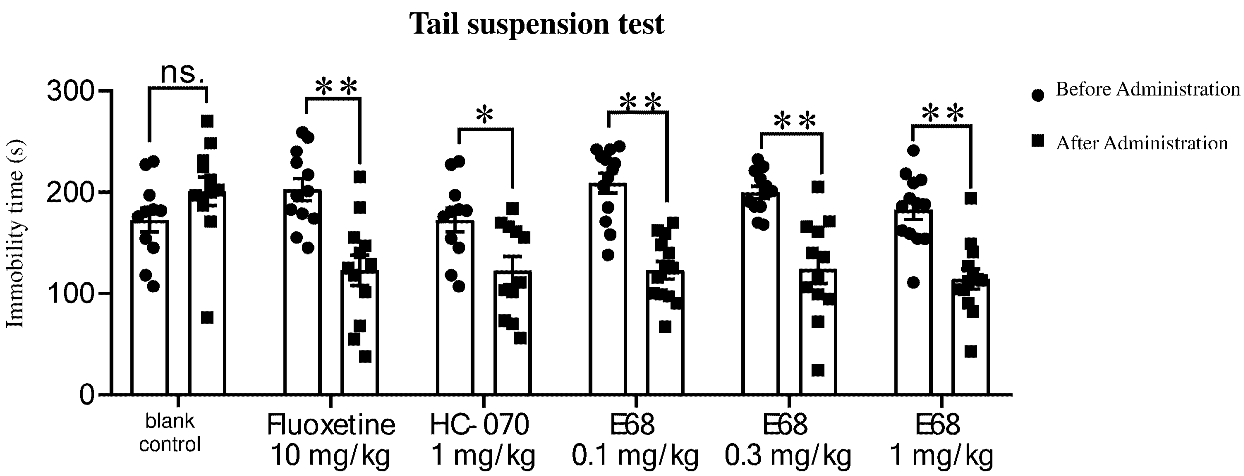
Tail suspension test
Immobility time (s)
300
200
100
0
ns.
**
*
**
**
**
blank control
Fluoxetine 10 mg/kg
HC-070 1 mg/kg
E68 0.1 mg/kg
E68 0.3 mg/kg
E68 1 mg/kg
Before Administration
After Administration

Figure 4

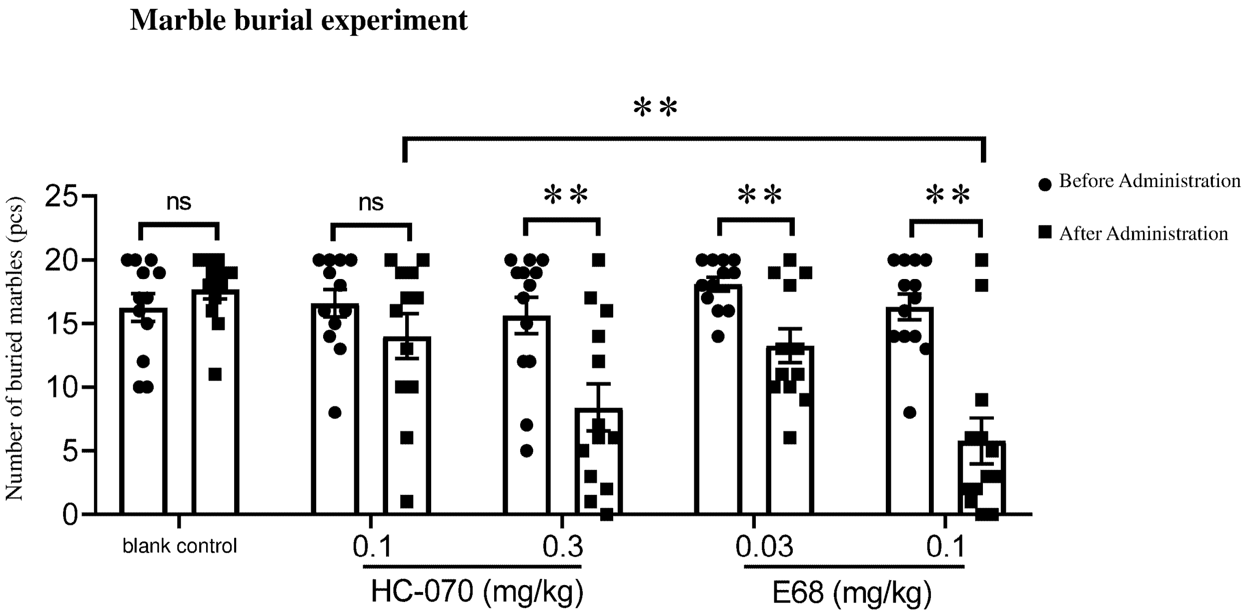
Marble burial experiment
**
ns
ns
**
**
**
Before Administration
After Administration
Number of buried marbles (pcs)
25
20
15
10
5
0
blank control
0.1
0.3
0.03
0.1
HC-070 (mg/kg)
E68 (mg/kg)

Figure 5

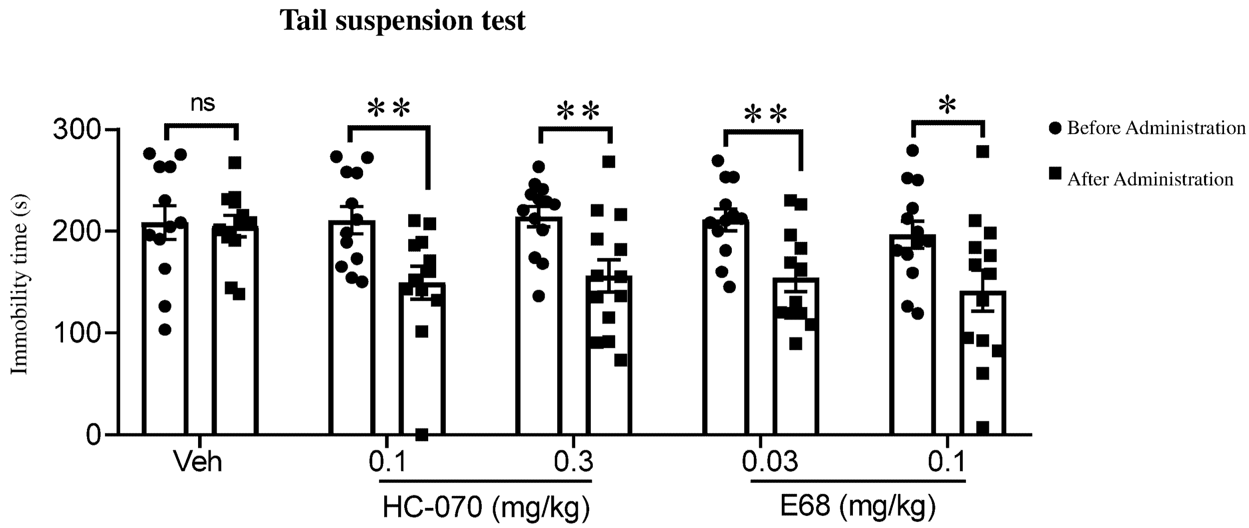
Tail suspension test
Immobility time (s)
300
200
100
0
ns
**
**
**
*
Veh
0.1
0.3
0.03
0.1
HC-070 (mg/kg)
E68 (mg/kg)
Before Administration
After Administration

Figure 6

**INTERNATIONAL SEARCH REPORT**

International application No.
**PCT/CN2024/072849**

**A. CLASSIFICATION OF SUBJECT MATTER**

C07D 473/04(2006.01)i; C07D487/04(2006.01)i; A61K31/519(2006.01)i; A61P25/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, ENTXTC, CAPLUS (STN), REGISTRY (STN), CNKI: 中国科学院上海药物研究所, 中国药科大学, 王凯, 曹征宇, 沈建华, 刘梦茹, 宋兆祥, 胡一信, 许惕非, 赵芳, 梁化端, TRPC?, TRP?, TRP+, 黄嘌呤, xanthine, structural formula search.

**C. DOCUMENTS CONSIDERED TO BE RELEVANT**

| Category* | Citation of document, with indication, where appropriate, of the relevant passages | Relevant to claim No. |
|---|---|---|
| A | CN 105143229 A (HYDRA BIOSCIENCES INC.) 09 December 2015 (2015-12-09) claims 23-35, and description, page 220 | 1-10 |
| A | CN 113166150 A (BOEHRINGER INGELHEIM INTERNATIONAL GMBH et al.) 23 July 2021 (2021-07-23) claims 1-11, and description, page 9 | 1-10 |
| A | CN 113166151 A (BOEHRINGER INGELHEIM INTERNATIONAL GMBH et al.) 23 July 2021 (2021-07-23) claims 1-11, and description, page 10 | 1-10 |
| A | CN 110997675 A (BOEHRINGER INGELHEIM INTERNATIONAL GMBH et al.) 10 April 2020 (2020-04-10) claims 1-16 | 1-10 |
| A | CN 115636831 A (CHINA PHARMACEUTICAL UNIVERSITY) 24 January 2023 (2023-01-24) claims 1-10, and description, page 24 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 March 2024** | **25 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2024/072849**

**C. DOCUMENTS CONSIDERED TO BE RELEVANT**

| Category* | Citation of document, with indication, where appropriate, of the relevant passages | Relevant to claim No. |
|---|---|---|
| A | WO 2021067946 A1 (GOLDFINCH BIO, INC.) 08 April 2021 (2021-04-08) description, pages 23-30 | 1-10 |
| A | WO 2014152287 A2 (HYDRA BIOSCIENCES, INC.) 25 September 2014 (2014-09-25) claims 1-25, and description, page 29 | 1-10 |

Form PCT/ISA/210 (second sheet) (July 2022)

**INTERNATIONAL SEARCH REPORT**
**Information on patent family members**

International application No.
**PCT/CN2024/072849**

| Patent document cited in search report | Publication date (day/month/year) | Patent family member(s) | Publication date (day/month/year) |
|---|---|---|---|
| CN 105143229 A | 09 December 2015 | HUE 033528 T2 | 28 December 2017 |
| | | HUE 033528 T4 | 02 May 2018 |
| | | IL 240216 A0 | 24 September 2015 |
| | | IL 240216 B | 29 November 2018 |
| | | US 2019322667 A1 | 24 October 2019 |
| | | US 11208409 B2 | 28 December 2021 |
| | | US 2016237089 A1 | 18 August 2016 |
| | | MX 2015011617 A | 25 April 2016 |
| | | MX 370372 B | 11 December 2019 |
| | | SG 11201506479 TA | 29 September 2015 |
| | | ES 2636835 T3 | 09 October 2017 |
| | | ES 2636835 T7 | 20 November 2018 |
| | | HRP 20170840 T1 | 08 September 2017 |
| | | HRP 20170840 T4 | 16 November 2018 |
| | | EP 2970303 A2 | 20 January 2016 |
| | | EP 2970303 B1 | 10 May 2017 |
| | | EP 2970303 B9 | 20 September 2017 |
| | | EP 2970303 B3 | 01 August 2018 |
| | | BR 112015021164 A2 | 18 July 2017 |
| | | BR 112015021164 A8 | 03 December 2019 |
| | | WO 2014143799 A2 | 18 September 2014 |
| | | WO 2014143799 A3 | 06 November 2014 |
| | | WO 2014143799 A9 | 24 December 2014 |
| | | WO 2014143799 A4 | 12 February 2015 |
| | | CL 2015002738 A1 | 10 June 2016 |
| | | UY 35488 A | 31 October 2014 |
| | | EA 201591615 A1 | 31 May 2016 |
| | | EA 028815 B1 | 31 January 2018 |
| | | US 2023040259 A1 | 09 February 2023 |
| | | MY 189912 A | 21 March 2022 |
| | | US 2018244674 A1 | 30 August 2018 |
| | | US 10399982 B2 | 03 September 2019 |
| | | CY 1119042 T1 | 10 January 2018 |
| | | ZA 201505414 B | 30 March 2016 |
| | | US 2017305910 A1 | 26 October 2017 |
| | | US 9969736 B2 | 15 May 2018 |
| | | US 2014275071 A1 | 18 September 2014 |
| | | US 9359359 B2 | 07 June 2016 |
| | | NZ 711718 A | 29 May 2020 |
| | | HK 1217329 A1 | 06 January 2017 |
| | | PL 2970303 T3 | 31 August 2017 |
| | | PL 2970303 T6 | 31 December 2018 |
| | | PT 2970303 T | 23 June 2017 |
| | | DK 2970303 T3 | 26 June 2017 |
| | | DK 2970303 T5 | 02 January 2018 |
| | | DK 2970303 T6 | 08 October 2018 |
| | | PH 12015502141 A1 | 25 January 2016 |
| | | CA 2899646 A1 | 18 September 2014 |
| | | CA 2899646 C | 31 August 2021 |
| | | SI 2970303 T1 | 31 August 2017 |

Form PCT/ISA/210 (patent family annex) (July 2022)

**INTERNATIONAL SEARCH REPORT**
**Information on patent family members**

International application No.
**PCT/CN2024/072849**

| Patent document cited in search report | Publication date (day/month/year) | Patent family member(s) | Publication date (day/month/year) |
|---|---|---|---|
| | | RS 56066 B1 | 31 October 2017 |
| | | RS 56066 B2 | 28 September 2018 |
| | | LT 2970303 T | 25 July 2017 |
| | | UA 117470 C2 | 10 August 2018 |
| | | AU 2014228206 A1 | 24 September 2015 |
| | | AU 2014228206 B2 | 10 May 2018 |
| | | AU 2014228206 C1 | 25 October 2018 |
| | | KR 20150133233 A | 27 November 2015 |
| | | KR 102227629 B1 | 16 March 2021 |
| | | JP 2016513717 A | 16 May 2016 |
| | | JP 6360149 B2 | 18 July 2018 |
| | | PE 20151779 A1 | 20 November 2015 |
| | | TW 201533044 A | 01 September 2015 |
| | | TWI 609867 B | 01 January 2018 |
| CN 113166150 A | 23 July 2021 | EP 3894409 A1 | 20 October 2021 |
| | | EP 3894409 B1 | 23 August 2023 |
| | | US 2022056032 A1 | 24 February 2022 |
| | | WO 2020120449 A1 | 18 June 2020 |
| | | JP 2022512374 A | 03 February 2022 |
| | | JP 7408662 B2 | 05 January 2024 |
| CN 113166151 A | 23 July 2021 | US 2020190093 A1 | 18 June 2020 |
| | | US 11198696 B2 | 14 December 2021 |
| | | EA 202191588 A1 | 18 October 2021 |
| | | KR 20210102955 A | 20 August 2021 |
| | | TW 202039495 A | 01 November 2020 |
| | | BR 112021008316 A2 | 03 August 2021 |
| | | MX 2021007019 A | 10 September 2021 |
| | | JP 2022512373 A | 03 February 2022 |
| | | CA 3119229 A1 | 18 June 2020 |
| | | IL 283768 A | 29 July 2021 |
| | | IL 283768 B1 | 01 January 2024 |
| | | EP 3894410 A1 | 20 October 2021 |
| | | EP 3894410 B1 | 11 October 2023 |
| | | WO 2020120450 A1 | 18 June 2020 |
| | | AU 2019396499 A1 | 27 May 2021 |
| | | CL 2021001509 A1 | 17 December 2021 |
| | | DK 3894410 T3 | 27 November 2023 |
| | | US 2022177479 A1 | 09 June 2022 |
| CN 110997675 A | 10 April 2020 | DK 3652176 T3 | 17 January 2022 |
| | | PL 3652176 T3 | 04 April 2022 |
| | | IL 271799 A | 27 February 2020 |
| | | IL 271799 B | 01 August 2022 |
| | | LT 3652176 T | 25 February 2022 |
| | | EP 3652176 A1 | 20 May 2020 |
| | | EP 3652176 B1 | 15 December 2021 |
| | | JP 2020526591 A | 31 August 2020 |
| | | JP 7114702 B2 | 08 August 2022 |
| | | ES 2903268 T3 | 31 March 2022 |
| | | SI 3652176 T1 | 29 April 2022 |
| | | CL 2020000056 A1 | 12 June 2020 |

Form PCT/ISA/210 (patent family annex) (July 2022)

**INTERNATIONAL SEARCH REPORT**
**Information on patent family members**

International application No.
**PCT/CN2024/072849**

| Patent document cited in search report | Publication date (day/month/year) | Patent family member(s) | Publication date (day/month/year) |
|---|---|---|---|
| | | HUE 057600 T2 | 28 May 2022 |
| | | AU 2018299824 A1 | 19 December 2019 |
| | | AU 2018299824 B2 | 16 June 2022 |
| | | TW 201908318 A | 01 March 2019 |
| | | TWI 801398 B | 11 May 2023 |
| | | HRP 20220029 T1 | 15 April 2022 |
| | | UA 124793 C2 | 17 November 2021 |
| | | BR 112019025611 A2 | 16 June 2020 |
| | | PH 12020500079 A1 | 05 October 2020 |
| | | CA 3066922 A1 | 17 January 2019 |
| | | AR 112448 A1 | 30 October 2019 |
| | | EA 202090270 A1 | 24 April 2020 |
| | | EA 039526 B1 | 07 February 2022 |
| | | SG 11201912168 TA | 30 January 2020 |
| | | PT 3652176 T | 13 January 2022 |
| | | US 2019016722 A1 | 17 January 2019 |
| | | US 10329292 B2 | 25 June 2019 |
| | | CY 1125014 T1 | 24 March 2023 |
| | | SA 519410851 B1 | 26 February 2023 |
| | | CO 2019015102 A2 | 15 May 2020 |
| | | MX 2020000402 A | 17 August 2020 |
| | | RS 62826 B1 | 28 February 2022 |
| | | WO 2019011802 A1 | 17 January 2019 |
| | | KR 20200030048 A | 19 March 2020 |
| CN 115636831 A | 24 January 2023 | None | |
| WO 2021067946 A1 | 08 April 2021 | AU 2020357178 A1 | 12 May 2022 |
| | | IL 291903 A | 01 June 2022 |
| | | KR 20220079907 A | 14 June 2022 |
| | | MX 2022004049 A | 11 July 2022 |
| | | JP 2022551580 A | 12 December 2022 |
| | | EP 4037680 A1 | 10 August 2022 |
| | | EP 4037680 A4 | 04 October 2023 |
| | | CA 3156814 A1 | 08 April 2021 |
| WO 2014152287 A2 | 25 September 2014 | WO 2014152287 A3 | 04 December 2014 |
| | | WO 2014152287 A4 | 19 February 2015 |
| | | WO 2014152287 A9 | 23 April 2015 |
| | | US 2016145257 A1 | 26 May 2016 |
| | | EP 2970316 A2 | 20 January 2016 |
| | | EP 2970316 B1 | 08 November 2017 |
| | | JP 2016513687 A | 16 May 2016 |
| | | AR 095561 A1 | 28 October 2015 |
| | | TW 201444844 A | 01 December 2014 |
| | | UY 35424 A | 31 October 2014 |
| | | US 2014275528 A1 | 18 September 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014143799 A
- WO 2019011802 A


### Non-patent literature cited in the description


- *PLoS ONE.*, 2018, vol. 13, e0191225